(19)

Europäisches
Patentamt
European
Patent Office
Office européen
des brevets

(11) **EP 2 957 637 A2**

(12) **EUROPEAN PATENT APPLICATION**

(43) Date of publication:
23.12.2015 Bulletin 2015/52

(21) Application number: 15175599.8

(22) Date of filing: 19.04.2010

(51) Int Cl.:
*C12N 15/82* (2006.01)      *C07K 14/415* (2006.01)
*A01H 5/00* (2006.01)      *A01H 5/10* (2006.01)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(30) Priority: 29.04.2009 EP 09159059
26.05.2009 US 180948 P
07.07.2009 EP 09164832
08.07.2009 US 223712 P

(62) Document number(s) of the earlier application(s) in
accordance with Art. 76 EPC:
10713984.2 / 2 424 994

(27) Previously filed application:
19.04.2010 PCT/EP2010/055099

(71) Applicants:
• BASF Plant Science Company GmbH
67056 Ludwigshafen (DE)
• Crop Functional Genomics Center
Gwanak-Gu
Seoul 151-921 (KR)

(72) Inventors:
• Kim, Ju Kon
Sungnam 463-846 (KR)
• Choi, Yang Do
Seoul 137-909 (KR)
• Jeong, Jin Seo
Yongin 449-706 (KR)
• Reuzeau, Christophe
24350 La Chapelle Gonaguet (FR)

(74) Representative: **Mistry, Meeta**
**Greaves Brewster LLP**
**Copa House**
**Station Road**
**Cheddar, BS27 3AH (GB)**

Remarks:
•The complete document including Reference Tables
and the Sequence Listing can be downloaded from
the EPO website
•This application was filed on 07-07-2015 as a
divisional application to the application mentioned
under INID code 62.

(54) **PLANTS HAVING ENHANCED YIELD-RELATED TRAITS AND A METHOD FOR MAKING THE SAME**

(57) The present invention relates generally to the field of molecular biology and concerns a method for improving various plant growth characteristics by modulating expression in a plant of a nucleic acid encoding a NAC10-like polypeptide. The present invention also concerns plants having modulated expression of a nucleic acid encoding a NAC10-like polypeptide, which plants have improved growth characteristics relative to corresponding wild type plants or other control plants. The invention also provides constructs useful in the methods of the invention.

EP 2 957 637 A2

**Description**

Plants having enhanced yield-related traits and a method for making the same The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding an IAA2-like (auxin/indoleacetic acid 2 like) polypeptide. The present invention also concerns plants having modulated expression of a nucleic acid encoding IAA2-like polypeptide, which plants have enhanced yield-related traits relative to control plants. The invention also provides constructs comprising IAA2-like-encoding nucleic acids, useful in performing the methods of the invention.

The present invention also relates generally to the field of molecular biology and concerns a method for improving various plant growth characteristics by modulating expression in a plant of a nucleic acid encoding a NAC10-like polypeptide. The present invention also concerns plants having modulated expression of a nucleic acid encoding a NAC10-like polypeptide, which plants have improved growth characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various yield-related traits may be improved in plants by modulating expression in a plant of a nucleic acid encoding an IAA2-like polypeptide in a plant.

It has now also been found that various growth characteristics may be improved in plants by modulating expression in a plant of a nucleic acid encoding a NAC10-like polypeptide in a plant.

Background

1. IAA2-like polypeptides

The AUX/IAA (auxin/indoleacetic acid) genes encode a family of proteins whose expression is tightly regulated by auxin. The plant hormone auxin is involved in various processes like cell division, cell expansion and differentiation, patterning of embryos, vasculature or other tissues, regulation of growth of primary and lateral root or shoot meristems. AUX/IAA proteins furthermore are usually expressed in a tissue-specific manner.

AUX/IAA proteins typically have four conserved amino acid sequence motifs (domains I, II, III and IV) and have nuclear localisation signal sequences. Domains I and II are postulated to destabilize the protein and may be involved in protein turnover. Domains III and IV are postulated to be involved in protein-protein interactions: AUX/IAA proteins can form homodimers and are known to associate with ARF proteins. The AUX/IAA-ARF complexes are likely to be involved in auxin mediated gene expression. The Aux/IAA proteins are negative regulators of the auxin response factors (ARFs) that regulate expression of auxin-responsive genes. Aux/IAA proteins bind to the DNA-bound ARF partner proteins and repress ARF activity. In the auxin activated status, Aux/IAA proteins are ubiquitinated via interactions with the auxin-modified SCFTIR1complex and subsequently degraded by 26S proteasome action. An overview of roles and activities of AUX/IAA proteins is given by Reed (Trends in Plant Science 6, 420-425, 2001). The structure and expression analysis of early auxin-responsive Aux/IAA gene family in rice (Oryza sativa) has recently been reported by Jain et al. 2006 Funct Integr Genomics. 2006 Jan;6(1):47-59.

2. NAC10-like polypeptides

The rice and Arabidopsis genome codes for over 1300 transcriptional regulators, which account for 6% of its estimated total number of genes. About 45% of these transcription factors are reported to be from families specific to plants (Riechmann et al., 2000; Kikuch et al., 2003). One example of such a plant-specific family of transcription factors is the family of NACs. NAC is an acronym derived from the names of the three genes first described as containing a NAC domain, namely NAM (no apical meristem), ATAF1,2 and CUC2 (cup-shaped cotyledon). NAC proteins contain a highly conserved N-terminal DNA-binding domain, which is referred to as the "NAC-domain", and a variable C-terminal domain (Ooka et al., 2003). NAC proteins appear to be widespread in plants with the genomes of rice and Arabidopsis predicted to contain 75 and 105 NAC genes, respectively (Ooka et al., 2003; Xiong et al., 2005). Only a few of them have been characterized so far for their diverse functions in plant development and stress responses. The early reported NAC genes include NAM from petunia that determines the position of the shoot apical meristem (Souer et al., 1996) and CUC2 from Arabidopsis that participates in the development of embryos and flowers (Aida et al., 1997). The Arabidopsis NAP gene regulates cell division and cell expansion in flower organs (Sablowski et al., 1998). The AtNAC1 mediates auxin signaling to promote lateral root development (Xie et al., 2000). Genes in the ATAF subfamily, including StNAC (Collinge et al., 2001) from Potato (Solanum tuberosum) and ATAF1-2 (Aida et al., 1997) from Arabidopsis, were induced by pathogen attack and wounding. More recently, AtNAC072(RD29), AtNAC019, AtNAC055, and ANAC102 from Arabidopsis (Fujita et al., 2004; Tran et al., 2004; Christianson et al., 2009), BnNAC from Brassica napus (Hegedus et al., 2003), SNAC1 and SNAC2 from rice (Oryza sativa)(Hu et al., 2006; 2008), were found to be involved in response to various environmental stresses. AtNAC2, another stress-related NAC gene of Arabidopsis, acts downstream of the ethylene and auxin signal pathways and enhanced salt tolerance when overexpressed (He et al., 2005). A wheat NAC gene, NAM-B1, was reported to be involved in nutrient remobilization from leaves to developing grains (Uauy et al., 2006). Drought is a serious threat to the sustainability of rice yields in rainfed agriculture. In particular, exposure to drought conditions during the stage of panicle development results in a delayed flowering time, reduced number of spikelets and poor grain filling. To date, a number of studies have suggested that overexpression of stress related genes could improve drought tolerance in rice to some extent (Xu et al., 1996; Garg et al., 2002; Jang et al., 2003; Ito et al., 2006; Hu et al., 2006, 2008; Nakashima et al., 2007; Oh et al., 2007). Despite such efforts to develop drought-tolerant rice plants, very few of these have been shown to improve grain yields under field conditions.

Summary

1. IAA2-like polypeptides

Surprisingly, it has now been found that modulating expression of a nucleic acid encoding an IAA2-like polypeptide gives plants having enhanced yield-related traits relative to control plants.

According one embodiment, there is provided a method for enhancing yield-related traits of a plant relative to control plants, comprising modulating expression of a nucleic acid encoding an IAA2-like polypeptide in a plant.

2. NAC10-like polypeptides

Surprisingly, it has now been found that modulating expression of a nucleic acid encoding a NAC10-like polypeptide under control of a constitutive promoter or under control of a root-specific promoter gives plants having enhanced yield-related traits when grown under non-stress (normal) conditions and/or when grown under abiotic stress conditions, in particular improved tolerance to drought, high salinity and low temperature at vegetative stage and/or enhanced drought-tolerance at the reproductive stage, relative to control plants.

According one embodiment, there is provided a method for improving yield-related traits of a plant relative to control plants, comprising modulating expression of a nucleic acid encoding a NAC10-like polypeptide in a plant.

Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

Table 1: Examples of conserved amino acid substitutions

| Residue | Conservative Substitutions | Residue | Conservative Substitutions |
|---------|---------------------------|---------|---------------------------|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for in silico analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 \times \log_{10}[Na^+]^a + 0.41 \times \%[G/C^b] - 500 \times [L^c]^{-1} - 0.61 \times \% \text{ formamide}$$

2) DNA-RNA or RNA-RNA hybrids:

$$T_m = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (l_n)$

For 20-35 nucleotides: $T_m = 22 + 1.46 (I_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = $2\times$(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. $1\times$SSC is 0.15M NaCl and 15mM sodium citrate; the hybridisation solution and wash solutions may additionally include 5x Denhardt's reagent, 0.5-1.0% SDS, 100 $\mu$g/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

Table 2a: Examples of constitutive promoters

| Gene Source | Reference |
| --- | --- |
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
| --- | --- |
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

Table 2c: Examples of seed-specific promoters

| Gene source | Reference |
| --- | --- |
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PROO147, trypsin inhibitor ITR1 (barley) | unpublished |
| PRO0151, rice WSI18 | WO 2004/070039 |
| PRO0175, rice RAB21 | WO 2004/070039 |

(continued)

| Gene source | Reference |
|---|---|
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

Table 2d: examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

Table 2e: Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |

(continued)

| Gene source | Reference |
|---|---|
| PRO005 | WO 2004/070039 |
| PRO0095 | WO 2004/070039 |

Table 2f: Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

Table 2g: Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

Table 2h: Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

  (a) the nucleic acid sequences encoding proteins useful in the methods of the invention, or
  (b) genetic control sequence(s) which is operably linked with the nucleic acid sequence according to the invention, for example a promoter, or
  (c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.
A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.
Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an $\alpha$-anomeric nucleic acid sequence. An $\alpha$-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, such as plants used as a model, like Arabidopsis (Arabidopsis thaliana is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis.

Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Traits

Yield related traits comprise one or more of yield, biomass, seed yield, early vigour, greenness index, increased growth rate, improved agronomic traits (such as improved Water Use Efficiency (WUE), Nitrogen Use Efficiency (NUE), etc.).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production

(includes both harvested and appraised production) by planted square meters. The term "yield" of a plant may relate to vegetative biomass (root and/or shoot biomass), to reproductive organs, and/or to propagules (such as seeds) of that plant.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Nutrient deficiency may result from a lack of nutrients such as nitrogen, phosphates and other phosphorous-containing compounds, potassium, calcium, magnesium, manganese, iron and boron, amongst others.

The term salt stress is not restricted to common salt (NaCl), but may be any one or more of: NaCl, KCl, LiCl, $MgCl_2$, $CaCl_2$, amongst others.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Detailed description of the invention

Surprisingly, it has now been found that modulating expression in a plant of a nucleic acid encoding an IAA2-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding an IAA2-like polypeptide and wherein the yield related traits do not encompass increased root growth.

Furthermore, it has now been surprisingly found that modulating expression in a plant of a nucleic acid encoding a NAC10-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding a NAC10-like polypeptide and optionally selecting for plants having enhanced yield-related traits.

A preferred method for modulating (preferably, increasing) expression of a nucleic acid encoding an IAA2-like polypeptide or a NAC10-like polypeptide is by introducing and expressing in a plant a nucleic acid encoding an IAA2-like polypeptide or a NAC10-like polypeptide.

Concerning a IAA2-like polypeptides, any reference hereinafter to a "protein (or polypeptide) useful in the methods of the invention" is taken to mean an IAA2-like polypeptide as defined herein. Any reference hereinafter to a "nucleic acid useful in the methods of the invention" is taken to mean a nucleic acid capable of encoding such an IAA2-like polypeptide. The nucleic acid to be introduced into a plant (and therefore useful in performing the methods of the invention) is any nucleic acid encoding the type of protein which will now be described, hereafter also named "IAA2-like nucleic acid" or "IAA2-like gene".

Concerning a NAC10-like polypeptides, any reference hereinafter to a "protein useful in the methods of the invention" is taken to mean a NAC10-like polypeptide as defined herein. Any reference hereinafter to a "nucleic acid useful in the methods of the invention" is taken to mean a nucleic acid capable of encoding such a NAC10-like polypeptide. The nucleic acid to be introduced into a plant (and therefore useful in performing the methods of the invention) is any nucleic acid encoding the type of protein which will now be described, hereafter also named "NAC10-like nucleic acid" or "NAC10-like gene".

An "IAA2-like polypeptide" as defined herein refers to any polypeptide comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

A preferred "IAA2-like polypeptide" of the invention further comprises one of more of the following motifs:

Motif 2, SEQ ID NO: 23: (Q/G)LLDG(S/T)G(E/V)YTL
Motif 3, SEQ ID NO: 24: LLDGSGEYTLVY
Motif 4, SEQ ID NO: 25: KKLELRLGPPG

wherein amino acids indicated between brackets at a position represent alternative amino acids at that position.

The AUX/IAA domain spans amino acids 49 to 335 in SEQ ID NO: 2. A consensus sequence comprising the most highly conserved amino acids in IAA2-like polypeptide may be represented by SEQ ID NO: 21.

In addition, on another preferred embodiment the IAA2-like polypeptide of the invention has in increasing order of preference at least 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence of the AUX/IAA domain of any of the polypeptides of Table A1, preferably the amino acid sequence of the AUX/IAA domain of SEQ ID NO: 2, even more preferably to the sequence of the consensus AUX/IAA domain in IAA2-like polypeptides as represented by SEQ ID NO: 21.

Alternatively, the homologue of an IAA2-like protein has in increasing order of preference at least 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any of the polypeptide sequences of Table A1, preferably by SEQ ID NO: 2, provided that the homologous protein comprises an AUX/IAA domain and one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

A "NAC10-like polypeptide" as defined herein refers to any polypeptide comprising a NAM domain (Pfam accession PF02365, also known as NAC domain, Prosite accession PS51005).

Additionally or alternatively, a "NAC10-like polypeptide" as defined herein is one comprising any one or more of the Motifs described below.

Motif I: A/P/S/N/V/T T/V/L/I/A/S T/P/S/D/V/Q D/V/I/F I A/T/G/P (SEQ ID NO: 379), or a motif having in increasing order of preference 3, 2 or 1 conservative amino acid substitution compared to the sequence of Motif I.

Motif I is preferably P/T V/A/S/L S/P I/F I A/P, most preferably Motif I is PVPIIA.

Motif II: R/N/K E/G/S I/R/S/Q/A/V/L R/Q A/P N/S/R (SEQ ID NO: 380), or a motif having in increasing order of preference 3, 2 or 1 conservative amino acid substitution compared to the sequence of Motif II.

Motif II is preferably N/K G A/V/I/L RPN, most preferably Motif II is NGARPN.

Motif III: C/Y R/K L/I/V S/Y/H/F/R N/K/Q K D/K/N/C/S/T/G/M/A/R (SEQ ID NO: 381), or a motif having in increasing order of preference 3, 2 or 1 conservative amino acid substitution compared to the sequence of Motif III.

Motif III is preferably C R V/I H/Y/R N/K/Q K G/M/A/S/R/K, most preferably Motif III is CRIHKKS

More preferably, the NAC10-like polypeptide comprises in increasing order of preference, at least 2, or all 3 motifs.

Alternatively or additionally, the homologue of a NAC10-like protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 28, provided that the homologous protein comprises the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a NAC10-like polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the motifs represented by SEQ ID NO: 379 to SEQ ID NO: 381 (Motifs I to III).

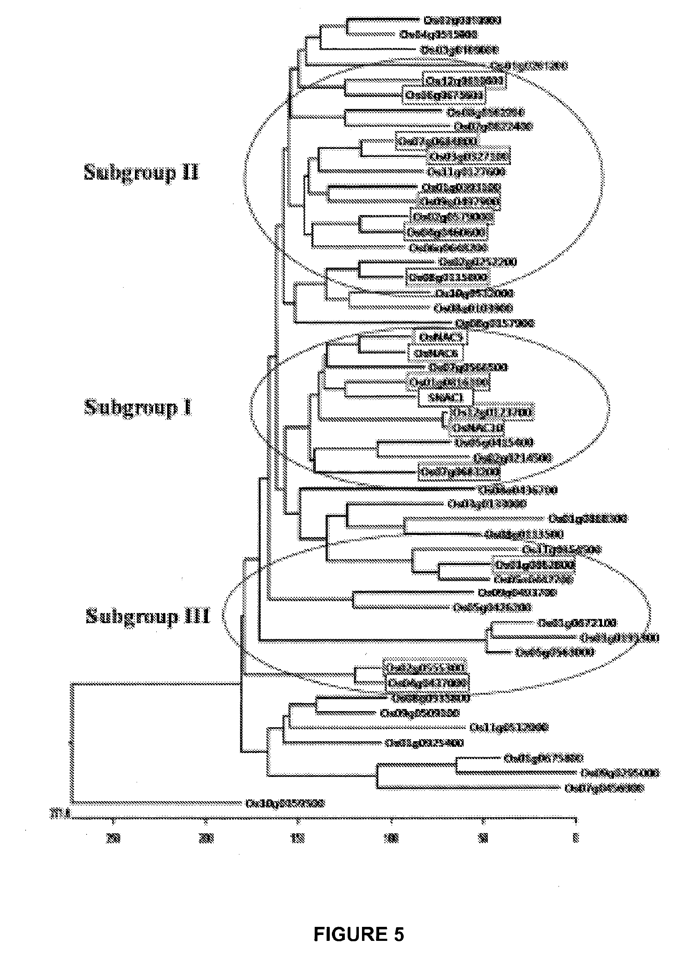

Preferably, the polypeptide sequence which when used in the construction of a phylogenetic tree, such as the one depicted in Figure 5, clusters with the group of NAC10-like polypeptides comprising the amino acid sequence represented by SEQ ID NO: 28 (Subgroup I) rather than with any other group.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; . Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for in silico analysis of protein sequences is available on the ExPASy proteomics server (Swiss Institute of Bioinformatics; Gasteiger et al., Nucleic Acids Res. 31:3784-3788 (2003)). Domains or motifs may also be identified using routine techniques, such as by sequence alignment.

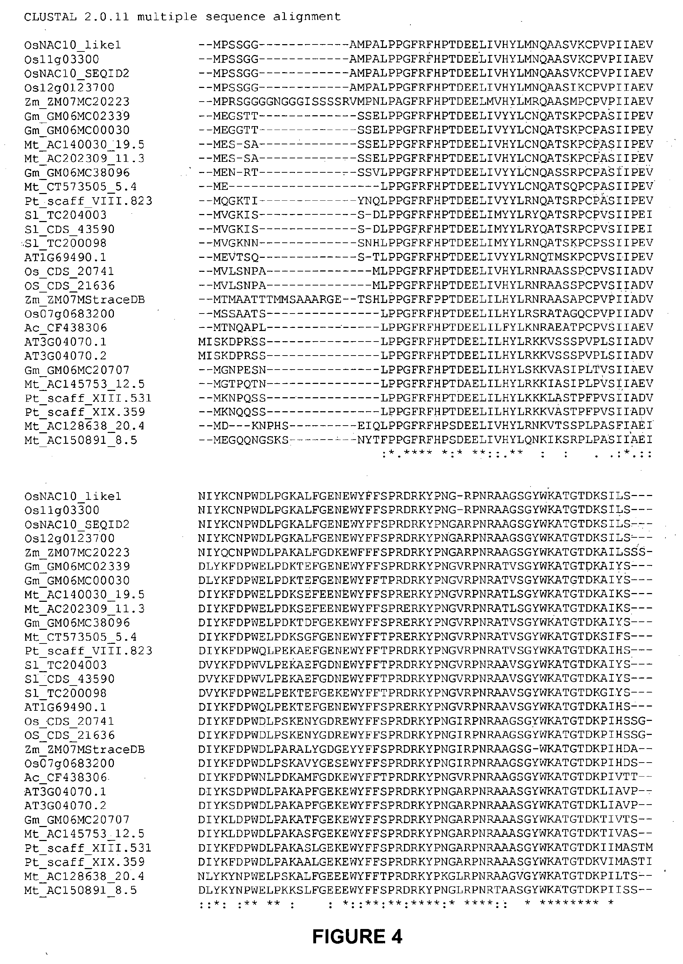

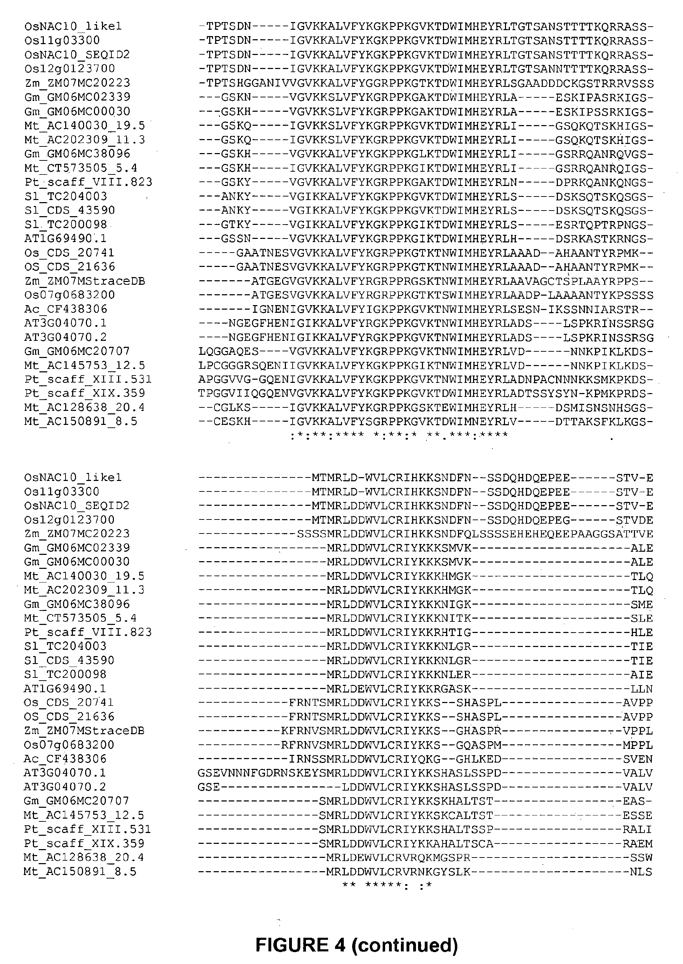

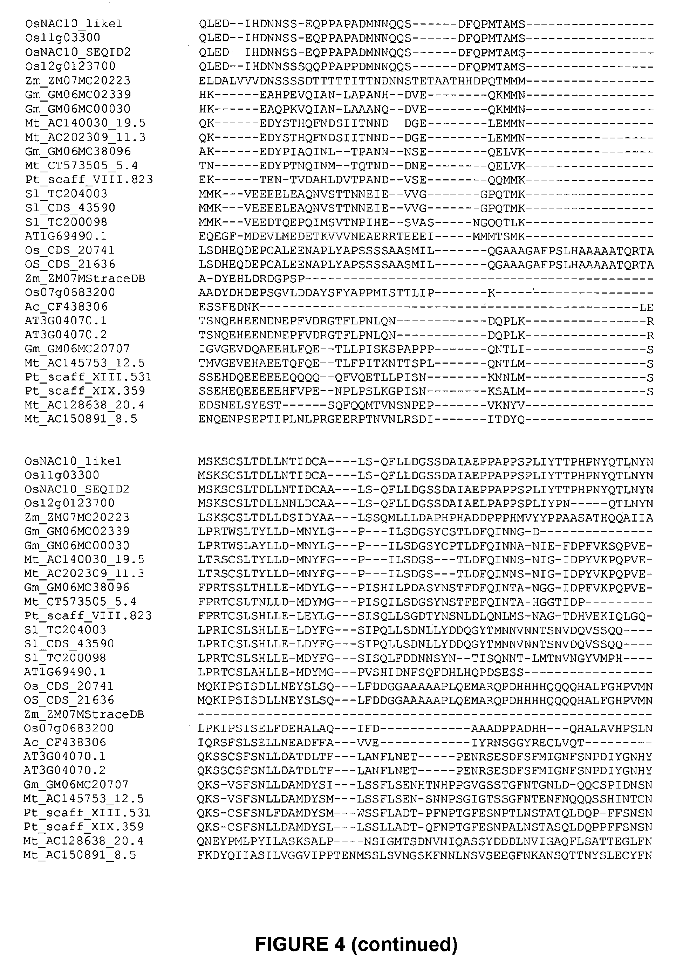

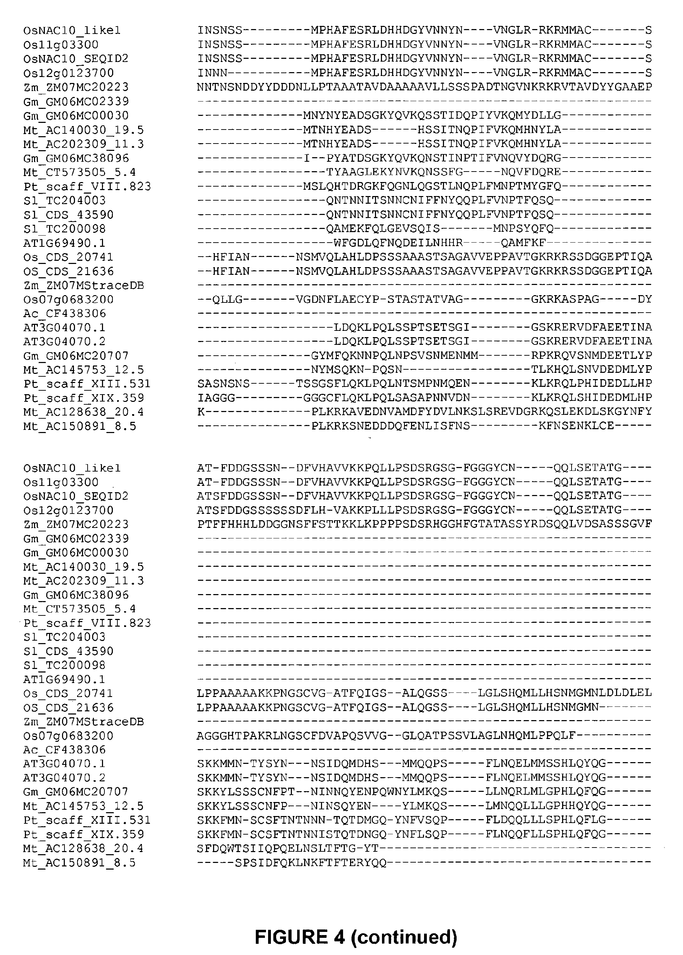

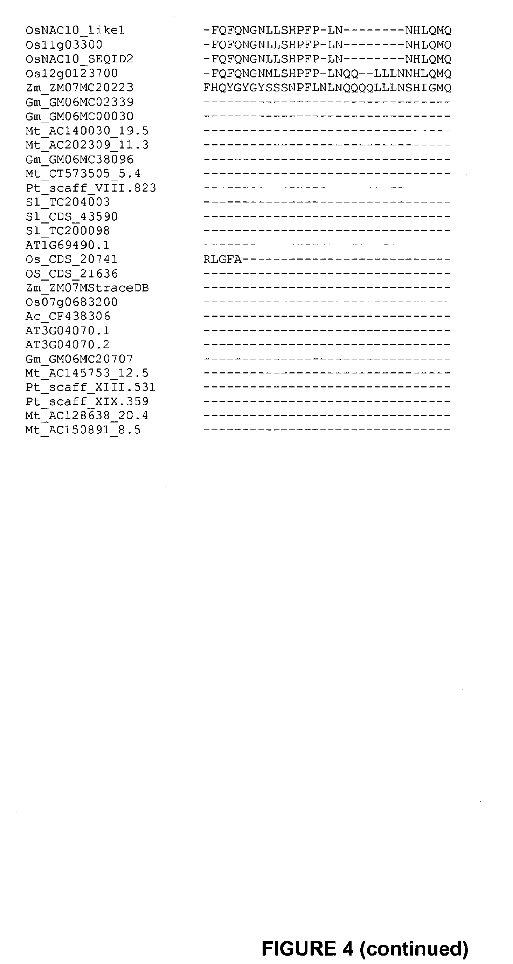

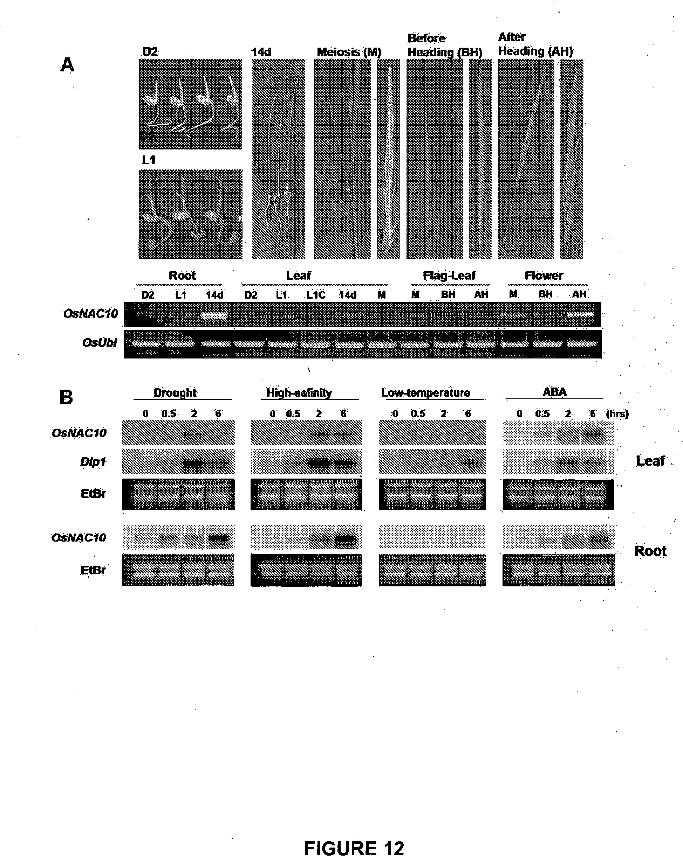

Concerning NAC10-like polypeptides, an alignment of the polypeptides of Table A2 herein is shown in Figure 5 and Figure 12. Such alignments are useful for identifying the most conserved domains or motifs between the NAC10-like polypeptides as defined herein. One such domain is NAM domain (Pfam accession PF02365), indicated in bold in Figure 4, whereas motifs I to III are underlined.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Concerning NAC10-like polypeptides, Example 3 describes in Table B1 the percentage identity between various NAC10-like polypeptides, in particular the percentage identity between SEQ ID NO: 28 and other NAC10-like polypeptides, which can be as low as 30 %.

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others.

Concerning NAC10-like polypeptides, the predicted subcellular localisation of SEQ ID NO: 28 using the PSort algorithm is the cytoplasmic or nuclear compartment (see Example 5). Given its function as transcriptional activator, the subcellular localisation is most likely the nucleus.

Furthermore, IAA2-like polypeptides (at least in their native form) typically have protein binding activity. Tools and techniques for measuring protein binding activity activity are well known in the art and include for example, inmuno precipitation of protein complexes or yeast two hybrid. Tools and techniques for measuring the association of IAA and ARF polypeptide are well known in the art., and include for example yeast two hybrid analysis (see for example Fukaki et al. (Plant J. 44, 382-395, 2005).

In addition, IAA2-like polypeptides, when expressed in rice according to the methods of the present invention as outlined in the Examples section, give plants having increased yield related traits, in particular increased seed yield

In addition, IAA2-like polypeptides, when expressed in rice according to the methods of the present invention as outlined in the Examples section, give plants having increased yield related traits such as increase seed fill rate and increased harvest index.

Additionally, IAA2-like polypeptides may display a preferred subcellular localization, typically one or more of nuclear, citoplasmic, chloroplastic, or mitochondrial. The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others.

Furthermore, NAC10-like polypeptides (at least in their native form) typically have DNA-binding activity. Tools and techniques for measuring DNA-binding activity are well known in the art, including PCR-assisted DNA binding site selection and a DNA binding gel-shift assay; for a general reference, see Current Protocols in Molecular Biology, Volumes 1 and 2, Ausubel et al. (1994), Current Protocols.

In addition, NAC10-like polypeptides, when expressed in rice according to the methods of the present invention as outlined in the Examples section, give plants having increased yield related traits, in particular increased resistance to salt, drought or cold stress in the vegetative phase of plant growth.

Concerning IAA2-like polypeptides, the present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any IAA2-like-encoding nucleic acid or IAA2-like polypeptide as defined herein.

Examples of nucleic acids encoding IAA2-like polypeptides are given in Table A1 of the Examples section herein. Such nucleic acids are useful in performing the methods of the invention. The amino acid sequences given in Table A1 of the Examples section are example sequences of orthologues and paralogues of the IAA2-like polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning NAC10-like polypeptides, the present invention is illustrated by transforming plants with the nucleic acid sequence represented by SEQ ID NO: 27, encoding the polypeptide sequence of SEQ ID NO: 28. However, performance of the invention is not restricted to these sequences; the methods of the invention may advantageously be performed using any NAC10-like-encoding nucleic acid or NAC10-like polypeptide as defined herein.

Examples of nucleic acids encoding NAC10-like polypeptides are given in Table A2 of the Examples section herein. Such nucleic acids are useful in performing the methods of the invention. The amino acid sequences given in Table A2 of the Examples section are example sequences of orthologues and paralogues of the NAC10-like polypeptide represented by SEQ ID NO: 28, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A2 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 27 or SEQ ID NO: 28, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A1 or A2 of the Examples section, the terms "homologue" and "derivative" being as defined herein. Also useful in the methods of the invention are nucleic acids encoding homologues and derivatives of orthologues or paralogues of any one of the amino acid sequences given in Table A1 or A2 of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding IAA-like polypeptides or NAC10-like polypeptides, nucleic acids hybridising to nucleic acids encoding IAA-like polypeptides or NAC10-like polypeptides, splice variants of nucleic acids encoding IAA-like polypeptides or NAC10-like polypeptides, allelic variants of nucleic acids encoding IAA-like polypeptides or NAC10-like polypeptides and variants of nucleic acids encoding IAA-like polypeptides or NAC10-like polypeptides obtained by gene shuffling. The terms hybridising sequence, splice variant, allelic variant and gene shuffling are as described herein.

Nucleic acids encoding IAA-like polypeptides or NAC10-like polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 or A2 of the Examples section, or a portion of a nucleic acid encoding an orthologue, paralogue or homologue of any of the amino acid sequences given in Table A1 or A2 of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Concerning IAA2-like polypeptides, portions useful in the methods of the invention, encode an IAA2-like polypeptide as defined herein, and have substantially the same biological activity as the amino acid sequences given in Table A1 of the Examples section. Preferably, the portion is a portion of any one of the nucleic acids given in Table A1 of the Examples section, or is a portion of a nucleic acid encoding an orthologue or paralogue of any one of the amino acid sequences given in Table A1 of the Examples section. Preferably the portion is at least 100, 200, 300, 400, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700 consecutive nucleotides in length, the consecutive nucleotides being of any one of the nucleic acid sequences given in Table A1 of the Examples section, or of a nucleic acid encoding an orthologue or paralogue of any one of the amino acid sequences given in Table A1 of the Examples section. Most preferably the portion is a portion of the nucleic acid of SEQ ID NO: 1. Preferably, the portion encodes a fragment of an amino acid sequence comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

Concerning NAC10-like polypeptides, portions useful in the methods of the invention, encode a NAC10-like polypeptide as defined herein, and have substantially the same biological activity as the amino acid sequences given in Table A2 of the Examples section. Preferably, the portion is a portion of any one of the nucleic acids given in Table A2 of the Examples section, or is a portion of a nucleic acid encoding an orthologue or paralogue of any one of the amino acid sequences given in Table A2 of the Examples section. Preferably the portion is at least 500, 550, 600, 650, 700, 750, 800, 850, 900, 950, 1000, 1050, 1100, 1150, 1200, 1250, 1300, 1350, 1400, 1450,1500, 1550, 1600, 1650, 1700, 1750, 1800, 1850, 1900 consecutive nucleotides in length, the consecutive nucleotides being of any one of the nucleic acid sequences given in Table A2 of the Examples section, or of a nucleic acid encoding an orthologue or paralogue of any one of the amino acid sequences given in Table A2 of the Examples section. Most preferably the portion is a portion of the nucleic acid of SEQ ID NO: 27, comprising the NAM domain and one or more of the motifs I to III. Preferably, the portion encodes a fragment of an amino acid sequence which, when used in the construction of a phylogenetic tree, such as the one depicted in Figure 5, clusters with the group of NAC10-like polypeptides comprising the amino acid sequence represented by SEQ ID NO: 28 (Subgroup I) rather than with any other group and is functionally equivalent to SEQ ID NO: 28.

Another nucleic acid variant useful in the methods of the invention is a nucleic acid capable of hybridising, under reduced stringency conditions, preferably under stringent conditions, with a nucleic acid encoding an IAA-like polypeptide or a NAC10-like polypeptide as defined herein, or with a portion as defined herein.

According to the present invention, there is provided a method for enhancing yield-related traits in plants, comprising introducing and expressing in a plant a nucleic acid capable of hybridising to any one of the nucleic acids given in Table A1 and/or A2 of the Examples section, or comprising introducing and expressing in a plant a nucleic acid capable of hybridising to a nucleic acid encoding an orthologue, paralogue or homologue of any of the nucleic acid sequences given in Table A1 and/or A2 of the Examples section.

Concerning IAA2-like polypeptides, hybridising sequences useful in the methods of the invention encode an IAA2-like polypeptide as defined herein, having substantially the same biological activity as the amino acid sequences given in Table A1 of the Examples section. Preferably, the hybridising sequence is capable of hybridising to the complement of any one of the nucleic acids given in Table A1 of the Examples section, or to a portion of any of these sequences, a portion being as defined above, or the hybridising sequence is capable of hybridising to the complement of a nucleic acid encoding an orthologue or paralogue of any one of the amino acid sequences given in Table A1 of the Examples section. Most preferably, the hybridising sequence is capable of hybridising to the complement of a nucleic acid as represented by SEQ ID NO: 1 or to a portion thereof.

Preferably, the hybridising sequence or its complementary sequence encodes a polypeptide with an amino acid sequence comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

Concerning NAC10-like polypeptides, hybridising sequences useful in the methods of the invention encode a NAC10-like polypeptide as defined herein, having substantially the same biological activity as the amino acid sequences given in Table A2 of the Examples section. Preferably, the hybridising sequence is capable of hybridising to the complement of any one of the nucleic acids given in Table A2 of the Examples section, or to a portion of any of these sequences, a portion being as defined above, or the hybridising sequence is capable of hybridising to the complement of a nucleic acid encoding an orthologue or paralogue of any one of the amino acid sequences given in Table A2 of the Examples section. Most preferably, the hybridising sequence is capable of hybridising to the complement of a nucleic acid as represented by SEQ ID NO: 27 or to a portion thereof.

Preferably, the hybridising sequence encodes a polypeptide with an amino acid sequence which comprises the NAM domain and one or more of motifs I to III (as defined above) and which, when full-length and used in the construction of a phylogenetic tree such as the one depicted in Figure 5, clusters with the group of NAC10-like polypeptides comprising the amino acid sequence represented by SEQ ID NO: 28 (Subgroup I) rather than with any other group and is functionally equivalent to SEQ ID NO: 28.

Another nucleic acid variant useful in the methods of the invention is a splice variant encoding an IAA-like polypeptide or a NAC10-like polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A1 or Table A2 of the Examples section, or a splice variant of a nucleic acid encoding an orthologue, paralogue or homologue of any of the amino acid sequences given in Table A1 or Table A2 of the Examples section.

Concerning IAA2-like polypeptides, preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Concerning NAC10-like polypeptides, preferred splice variants are splice variants of a nucleic acid represented by SEQ ID NO: 27, or a splice variant of a nucleic acid encoding an orthologue or paralogue of SEQ ID NO: 28. Preferably, the amino acid sequence encoded by the splice variant which comprises the NAM domain and one or more of motifs I to III (as defined above) and which, when full-length and used in the construction of a phylogenetic tree such as the one depicted in Figure 5, clusters with the group of NAC10-like polypeptides comprising the amino acid sequence represented by SEQ ID NO: 28 (Subgroup I) rather than with any other group and is functionally equivalent to SEQ ID NO: 28.

Another nucleic acid variant useful in performing the methods of the invention is an allelic variant of a nucleic acid encoding an IAA-like polypeptide or a NAC10-like polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A1 or Table A2 of the Examples section, or comprising introducing and expressing in a plant an allelic variant of a nucleic acid encoding an orthologue, paralogue or homologue of any of the amino acid sequences given in Table A1 or Table A2 of the Examples section.

Concerning IAA2-like polypeptides, the polypeptides encoded by allelic variants useful in the methods of the present invention have substantially the same biological activity as the IAA2-like polypeptide of SEQ ID NO: 2 and any of the amino acids depicted in Table A1 of the Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Concerning NAC10-like polypeptides, the polypeptides encoded by allelic variants useful in the methods of the present invention have substantially the same biological activity as the NAC10-like polypeptide of SEQ ID NO: 28 and any of the amino acids depicted in Table A2 of the Examples section. Allelic variants exist in nature, and encompassed within the methods of the present invention is the use of these natural alleles. Preferably, the allelic variant is an allelic variant of SEQ ID NO: 27 or an allelic variant of a nucleic acid encoding an orthologue or paralogue of SEQ ID NO: 28. Preferably, the amino acid sequence encoded by the allelic variant, which comprises the NAM domain and one or more of motifs I to III (as defined above) and which, when full-length and used in the construction of a phylogenetic tree such as the one depicted in Figure 5, clusters with the group of NAC10-like polypeptides comprising the amino acid sequence represented by SEQ ID NO: 28 (Subgroup 1) rather than with any other group and is functionally equivalent to SEQ ID NO: 28.

Gene shuffling or directed evolution may also be used to generate variants of nucleic acids encoding IAA-like polypeptides or NAC10-like polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A1 or Table A2 of the Examples section, or comprising introducing and expressing in a plant a variant of a nucleic acid encoding an orthologue, paralogue or homologue of any of the amino acid sequences given in Table A1 or Table A2 of the Examples section, which variant nucleic acid is obtained by gene shuffling.

Concerning IAA2-like polypeptides, preferably, the amino acid sequence encoded by the variant nucleic acid obtained by gene shuffling comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Concerning NAC10-like polypeptides, preferably, the amino acid sequence encoded by the variant nucleic acid obtained by gene shuffling, which comprises the NAM domain and one or more of motifs I to III (as defined above) and which, when full-length and used in the construction of a phylogenetic tree such as the one depicted in Figure 5, clusters with the group of NAC10-like polypeptides comprising the amino acid sequence represented by SEQ ID NO: 28 (Subgroup I) rather than with any other group and is functionally equivalent to SEQ ID NO: 28.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding IAA2-like polypeptides may be derived from any natural or artificial source. The nucleic acid may be modified from its native form in composition and/or genomic environment through deliberate human manipulation. Preferably the IAA2-like polypeptide-encoding nucleic acid is from a plant, further preferably from a monocotyledonous plant, more preferably from the family Poaceae, most preferably the nucleic acid is from Oryza sativa.

Nucleic acids encoding NAC10-like polypeptides may be derived from any natural or artificial source. The nucleic acid may be modified from its native form in composition and/or genomic environment through deliberate human manipulation. Preferably the NAC10-like polypeptide-encoding nucleic acid is from a plant, further preferably from a monocotyledonous plant, more preferably from the family Poaceae, most preferably the nucleic acid is from Oryza sativa.

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others.

Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

The present invention provides a method for increasing yield, especially seed yield of plants, relative to control plants, which method comprises modulating expression in a plant of a nucleic acid encoding an IAA-like polypeptide or a NAC10-like polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding an IAA-like polypeptide or a NAC10-like polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects. The term "non-stress" conditions as used herein are those environmental conditions that allow optimal growth of plants. Persons skilled in the art are aware of normal soil conditions and climatic conditions for a given location. The term non-stress conditions as used herein, encompasses the occasional or everyday mild stresses to which a plant is exposed, as defined herein, but does not encompass severe stresses.

In particular, the methods of the present invention may be performed under conditions of drought, cold or salt stress, to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Concerning IAA2-like polypeptides, performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding an IAA2-like polypeptide.

Concerning NAC10-like polypeptides, performance of the methods of the invention gives plants grown under non-stress conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a NAC10-like polypeptide.

Performance of the methods of the invention gives plants grown under conditions of nutrient deficiency, particularly under conditions of nitrogen deficiency, increased yield relative to control plants grown under comparable conditions. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with increased yield-related traits, when grown under nitrogen-limiting conditions. Thus, according to the present invention, there is provided a method for increasing yield in plants grown under conditions of nutrient deficiency, which method comprises modulating expression in a plant of a nucleic acid encoding an IAA-like polypeptide or a NAC10-like polypeptide. Nutrient deficiency may result from a lack of nutrients such as nitrogen, phosphates and other phosphorous-containing compounds, potassium, calcium, magnesium, manganese, iron and boron, amongst others.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding an IAA-like polypeptide or a NAC10-like polypeptide. The term salt stress is not restricted to common salt (NaCl), but may be any one or more of: NaCl, KCl, LiCl, $MgCl_2$, $CaCl_2$, amongst others.

In a further embodiment, the invention provides genes having altered expression levels in plants overexpressing a NAC10-like protein relative to expression levels in corresponding wild type plants. Furthermore, the present invention provides means to modulate expression of these genes, which in turn allows for modulation of the biological processes that they control. The present invention provides methods to mimic NAC10-like protein level and/or activity by manipulating downstream factors involved in NAC10-like pathways. This strategy allows a fine-tuning of the effects of NAC10-like protein. Whereas overexpression of NAC10-like protein can be pleiotropic and/or can have pleiotropic effects, the invention provides methods to alter plant characteristics in a more controlled and targeted way, by using the NAC10-like protein target genes as defined by the present invention. Modulation of particular biological processes is now possible and may give rise to plants having altered characteristics, which may have particularly useful applications in agriculture and horticulture, such as improved yield-related traits for plants grown under normal or under stress conditions.

Therefore, according to the present invention, there is provided a method to enhance one or more yield-related traits in plants, comprising modifying in a plant expression of one or more nucleic acids and/or modifying level and/or activity of one or more proteins, which nucleic acids or proteins are essentially similar to any one of the genes listed in Tables I to K, and wherein said one or more yield-related traits are altered relative to corresponding wild type plants.

The present invention encompasses plants or parts thereof (including seeds) obtainable by the methods according to the present invention. The plants or parts thereof comprise a nucleic acid transgene encoding an IAA-like polypeptide or a NAC10-like polypeptide as defined above.

The invention also provides genetic constructs and vectors to facilitate introduction and/or expression in plants of nucleic acids encoding IAA-like polypeptides or NAC10-like polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an IAA-like polypeptide or a NAC10-like polypeptide as defined above;
(b) one or more control sequences capable of driving expression of the nucleic acid sequence of (a); and optionally
(c) a transcription termination sequence.

Preferably, the nucleic acid encoding an IAA-like polypeptide or a NAC10-like polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types. Concerning NAC10-like polypeptides, also useful in the methods of the invention is a root-specific promoter.

Concerning IAA2-like polypeptides, it should be clear that the applicability of the present invention is not restricted to the IAA2-like polypeptide-encoding nucleic acid represented by SEQ ID NO: 1, nor is the applicability of the invention restricted to expression of an IAA2-like polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 26, most preferably the constitutive promoter is as represented by SEQ ID NO: 26. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 26, and the nucleic acid encoding the IAA2-like polypeptide.

Concerning NAC10-like polypeptides, it should be clear that the applicability of the present invention is not restricted to the NAC10-like polypeptide-encoding nucleic acid represented by SEQ ID NO: 27, nor is the applicability of the invention restricted to expression of a NAC10-like polypeptide-encoding nucleic acid when driven by a constitutive promoter, or when driven by a root-specific promoter.

The constitutive promoter is preferably a medium strength constitutive promoter, more preferably selected from a plant, such as a GOS2 promoter, more preferably the promoter is a GOS2 promoter from rice. Further preferably the constitutive promoter is represented by a nucleic acid sequence substantially similar to SEQ ID NO: 382, most preferably the constitutive promoter is as represented by SEQ ID NO: 382. See the "Definitions" section herein for further examples of constitutive promoters.

According to another preferred feature of the invention, the nucleic acid encoding a NAC10-like polypeptide is operably linked to a root-specific promoter. The root-specific promoter is preferably an RCc3 promoter (Plant Mol Biol. 1995 Jan;27(2):237-48), more preferably the RCc3 promoter is from rice, further preferably the RCc3 promoter is represented by a nucleic acid sequence substantially similar to SEQ ID NO: 383, most preferably the promoter is as represented by SEQ ID NO: 383. Examples of other root-specific promoters which may also be used to perform the methods of the invention are shown in Table 3 in the "Definitions" section above.

Optionally, one or more terminator sequences may be used in the construct introduced into a plant. Preferably, the construct comprises an expression cassette comprising a GOS2 or an RCc3 promoter, substantially similar to SEQ ID NO: 382, respectively SEQ ID NO: 383, and the nucleic acid encoding the NAC10-like polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding an IAA-like polypeptide or a NAC10-like polypeptide as defined hereinabove.

More specifically, the present invention provides a method for the production of transgenic plants having enhanced yield-related traits, particularly increased stress resistance and/or increased yield, which method comprises:

(i) introducing and expressing in a plant or plant cell a nucleic acid encoding an IAA-like polypeptide or a NAC10-like polypeptide; and
(ii) cultivating the plant cell under conditions promoting plant growth and development.

The nucleic acid of (i) may be any of the nucleic acids capable of encoding an IAA-like polypeptide or a NAC10-like polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding an IAA-like polypeptide or a NAC10-like polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding an IAA-like polypeptide or a NAC10-like polypeptide. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding an IAA-like polypeptide or a NAC10-like polypeptide is by introducing and expressing in a plant a nucleic acid encoding an IAA-like polypeptide or a NAC10-like polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding NAC10-like polypeptides as described herein and use of these IAA-like polypeptides or NAC10-like polypeptides in enhancing any of the aforementioned yield-related traits in plants.

Nucleic acids encoding NAC10-like polypeptide described herein, or the IAA-like polypeptides or NAC10-like polypeptides themselves, may find use in breeding programmes in which a DNA marker is identified which may be genetically linked to a gene encoding an IAA-like polypeptide or a NAC10-like polypeptide. The nucleic acids/genes, or the IAA-like polypeptides or NAC10-like polypeptides themselves may be used to define a molecular marker. This DNA or protein marker may then be used in breeding programmes to select plants having enhanced yield-related traits as defined hereinabove in the methods of the invention.

Allelic variants of a nucleic acid/gene encoding IAA-like polypeptides or NAC10-like polypeptides may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding IAA-like polypeptides or NAC10-like polypeptides may also be used as probes for genetically and physically mapping the genes that they are a part of, and as markers for traits linked to those genes. Such information may be useful in plant breeding in order to develop lines with desired phenotypes. Such use of nucleic acids encoding IAA-like polypeptides or NAC10-like polypeptides requires only a nucleic acid sequence of at least 15 nucleotides in length. The nucleic acids encoding IAA-like polypeptides or NAC10-like polypeptides may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the NAC10-like-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding IAA-like polypeptides or NAC10-like polypeptides in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

Items

1. IAA2-like polypeptides

1. A method for enhancing yield-related traits in plants relative to control plants, comprising modulating expression in a plant of a nucleic acid encoding an IAA2-like polypeptide, wherein said IAA2-like polypeptide comprises an AUX/IAA domain (PFAM accession number PF2309, InterPro entry IPR003311) domain and the protein motif represented by SEQ ID NO: 22.

2. Method according to item 1, wherein said IAA2-like polypeptide comprises one or more of the following motifs:

>    (i) Motif 2, SEQ ID NO: 23: (Q/G)LLDG(S/T)G(E/V)YTL
>    (ii) Motif 3, SEQ ID NO: 24: LLDGSGEYTLVY
>    (iii) Motif 4, SEQ ID NO: 25: KKLELRLGPPG

wherein amino acids indicated between brackets at a position represent alternative amino acids at that position.

3. Method according to item 1 or 2, wherein said modulated expression is effected by introducing and expressing in a plant a nucleic acid encoding an IAA2-like polypeptide.

4. Method according to any one of items 1 to 3, wherein said nucleic acid encoding an IAA2-like polypeptide encodes any one of the proteins listed in Table A1 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A1.

6. Method according to any preceding item, wherein said enhanced yield-related traits comprise increased yield, preferably increased biomass and/or increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding an IAA2-like polypeptide is of plant origin, preferably from a monocotyledonous plant, further preferably from the family Poaceae, more preferably from the genus Oryza, most preferably from Oryza sativa.

11. Plant or part thereof, including seeds, obtainable by a method according to any one of items 1 to 10, wherein said plant or part thereof comprises a recombinant nucleic acid encoding an IAA2-like polypeptide.

12. Construct comprising:

>    (i) nucleic acid encoding an IAA2-like polypeptide as defined in items 1 or 2;
>    (ii) one or more control sequences capable of driving expression of the nucleic acid sequence of (a); and optionally
>    (iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased biomass and/or increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

>    (i) introducing and expressing in a plant a nucleic acid encoding an IAA2-like polypeptide as defined in item 1 or 2; and

(ii) cultivating the plant cell under conditions promoting plant growth and development.

17. Transgenic plant having increased yield, particularly increased biomass and/or increased seed yield, relative to control plants, resulting from modulated expression of a nucleic acid encoding an IAA2-like polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding an IAA2-like polypeptide in increasing yield, particularly in increasing seed yield and/or shoot biomass in plants, relative to control plants.

2. NAC10-like polypeptides

1. A method for enhancing yield-related traits in plants relative to control plants, comprising modulating expression in a plant of a nucleic acid encoding a NAC10-like polypeptide, wherein said NAC10-like polypeptide comprises a NAM domain (Pfam PF02365).

2. Method according to item 1, wherein said NAC10-like polypeptide comprises one or more of the following motifs:

(i) Motif I: A/P/S/N/V/T T/V/L/I/A/S T/P/S/D/V/Q D/V/I/F I A/T/G/P,
(ii) Motif II: R/N/K E/G/S I/R/S/Q/A/V/L R/Q A/P N/S/R,
(iii) Motif III: C/Y R/K L/I/V S/Y/H/F/R N/K/Q K D/K/N/C/S/T/G/M/A/R

3. Method according to item 1 or 2, wherein said modulated expression is effected by introducing and expressing in a plant a nucleic acid encoding a NAC10-like polypeptide.

4. Method according to any one of items 1 to 3, wherein said nucleic acid encoding a NAC10-like polypeptide encodes any one of the proteins listed in Table A2 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A2.

6. Method according to any preceding item, wherein said enhanced yield-related traits comprise increased yield, preferably increased biomass and/or increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a root specific or to a constitutive promoter, preferably to an RCc3 or a GOS2 promoter, most preferably to an RCc3 or a GOS2 promoter from rice.

10. Method according to any one of items 1 to 9, wherein said nucleic acid encoding a NAC10-like polypeptide is of plant origin, preferably from a dicotyledonous plant, further preferably from the family Poaceae, more preferably from the genus Oryza, most preferably from Oryza sativa.

11. Plant or part thereof, including seeds, obtainable by a method according to any one of items 1 to 10, wherein said plant or part thereof comprises a recombinant nucleic acid encoding a NAC10-like polypeptide.

12. Construct comprising:

(i) nucleic acid encoding a NAC10-like polypeptide as defined in items 1 or 2;
(ii) one or more control sequences capable of driving expression of the nucleic acid sequence of (a); and optionally
(iii) a transcription termination sequence.

13. Construct according to item 12, wherein one of said control sequences is a root specific or a constitutive promoter, preferably an RCc3 or a GOS2 promoter, most preferably an RCc3 or a GOS2 promoter from rice.

14. Use of a construct according to item 12 or 13 in a method for making plants having increased yield, particularly increased biomass and/or increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

(i) introducing and expressing in a plant a nucleic acid encoding a NAC10-like polypeptide as defined in item 1 or 2; and
(ii) cultivating the plant cell under conditions promoting plant growth and development.

17. Transgenic plant having increased yield, particularly increased biomass and/or increased seed yield, relative to control plants, resulting from modulated expression of a nucleic acid encoding a NAC10-like polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding a NAC10-like polypeptide in increasing yield, particularly in increasing seed yield and/or shoot biomass in plants, relative to control plants.

Description of figures

The present invention will now be described with reference to the following figures in which:

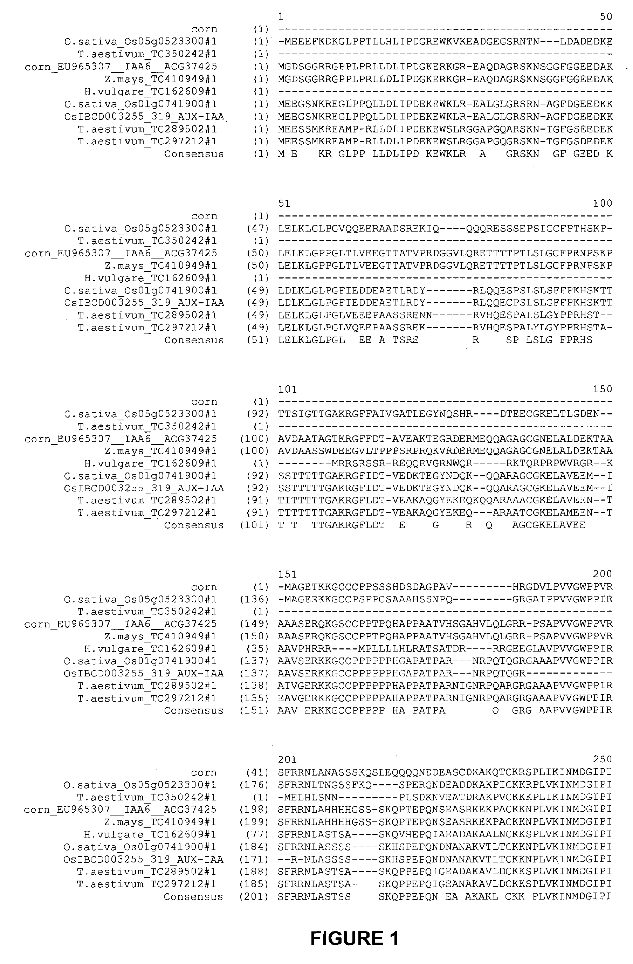

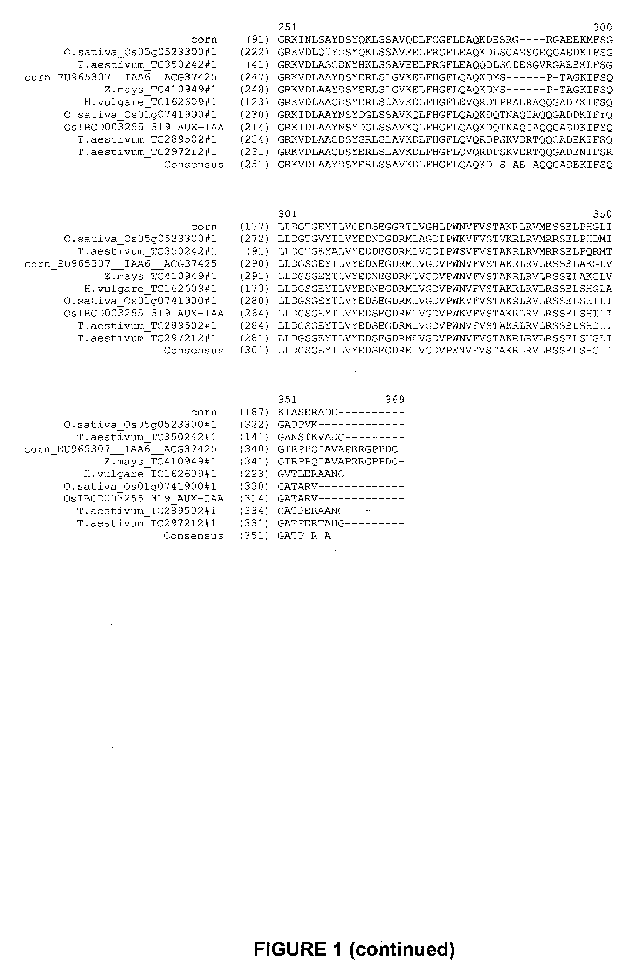

Figure 1 represents a multiple alignment of IAA2-like polypeptides.

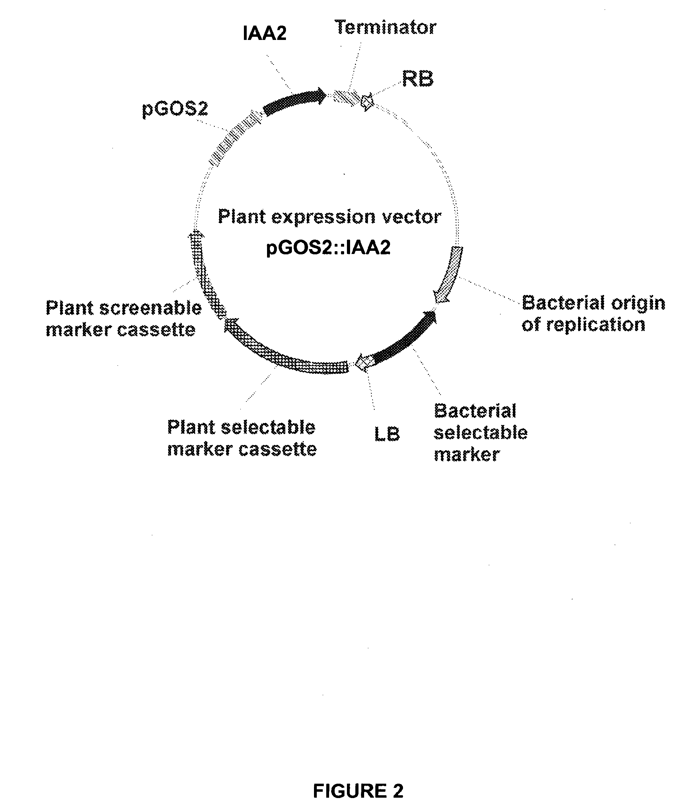

Figure 2 represents the binary vector used for increased expression in Oryza sativa of an IAA2-like-encoding nucleic acid under the control of a rice GOS2 promoter (pGOS2).

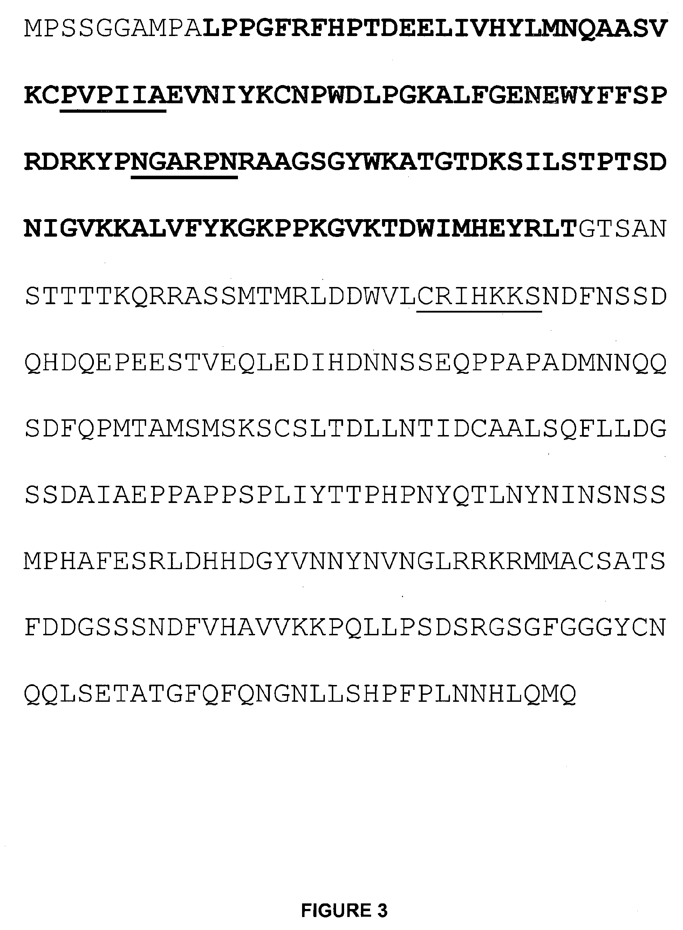

Figure 3 represents the domain structure of SEQ ID NO: 28 with the NAM domain (PF02365) indicated in bold and the conserved motifs I to III underlined.
Figure 4 represents a multiple alignment of various NAC10-like polypeptides, using ClustalW with default settings for a slow alignment.
Figure 5 shows the phylogenetic relationship of the rice NAC family. A phylogenic dendrogram was derived from an alignment of the deduced amino acid sequences of 54 rice NAC factors using the CLUSTAL W program. Of 105 NAC factors predicted from the rice genome, we selected eighteen that have EST information from NCBI database search using the tBLAST N program. Eighteen stress-inducible factors were indicated by boxes. Subgroups I to III shown in Table G are marked by circles.

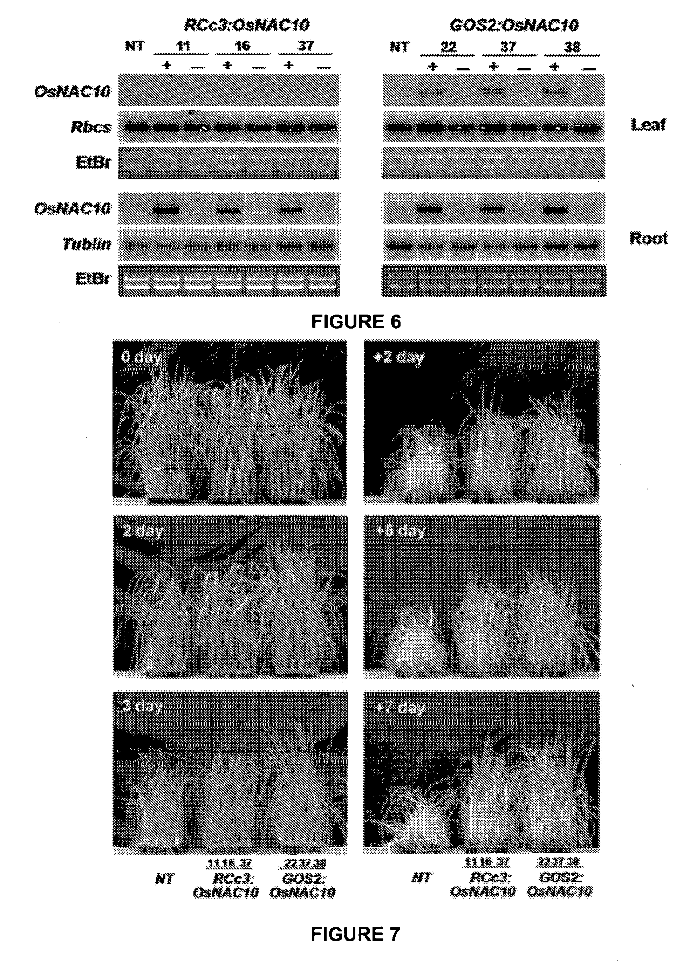

Figure 6 RNA gel-blot analysis was performed using total RNAs from roots and leaves of three homozygous T4 lines of RCc3:OsNAC10 and GOS2:OsNAC10 plants, respectively, and of nontransgenic (NT) control plants. The blots were hybridized with the OsNAC10 gene specific probes, and reprobed for RbcS (Jang et al., 1999) and Tublin. EtBr staining was used to determine equal loading of RNAs. (-) Nullizygous (without transgene) lines, (+) Transgenic lines.

Figure 7 Appearance of transgenic plants during drought stress. Three independent homozygous T4 lines of RCc3:OsNAC10 and GOS2:OsNAC10 plants and nontransgenic (NT) controls were grown for 4 weeks, subjected to 3 d of drought stress and followed by 7 d re-watering in the greenhouse. Images were taken at the indicated time points. "+" denotes the number of re-watering days under normal growth conditions.

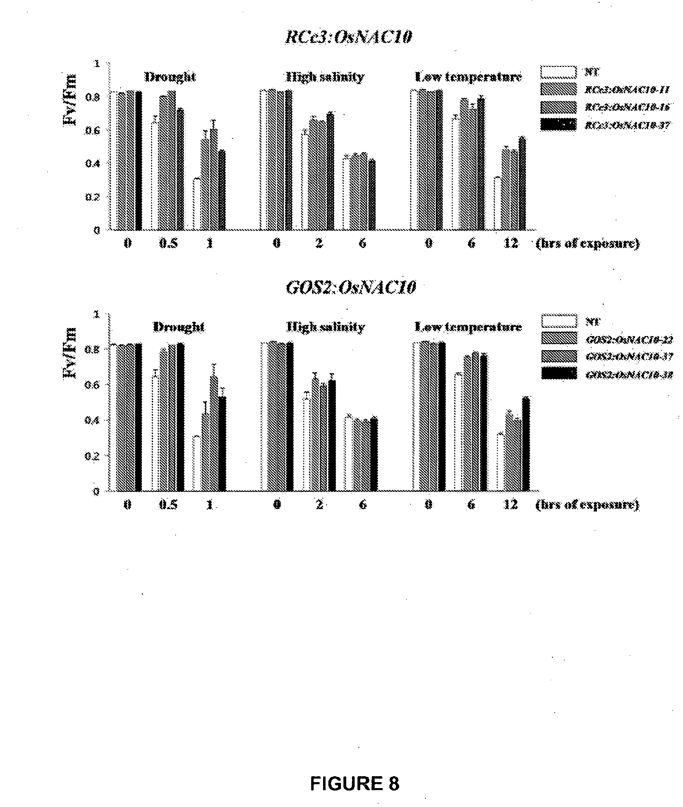

Figure 8 Changes in chlorophyll fluorescence (Fv/Fm) of rice plants under drought, high salinity and low temperature stress conditions. Three independent homozygous T4 lines of RCc3:OsNAC10 and GOS2:OsNAC10 plants and nontransgenic (NT) controls grown in MS medium for 14 days were subjected to various stress conditions as described in the Materials and Methods section. After stress treatments, the Fv/Fm values were measured using a pulse modulation fluorometer (mini-PAM, Walze, Germany). All plants were grown under a continuous light of 150 $\mu$mol m-2 s-1 prior to stress treatments. Each data point represents the mean $\pm$SE of triplicates (n=10).

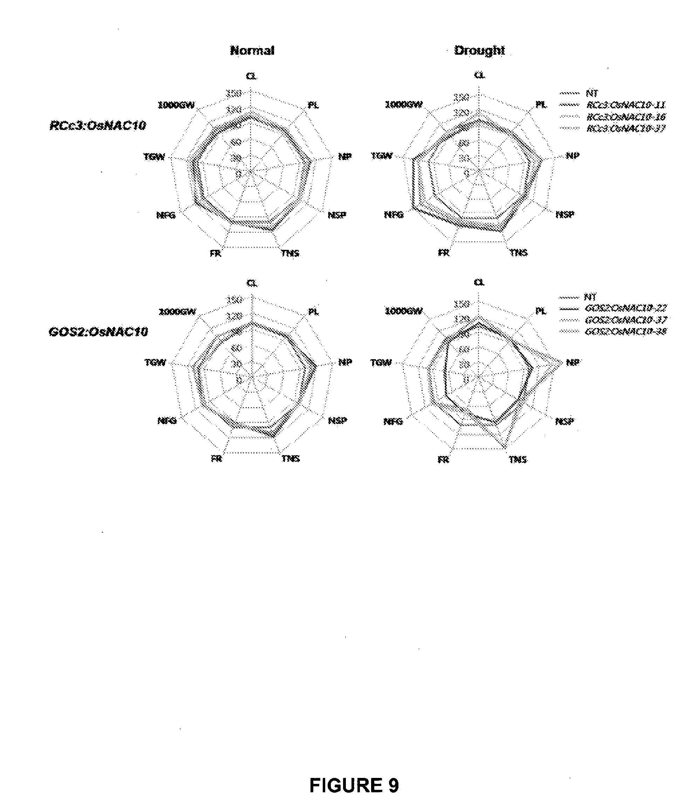

Figure 9 Agronomic Traits for RCc3:OsNAC10 and GOS2:OsNAC10 Plants Grown in the Field Under Normal and Stress Conditions. Spider plots of agronomic traits of three independent homozygous T4 lines of RCc3:OsNAC10 and GOS2:OsNAC10 plants and corresponding nontransgenic (NT) controls under normal and drought conditions were drawn by using the Microsoft Excel software. Each data point represents percentage of the mean values (n=20) listed in Supplemental Table I and II. Mean values from NT controls were set at 100% as a reference. CL, culm length; PL, panicle length; NP, number of panicles per hill; NSP, number of spikelets per panicle; TNS, total number of spikelets; FR, filling rate; NFG, number of filled grains; TGW, total grain weight; 1,000GW, thousand grain weight.

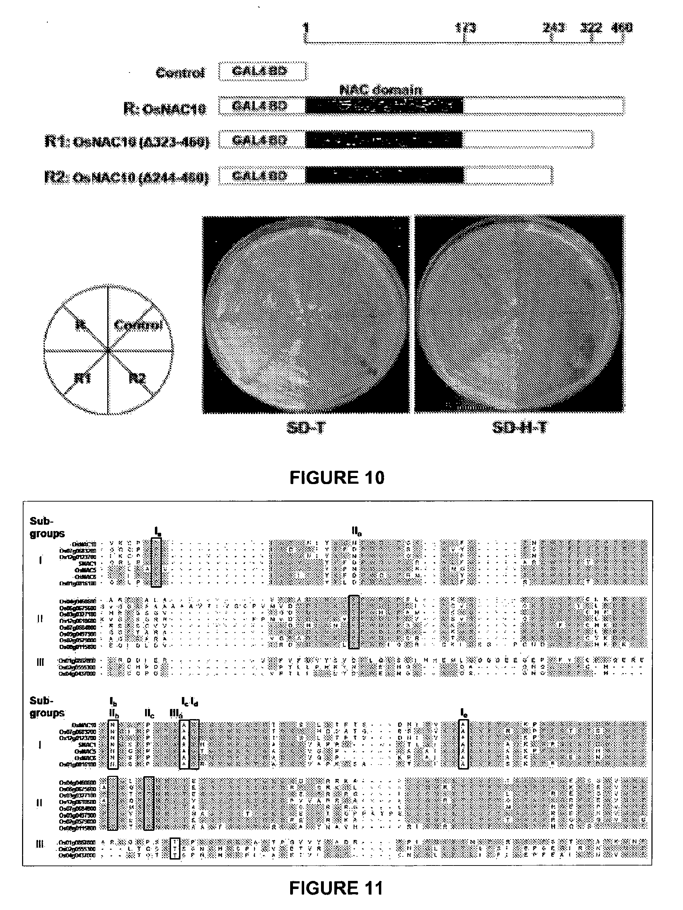

Figure 10 Transcription Activity of OsNAC10 in Yeast: A full-length cDNA of OsNAC10 and two C-terminal deletion fragments were amplified by PCR and fused to the GAL4-BD vector. Each construct was introduced into Saccharomyces cerevisiae strain AH109. R, the full- length sequence of OsNAC10; R1, C-terminal deletion sequence of OsNAC10 ($\Delta$323-460); R2, C-terminal deletion sequence of OsNAC10 ($\Delta$244-460). Filled box, NAC domain sequence of OsNAC10 (1-173); unfilled box, activation domain sequence of OsNAC10 (174-460). Transformants were spotted onto SD/-Trp and SD/-His/-Trp media and incubated at 30°C for 3days.

Figure 11 Alignments of NAC Domain Sequences from 18 Stress-Inducible Rice Genes:

Deduced amino acid sequences of the NAC domains of the 18 genes listed in Table 1 were aligned using the CLUSTAL W program. Identical and conserved residues are highlighted (gray). Signature motifs are indicated by boxes with marks on the top as follows: la-e, IIa-c, and IIIa for subgroups I-III, respectively.

Figure 12 Expression of OsNAC10 Under Stress Conditions and in Various Tissues at Different Developmental Stages of Rice:

A, Rice seeds were germinated and grown on Murashige and Skoog agar medium in the dark for 2 days (D2) and then in the light for 1 day at 28°C (L1), and then seedlings were transplanted into soil pots, and grown in the greenhouse for 11 days (14d), for until stage of meiosis (M), and for until just before the stage of heading (BH) and for until right after heading (AH). RT-PCR analyses were performed using RNAs from the indicated tissues at indicated stages of development and gene-specific primers. The expression level of a rice ubiquitin (OsUbi) was used as an internal control. L1C, coleoptiles from L1 seedlings. B, Ten $\mu$g of total RNA was prepared from leaf and root tissues of 14 d-old seedlings exposed to drought, high salinity, ABA or low temperature for indicated time points: for drought stress, the seedlings were air-dried at 28°C; for high-salinity stress, seedlings were exposed to 400 mM NaCl at 28°C; for low-temperature stress, seedlings were exposed to 4°C; for ABA, seedlings were exposed to a solution containing 100 $\mu$M ABA. Total RNAs were blotted and hybridized with the OsNAC10 gene-specific probes. The blots were then reprobed with the Dip1 (Oh et al., 2005b) gene which was used as a marker for up-regulation of key genes following stress treatments. EtBr staining was used to determine equal loading of RNAs.

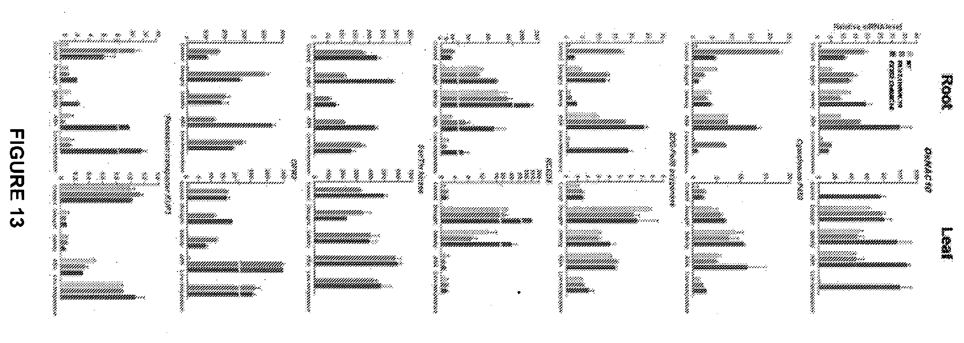

Figure 13 Regulated Genes in Roots and Leaves of RCc3:OsNAC10 and GOS2:OsNAC10 Plants under Normal and Stress Conditions. Transcript levels OsNAC10 and six target genes were determined by qRT-PCR (using primers listed in Supplemental Table S5 online). Transcript levels of the genes in RCc3:OsNAC10 and GOS2:OsNAC10 were presented as a relative value to that of untreated NT control in root and leaf, respectively. Data were normalized using the rice ubiquitin gene (OsUbi) transcript levels. Values are the means $\pm$ SD of three independent experiments.

Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

Example 1: Identification of sequences related to the nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

1.1. IAA2-like polypeptides

Table A1 provides a list of nucleic acid sequences related to the IAA2-like nucleic acid sequence useful in the methods of the present invention.

Table A1: Examples of IAA2-like polypeptides:

| Name | Nucleic acid SEQ ID NO: | Polypeptide SEQ ID NO: |
|------|------|------|
| O.sativa_Os01g0741900#1 | 1 | 2 |
| O.sativa_Os05g0523300#1 | 3 | 4 |
| corn IAA6likeACF83856.1 | 5 | 6 |
| corn_EU965307_IAA6_ACG37425 | 7 | 8 |
| H.vulgare_TC162609#1 | 9 | 10 |
| OsIBCD00325513191AUX-IAA indica | 11 | 12 |
| T.aestivum_TC289502#1 | 13 | 14 |
| T.aestivum_TC297212#1 | 15 | 16 |
| T.aestivum_TC350242#1 | 17 | 18 |
| Z.mays_TC410949#1 | 19 | 20 |

1.2. NAC10-like polypeptides

Table A2 provides a list of nucleic acid sequences related to the NAC10-like nucleic acid sequence used in the methods of the present invention.

Table A2: Examples of NAC10-like nucleic acids and polypeptides:

| Name | Nucleic acid SEQ ID NO | Polypeptide SEQ ID NO |
|---|---|---|
| OsNAC10 | 27 | 28 |
| Os03g0815100 | 29 | 30 |
| Os01g0884300 | 31 | 32 |
| Os11g0184900 | 33 | 34 |
| Os01g0136100 | 35 | 36 |
| Os01g0293600 | 37 | 38 |
| Os01g0666000 | 39 | 40 |
| Os01g0687400 | 41 | 42 |
| Os01g0700100 | 43 | 44 |
| Os01g0705200 | 45 | 46 |
| Os01g0775100 | 47 | 48 |
| Os01g0816100 | 49 | 50 |
| Os01g0845900 | 51 | 52 |
| Os01g0862800 | 53 | 54 |
| Os01g0928600 | 55 | 56 |
| Os01g0937500 | 57 | 58 |
| Os02g0111600 | 59 | 60 |
| Os02g0159800 | 61 | 62 |
| Os02g0252400 | 63 | 64 |
| Os02g0503900 | 65 | 66 |
| Os02g0555300 | 67 | 68 |
| Os02g0562600 | 69 | 70 |
| Os02g0569400 | 71 | 72 |
| Os02g0570700 | 73 | 74 |
| Os02g0579000 | 75 | 76 |
| Os02g0601500 | 77 | 78 |
| Os02g0606200 | 79 | 80 |
| Os02g0736200 | 81 | 82 |
| Os02g0759400 | 83 | 84 |
| Os02g0782500 | 85 | 86 |
| Os02g0808800 | 87 | 88 |
| Os03g0130100 | 89 | 90 |
| Os03g0132900 | 91 | 92 |
| Os03g0138700 | 93 | 94 |
| Os03g0183500 | 95 | 96 |
| Os03g0267000 | 97 | 98 |
| Os03g0269300 | 99 | 100 |

(continued)

| Name | Nucleic acid SEQ ID NO | Polypeptide SEQ ID NO |
|---|---|---|
| Os03g0327100 | 101 | 102 |
| Os03g0421000 | 103 | 104 |
| Os03g0432100 | 105 | 106 |
| Os03g0575200 | 107 | 108 |
| Os03g0584400 | 109 | 110 |
| Os03g0762000 | 111 | 112 |
| Os04g0104900 | 113 | 114 |
| Os04g0107600 | 115 | 116 |
| Os04g0206500 | 117 | 118 |
| Os04g0206600 | 119 | 120 |
| Os04g0301500 | 121 | 122 |
| Os04g0320700 | 123 | 124 |
| Os04g0339800 | 125 | 126 |
| Os04g0437000 | 127 | 128 |
| Os04g0460600 | 129 | 130 |
| Os04g0464100 | 131 | 132 |
| Os04g0469000 | 133 | 134 |
| Os04g0508500 | 135 | 136 |
| Os04g0565200 | 137 | 138 |
| Os04g0581100 | 139 | 140 |
| Os04g0583900 | 141 | 142 |
| Os04g0612700 | 143 | 144 |
| Os04g0632600 | 145 | 146 |
| Os04g0664500 | 147 | 148 |
| Os04g0675400 | 149 | 150 |
| Os05g0247800 | 151 | 152 |
| Os05g0298700 | 153 | 154 |
| Os05g0318600 | 155 | 156 |
| Os05g0376600 | 157 | 158 |
| Os05g0404700 | 159 | 160 |
| Os05g0469600 | 161 | 162 |
| Os05g0481900 | 163 | 164 |
| Os05g0542500 | 165 | 166 |
| Os05g0555600 | 167 | 168 |
| Os06g0104900 | 169 | 170 |
| Os06g0215900 | 171 | 172 |
| Os06g0317200 | 173 | 174 |
| Os06g0318600 | 175 | 176 |

(continued)

| Name | Nucleic acid SEQ ID NO | Polypeptide SEQ ID NO |
|---|---|---|
| Os06g0486400 | 177 | 178 |
| Os06g0548200 | 179 | 180 |
| Os06g0568600 | 181 | 182 |
| Os06g0671600 | 183 | 184 |
| Os06g0675600 | 185 | 186 |
| Os06g0728700 | 187 | 188 |
| Os07g0129300 | 189 | 190 |
| Os07g0129700 | 191 | 192 |
| Os07g0129800 | 193 | 194 |
| Os07g0130400 | 195 | 196 |
| Os07g0154100 | 197 | 198 |
| Os07g0171100 | 199 | 200 |
| Os07g0182000 | 201 | 202 |
| Os07g0190000 | 203 | 204 |
| Os07g0251900 | 205 | 206 |
| Os07g0262800 | 207 | 208 |
| Os07g0410700 | 209 | 210 |
| Os07g0486000 | 211 | 212 |
| Os07g0490100 | 213 | 214 |
| Os07g0558100 | 215 | 216 |
| Os07g0663700 | 217 | 218 |
| Os07g0674800 | 219 | 220 |
| Os07g0683200 | 221 | 222 |
| Os07g0684800 | 223 | 224 |
| Os07g0689600 | 225 | 226 |
| Os08g0115800 | 227 | 228 |
| Os08g0133700 | 229 | 230 |
| Os08g0203400 | 231 | 232 |
| Os08g0367000 | 233 | 234 |
| Os08g0474000 | 235 | 236 |
| Os08g0501000 | 237 | 238 |
| Os08g0518800 | 239 | 240 |
| Os08g0518900 | 241 | 242 |
| Os08g0519300 | 243 | 244 |
| Os09g0417600 | 245 | 246 |
| Os09g0417800 | 247 | 248 |
| Os09g0445700 | 249 | 250 |
| Os09g0448200 | 251 | 252 |

(continued)

| Name | Nucleic acid SEQ ID NO | Polypeptide SEQ ID NO |
|---|---|---|
| Os09g0457900 | 253 | 254 |
| Os09g0497900 | 255 | 256 |
| Os09g0507300 | 257 | 258 |
| Os09g0517200 | 259 | 260 |
| Os09g0551500 | 261 | 262 |
| Os09g0555500 | 263 | 264 |
| Os10g0134500 | 265 | 266 |
| Os10g0136500 | 267 | 268 |
| Os10g0151100 | 269 | 270 |
| Os10g0174800 | 271 | 272 |
| Os10g0204400 | 273 | 274 |
| Os10g0418100 | 275 | 276 |
| Os10g0451700 | 277 | 278 |
| Os10g0498300 | 279 | 280 |
| Os10g0505000 | 281 | 282 |
| Os10g0569600 | 283 | 284 |
| Os11g0118000 | 285 | 286 |
| Os11g0154500 | 287 | 288 |
| Os11g0282700 | 289 | 290 |
| Os11g0453900 | 291 | 292 |
| Os11g0514500 | 293 | 294 |
| Os11g0539600 | 295 | 296 |
| Os11g0567800 | 297 | 298 |
| Os11g0687100 | 299 | 300 |
| Os11g0701000 | 301 | 302 |
| Os11g0701100 | 303 | 304 |
| Os11g0701200 | 305 | 306 |
| Os11g0701400 | 307 | 308 |
| Os11g0701500 | 309 | 310 |
| Os11g0701800 | 311 | 312 |
| Os11g0701900 | 313 | 314 |
| Os11g0702200 | 315 | 316 |
| Os11g0702400 | 317 | 318 |
| Os12g0123700 | 319 | 320 |
| Os12g0197100 | 321 | 322 |
| Os12g0227500 | 323 | 324 |
| Os12g0431100 | 325 | 326 |
| Os12g0485900 | 327 | 328 |

(continued)

| Name | Nucleic acid SEQ ID NO | Polypeptide SEQ ID NO |
|---|---|---|
| Os12g0597800 | 329 | 330 |
| Os12g0610600 | 331 | 332 |
| A.cepa_CF438306 | 333 | 334 |
| Os11g03300.1 | 335 | 336 |
| Z.mays_ZM07MC20223_BFb0131A04@20172 | 337 | 338 |
| O. sativa TraitMillCDS :CDS 1 | 339 | 340 |
| O. sativa TraitMillCDS :CDS 2 | 341 | 342 |
| Z.mays_ZM07MStraceDB_BFb0239J01.f_1120947912@54470 | 343 | 344 |
| A.thaliana_AT3G04070.1 | 345 | 346 |
| A.thaliana_AT3G04070.2 | 347 | 348 |
| G.max_GM06MC20707_59724361 @20295 | 349 | 350 |
| M.truncatula_AC145753_12.5 | 351 | 352 |
| P.trichocarpa_scaff_XIII.531 | 353 | 354 |
| P.trichocarpa_scaff_XIX.359 | 355 | 356 |
| A.thaliana_AT1G69490.1 | 357 | 358 |
| G.max_GM06MC02339_48986826@2322 | 359 | 360 |
| G.max_GM06MC00030_47169159@30 | 361 | 362 |
| M.truncatula_AC140030_19.5 | 363 | 364 |
| G.max_GM06MC38096_sv19a12@37209 | 365 | 366 |
| M.truncatula_CT573505_5.4 | 367 | 368 |
| P.trichocarpa_scaff_VIII.823 | 369 | 370 |
| S.lycopersicum_TC204003 | 371 | 372 |
| S.lycopersicum_TC200098 | 373 | 374 |
| M.truncatula_AC128638_20.4 | 375 | 376 |
| M.truncatula_AC_150891_8.5 | 377 | 378 |
| OSNAC1 | 379 | 380 |
| OsNAC6 | 381 | 382 |
| OsNAC5 | 383 | 384 |
| Os12g0597800 | 385 | 386 |
| Os01g0264000 | 387 | 388 |
| Os11g0694100 | 389 | 390 |
| Os02g0530800 | 391 | 392 |
| Os05g0469800 | 393 | 394 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

Example 2: Alignment of sequences related to the polypeptide sequences used in the methods of the invention

## 2.1. IAA2-like polypeptides

Alignment of polypeptide sequences was performed using the AlignX programme from the Vector NTI (Invitrogen), which is based on the ClustalW 2.0 algorithm for progressive alignment (Thompson et al. (1997) Nucleic Acids Res 25:4876-4882; Chenna et al. (2003). Nucleic Acids Res 31:3497-3500); Alingment was performed with standard settings: gap opening penalty 10, gap extension penalty: 0.2. Minor manual editing was done to further optimise the alignment. The IAA2-like polypeptides are aligned in Figure 1.

Highly conserved amino acid residues are indicated in the consensus sequence.

## 2.2. NAC10-like polypeptides

Alignment of polypeptide sequences as shown in Figure 4 was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment. This alignment can be used for determining conserved signature sequences of about 5 to 10 amino acids in length. Preferably the conserved regions of the proteins are used, recognisable by the asterisks (identical residues), the colons (highly conserved substitutions) and the dots (conserved substitutions).

A phylogenetic tree of NAC10-like polypeptides (Figure 3) was constructed using ClustalW as described in the legend.

Example 3: Calculation of global percentage identity between polypeptide sequences useful in performing the methods of the invention

## 3.1. IAA2-like polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix.

Parameters that may be used in the comparison:

| | |
|---|---|
| Scoring matrix: | Blosum62 |
| First Gap: | 12 |
| Extending gap: | 2 |

## 3.2. NAC10-like polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were: Blosum62 (Scoring matrix), 12 (First Gap penalty), 2 (Extending gap penalty).

Results of the software analysis are shown in Table B1 for the global similarity and identity over the full length of the polypeptide sequences. Percentage identity is given above the diagonal and percentage similarity is given below the diagonal.

The percentage identity between the NAC10-like polypeptide sequences useful in performing the methods of the invention can be as low as 30% sequence identity compared to SEQ ID NO: 28.

Table B1: MatGAT results for global similarity and identity over the full length of the polypeptide sequences.

| | 1 | 2 | 3 | 4 | 5 | 6 | 7 | 8 | 9 | 10 | 11 | 12 | 13 |
|---|---|---|---|---|---|---|---|---|---|---|---|---|---|
| 1. OsNAC10_SEQID2 | | 34.9 | 99.0 | 92.0 | 47.8 | 39.1 | 40.3 | 30.7 | 38.9 | 38.0 | 39.1 | 41.5 | 41.2 |
| 2. Ac_CF438306 | 44.0 | | 35.1 | 34.8 | 29.4 | 35.8 | 36.9 | 51.7 | 41.4 | 39.8 | 39.9 | 38.4 | 39.7 |
| 3. Os11g03300 | 99.0 | 44.2 | | 91.0 | 47.0 | 39.1 | 40.3 | 30.6 | 38.9 | 37.4 | 37.9 | 41.1 | 41.3 |
| 4. Os12g0123700 | 95.9 | 43.8 | 94.9 | | 48.3 | 40.0 | 40.7 | 30.8 | 39.4 | 40.5 | 41.0 | 42.4 | 43.1 |
| 5. Zm_ZM07MC20223 | 58.3 | 37.3 | 57.5 | 59.4 | | 38.8 | 38.8 | 27.3 | 35.9 | 35.6 | 35.6 | 34.9 | 34.5 |
| 6. Os_CDS_20741_Os_NAM_CREST | 53.5 | 44.8 | 55.4 | 53.7 | 50.1 | | 97.0 | 36.5 | 51.7 | 38.4 | 39.1 | 40.5 | 39.3 |
| 7. OS_CDS_21636_CREST_control | 57.4 | 46.2 | 57.1 | 56.7 | 49.1 | 97.0 | | 37.6 | 53.2 | 38.6 | 39.2 | 41.6 | 39.9 |
| 8. Zm_ZM07MStraceDB | 38.4 | 63.9 | 38.5 | 39.2 | 33.1 | 41.3 | 42.6 | | 43.6 | 35.2 | 35.8 | 34.8 | 33.9 |
| 9. Os07g0683200 | 53.5 | 51.5 | 52.7 | 53.9 | 45.9 | 60.6 | 62.8 | 51.2 | | 40.6 | 39.9 | 42.5 | 39.6 |
| 10. AT3G04070.1 | 54.5 | 48.7 | 54.8 | 56.5 | 48.0 | 53.0 | 53.3 | 41.8 | 54.9 | | 95.5 | 49.7 | 49.6 |
| 11. AT3G04070.2 | 54.0 | 49.0 | 53.7 | 56.0 | 47.6 | 52.2 | 52.6 | 43.1 | 56.6 | 95.5 | | 51.1 | 51.0 |
| 12. Gm_GM06MC20707 | 54.7 | 47.3 | 54.8 | 55.5 | 47.6 | 52.2 | 54.6 | 41.0 | 53.8 | 65.5 | 65.8 | | 75.9 |
| 13. Mt_AC145753_12.5 | 54.0 | 47.9 | 54.0 | 54.5 | 45.9 | 52.7 | 53.6 | 41.5 | 55.3 | 66.0 | 67.9 | 85.8 | |
| 14. Pt_scaff_XIII.531 | 52.4 | 45.9 | 52.2 | 55.5 | 48.4 | 50.7 | 52.6 | 41.0 | 53.5 | 68.2 | 67.1 | 72.8 | 70.4 |
| 15. Pt_scaff_XIX.359 | 56.5 | 47.1 | 54.5 | 57.5 | 49.3 | 53.7 | 54.6 | 40.6 | 54.0 | 68.1 | 65.4 | 72.8 | 68.9 |
| 16. AT1G69490.1 | 46.5 | 64.2 | 46.8 | 46.8 | 38.7 | 45.3 | 46.4 | 53.0 | 52.6 | 50.7 | 52.2 | 49.3 | 52.9 |
| 17. Gm_GM06MC02339 | 43.5 | 68.2 | 44.7 | 42.5 | 35.8 | 42.1 | 43.9 | 61.8 | 46.5 | 47.1 | 47.8 | 45.0 | 48.8 |
| 18. Gm_GM06MC00030 | 47.6 | 58.6 | 47.8 | 47.1 | 40.6 | 47.0 | 46.9 | 51.4 | 51.8 | 51.3 | 52.5 | 51.6 | 53.5 |
| 19. Mt_AC140030_19.5 | 48.3 | 59.4 | 48.6 | 48.6 | 38.5 | 43.1 | 44.4 | 52.4 | 48.0 | 49.6 | 51.6 | 52.1 | 51.8 |
| 20. Gm_GM06MC38096 | 48.1 | 59.5 | 48.6 | 48.3 | 41.1 | 47.0 | 49.5 | 49.5 | 50.9 | 52.4 | 54.2 | 51.6 | 52.6 |
| 21. Mt_CT573505_5.4 | 47.1 | 62.7 | 47.3 | 46.3 | 39.4 | 44.1 | 45.7 | 51.5 | 50.3 | 50.1 | 51.9 | 50.7 | 50.9 |
| 22. Pt_scaff_VIII.823 | 47.8 | 56.9 | 47.0 | 48.3 | 40.2 | 45.8 | 47.2 | 51.9 | 51.5 | 52.1 | 53.6 | 51.0 | 50.6 |
| 23. SI_TC204003 | 48.3 | 60.3 | 48.8 | 49.6 | 42.9 | 46.3 | 47.7 | 50.4 | 52.9 | 52.1 | 53.6 | 51.0 | 53.5 |
| 24. SI_TC200098 | 48.8 | 60.0 | 49.1 | 48.3 | 39.4 | 47.8 | 49.0 | 53.1 | 51.8 | 50.7 | 52.5 | 53.6 | 53.2 |
| 25. Mt_AC128638_20.4 | 49.6 | 51.8 | 50.6 | 49.9 | 42.1 | 49.5 | 50.3 | 43.6 | 53.2 | 57.1 | 58.9 | 52.7 | 56.8 |

| | 14 | 15 | 16 | 17 | 18 | 19 | 20 | 21 | 22 | 23 | 24 | 25 |
|---|---|---|---|---|---|---|---|---|---|---|---|---|
| 1. OsNAC10_SEQID2 | 40.1 | 41.6 | 37.3 | 36.3 | 36.7 | 40.7 | 38.6 | 37.9 | 39.8 | 38.4 | 38.4 | 36.9 |
| 2. Ac_CF438306 | 38.6 | 39.2 | 47.7 | 52.2 | 46.9 | 46.6 | 45.3 | 48.2 | 45.2 | 47.1 | 46.3 | 39.0 |
| 3. Os11g03300 | 39.5 | 40.5 | 37.5 | 36.1 | 37.1 | 40.4 | 38.3 | 37.5 | 39.2 | 39.5 | 38.2 | 36.8 |
| 4. Os12g0123700 | 42.0 | 42.4 | 38.1 | 35.6 | 36.6 | 40.2 | 37.3 | 37.7 | 40.6 | 40.7 | 37.9 | 37.2 |
| 5. Zm_ZM07MC20223 | 35.8 | 36.7 | 31.6 | 30.1 | 32.0 | 31.4 | 32.6 | 31.6 | 32.4 | 34.9 | 29.7 | 31.4 |
| 6. Os_CDS_20741_Os_NAM_CREST | 40.0 | 41.5 | 36.7 | 34.3 | 36.3 | 33.9 | 35.1 | 35.6 | 37.0 | 34.9 | 37.0 | 35.4 |
| 7. OS_CDS_21636_CREST_control | 40.4 | 42.1 | 37.8 | 35.4 | 36.4 | 34.2 | 36.2 | 36.5 | 37.6 | 36.0 | 37.9 | 35.9 |
| 8. Zm_ZM07MStraceDB | 33.9 | 34.0 | 41.4 | 45.7 | 38.3 | 39.5 | 37.6 | 38.3 | 39.9 | 38.2 | 39.6 | 31.3 |
| 9. Os07g0683200 | 41.8 | 41.4 | 41.5 | 37.2 | 40.4 | 37.2 | 39.8 | 39.0 | 41.6 | 41.1 | 39.2 | 37.5 |
| 10. AT3G04070.1 | 53.4 | 53.1 | 39.0 | 37.3 | 39.6 | 39.8 | 39.8 | 38.7 | 39.3 | 40.9 | 38.4 | 39.7 |
| 11. AT3G04070.2 | 53.6 | 53.5 | 39.9 | 37.9 | 39.9 | 41.1 | 40.5 | 39.7 | 40.2 | 42.0 | 39.5 | 41.0 |
| 12. Gm_GM06MC20707 | 59.9 | 60.4 | 38.4 | 38.0 | 41.0 | 40.6 | 40.2 | 38.7 | 40.5 | 41.8 | 41.0 | 39.5 |
| 13. Mt_AC145753_12.5 | 58.1 | 58.2 | 42.3 | 40.3 | 42.0 | 41.1 | 41.3 | 41.6 | 41.8 | 42.3 | 42.7 | 41.2 |
| 14. Pt_scaff_XIII.531 | | 79.8 | 38.3 | 35.9 | 39.8 | 39.8 | 39.7 | 38.9 | 38.6 | 40.1 | 38.9 | 40.3 |
| 15. Pt_scaff_XIX.359 | 87.2 | | 40.3 | 36.9 | 37.8 | 41.3 | 40.4 | 39.6 | 40.9 | 42.7 | 40.9 | 38.0 |
| 16. AT1G69490.1 | 50.5 | 49.9 | | 59.1 | 55.7 | 55.2 | 55.3 | 56.5 | 58.6 | 52.9 | 56.3 | 39.3 |
| 17. Gm_GM06MC02339 | 45.1 | 45.0 | 69.4 | | 78.9 | 66.2 | 64.1 | 65.3 | 58.3 | 53.7 | 56.0 | 39.3 |
| 18. Gm_GM06MC00030 | 50.8 | 49.9 | 71.4 | 80.7 | | 73.0 | 72.8 | 65.8 | 62.0 | 54.9 | 55.6 | 41.8 |
| 19. Mt_AC140030_19.5 | 51.4 | 49.9 | 69.0 | 73.1 | 81.4 | | 66.3 | 66.1 | 58.8 | 55.8 | 53.7 | 42.7 |
| 20. Gm_GM06MC38096 | 51.4 | 51.8 | 70.6 | 74.2 | 83.9 | 81.4 | | 77.1 | 65.7 | 55.8 | 59.7 | 40.5 |
| 21. Mt_CT573505_5.4 | 48.9 | 49.6 | 71.6 | 76.9 | 76.8 | 78.2 | 85.3 | | 60.2 | 54.8 | 60.8 | 40.9 |
| 22. Pt_scaff_VIII.823 | 50.3 | 51.2 | 70.7 | 68.2 | 74.9 | 71.0 | 78.4 | 74.6 | | 61.7 | 60.8 | 41.8 |
| 23. SI_TC204003 | 50.5 | 53.1 | 69.9 | 66.3 | 72.0 | 71.6 | 69.5 | 70.6 | 74.6 | | 67.4 | 39.9 |
| 24. SI_TC200098 | 52.7 | 51.5 | 70.5 | 67.6 | 70.7 | 72.0 | 74.9 | 73.5 | 72.8 | 77.0 | | 39.6 |
| 25. Mt_AC128638_20.4 | 54.3 | 52.9 | 53.4 | 50.9 | 56.4 | 53.7 | 53.0 | 53.7 | 54.3 | 53.0 | 54.9 | |

Example 4: Identification of domains comprised in polypeptide sequences useful in performing the methods of the invention

4.1. IAA2-like polypeptides

The presence of conserved protein domains in SEQ ID NO: 2 was determined by searching the pfam database. Pfam is a large collection of multiple sequence alignments and hidden Markov models covering many common protein domains and families. Pfam is hosted at the Sanger Institute server in the United Kingdom.
The results of the search of the Pfam with the query sequence as represented by SEQ ID NO: 2 are presented in Table C1.

Table C1: Pfam search results (major accession numbers) of the polypeptide sequence as represented by SEQ ID NO: 2.

| | Description | Entry type | Sequence (amino acid coordinates of the domain in the query sequence) | | E-value | Alignment mode |
|---|---|---|---|---|---|---|
| | | | Start | End | | |
| Pfam-A | AUX/IAA family | Family | 49 | 335 | 3E-13 | ls |

The Alignment mode used for PF2309 is the so called "ls". Parameters used in the model are given in Table C2.

Table C2:

| Is model: hmmbuild -F HMM_Is SEED hmmcalibrate --cpu 1 --seed 0 HMM_Is | | |
|---|---|---|
| Parameter | Is | |
| | Sequence | Domain |
| Gathering cut-off | -83 | -83 |
| Trusted cut-off | -82 | -82 |
| Noise cut-off | -83.5 | -83.5 |

4.2. NAC10-like polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 28 are presented in Table C3.

Table C3: InterPro scan results (major accession numbers) of the polypeptide sequence as represented by SEQ ID NO: 28.

| Database | Accession number | Accession name | Amino acid coordinates on SEQ ID NO: 28 |
|---|---|---|---|
| PFAM | PF02365 | NAM | 11-139 |
| PROFILE | PS51005 | NAC | 11-172 |
| SUPERFAMILY | SSF101941 | NAM | 7-173 |

Example 5: Topology prediction of the polypeptide sequences useful in performing the methods of the invention

5.1. IAA2-like polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)

- PLOC (Park and Kanehisa, Bioinformatics, 19, 1656-1663, 2003).

5.2. NAC10-like polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 28 are presented Table D1. The "plant" organism group has been selected, no cutoffs defined, and the predicted length of the transit peptide requested. The subcellular localization of the polypeptide sequence as represented by SEQ ID NO: 28 may be the cytoplasm or nucleus, no transit peptide is predicted.

Table D1: TargetP 1.1 analysis of the polypeptide sequence as represented by SEQ ID NO: 28. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|------|-----|-----|-----|-----|-------|-----|-----|-------|
| SEQ ID NO:28 | 393 | 0.056 | 0.204 | 0.167 | 0.848 | _ | 2 | - |
| cutoff | | 0.000 | 0.000 | 0.000 | 0.000 | | | |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

Example 6: Cloning of the nucleic acid sequence used in the methods of the invention and rice transformation

6.1. IAA2-like polypeptides

The nucleic acid having the sequence of SEQ ID NO: 1 was isolated using routine molecular biology techniques from rice and cloned into an entry vector of the Gateway technology (Invitrogen).

The entry clone comprising SEQ ID NO: 1 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 26) for constitutive specific expression was located upstream of this Gateway cassette.

After the LR recombination step, the resulting expression vector pGOS2::IAA2-like (Figure 2) was transformed into Agrobacterium strain LBA4044 according to methods well known in the art.

6.2. NAC10-like polypeptides

In one experiment, the following transformation method was used:

The coding region of OsNAC10 was amplified from rice total RNA using an RT-PCR system (Promega, WI), according to the manufacturer's instructions. Primer pairs were as follows:

Forward: (5'-ATGCCGAGCAGCGGCGGCGC-3') (primer A, SEQ ID NO: 384)
Reverse: (5-CTACTGCATCTGCAGATGAT-3') (primer B, SEQ ID NO: 385).

To enable the overexpression of the OsNAC10 gene in rice, isolated cDNA was linked to the GOS2 promoter for constitutive expression (de Pater et al., 1992), and the RCc3 promoter for root specific expression (Xu et al., 1995) respectively, using the Gateway system (Invitrogen, Carlsbad, CA). Plasmids were introduced into Agrobacterium tumefaciens LBA4404 by triparental mating and embryogenic (Oryza sativa cv Nipponbare) calli from mature seeds were transformed as previously described (Jang et al., 1999).

To enable the overexpression of the OsNAC10 genes in rice, their full-length cDNAs were isolated and linked to the RCc3 promoter (Xu et al., 1995) for root-specific expression, and the GOS2 promoter (de Pater et al., 1992) for constitutive expression, generating the constructs RCc3::OsNAC10 and GOS2::OsNAC10. These constructs were then introduced into rice by Agrobacterium-mediated transformation (Hiei et al., 1994), which yielded 15 to 20 independent transgenic lines per construct. Transgenic T1-4 seeds were collected and three independent T4 homozygous lines of both RCc3::OsNAC10 and GOS2::OsNAC10 plants were selected for further analysis. All of the transgenic lines grew normally with no stunting. The transcript levels of OsNAC10 in the RCc3::OsNAC10 and GOS2::OsNAC10 plants were determined by RNA-gel blot analysis. For this purpose, total RNAs were extracted from leaf and root tissues of 14-day-old seedlings grown under normal growth conditions (Figure 4). The OsNAC10 was clearly detectable in all the transgenic lines but not detected in the nontransgenic (NT) controls. Transgene was expressed at high levels in leaves and roots of the GOS2::OsNAC10 plants and in roots of the RCc3::OsNAC10 plants, but not in leaves of the latter, verifying the constitutive and root-specific nature of the promoters.

In another experiment, plasmids were constructed and plants were transformed as follows:

The nucleic acid sequence used in the methods of the invention was amplified by PCR using as template a custom-made Oryza sativa seedlings cDNA library (in pCMV Sport 6.0; Invitrogen, Paisley, UK). PCR was performed using Hifi Taq DNA polymerase in standard conditions, using 200 ng of template in a 50 $\mu$l PCR mix. The primers used were prm08754 (SEQ ID NO: 386; sense, start codon in bold): 5'-ggggacaagtttgtacaaaaaagcaggcttaaacaatg ccgagcagcg-3' and prm08755 (SEQ ID NO: 387; reverse, complementary): 5'-ggggaccactttg tacaagaaagctgggtctactgcatctgcagatga-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pNAC10. Plasmid pDONR201 is purchased from Invitrogen, as part of the Gateway® technology.

The entry clone comprising SEQ ID NO: 27 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice RCc3 promoter (SEQ ID NO: 383) for root-specific expression was located upstream of this Gateway cassette.

After the LR recombination step, the resulting expression vector pRCc3::NAC10 is transformed into Agrobacterium strain LBA4044 according to methods well known in the art.

Example 8: Plant transformation of other crops

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2% HgCl2, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

Agrobacterium strain LBA4404 containing the expression vector was used for co-cultivation. Agrobacterium was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 8: Transformation of other crops

Corn transformation

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse. T1 seeds are produced from plants that exhibit tolerance to the selection agent and that contain a single copy of the T-DNA insert.

Cotton transformation

Cotton is transformed using Agrobacterium tumefaciens according to the method described in US 5,159,135. Cotton seeds are surface sterilised in 3% sodium hypochlorite solution during 20 minutes and washed in distilled water with 500 $\mu$g/ml cefotaxime. The seeds are then transferred to SH-medium with 50$\mu$g/ml benomyl for germination. Hypocotyls of 4 to 6 days old seedlings are removed, cut into 0.5 cm pieces and are placed on 0.8% agar. An Agrobacterium suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 $\mu$g/ml MgCL2, and with 50 to 100 $\mu$g/ml cefotaxime and 400-500 $\mu$g/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

Example 9: Phenotypic evaluation procedure

9.1. IAA2-like polypeptides

9.1.1. Evaluation setup

Approximately 35 independent T0 rice transformants were generated. The primary transformants were transferred from a tissue culture chamber to a greenhouse for growing and harvest of T1 seed. Events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development. From the stage of sowing until the stage of maturity the plants were passed several times through a digital imaging cabinet. At each time point digital images (2048x1536 pixels, 16 million colours) were taken of each plant from at least 6 different angles.

Drought screen

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

Nitrogen use efficiency screen

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Salt stress screen

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.1.2. Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

9.1.3. Parameters measured

Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

9.2. NAC10-like polypeptides

In a first set of experiments, the following tests were performed:

Drought Treatments at the Vegetative Stage

Transgenic and nontransgenic (NT) rice (Oryza sativa cv Nipponbare) seeds were germinated in half-strength MS solid medium in a growth chamber in the dark at 28°C for 4 days, transplanted into soil and then grown in a greenhouse (16-h-light/8-h-dark cycles) at 28-30°C. Eighteen seedlings from each transgenic and nontransgenic line were grown in pots (3x3x5 cm; 1 plant per pot) for 4 weeks before undertaking the drought-stress experiments. To induce drought stress, 4-week-old transgenic and NT seedlings were withheld from watering for 3 days followed by 7 days of watering. The numbers of plants that survived or continued to grow were then scored.

Chlorophyll Fluorescence Under Conditions of Drought, High-Salinity and Low Temperature

Transgenic and nontransgenic rice (Oryza sativa cv Nipponbare) seeds were germinated and grown in half-strength MS solid medium for 14 days in a growth chamber (16-h-light of 150 $\mu$mol m$^{-2}$ s$^{-1}$ /8-h-dark cycles at 28°C). The green portions of approximately 10 seedlings were cut using a scissors prior to stress treatments in vitro. To induce low-temperature stress, the seedlings were incubated at 4°C in water for up to 6 h under continuous 150 $\mu$mol m$^{-2}$ s$^{-1}$ light. For high-salinity stress treatments, they were incubated in 400 mM NaCl for 2 h at 28°C under continuous 150 $\mu$mol m$^{-2}$ s$^{-1}$ and to simulate drought stress they were air-dried for 2 h at 28°C under continuous 150 $\mu$mol m$^{-2}$ s$^{-1}$ light. The Fv/Fm values were then measured as previously described (Oh et al., 2005b).

Drought Treatments and Grain Yield Analysis of Rice Plants Grown in the Field

To evaluate yield components of transgenic plants under normal field conditions, three independent T4 homozygous lines of the RCc3::OsNAC10 and GOS2::OsNAC10 plants, together with nontransgenic (NT) controls were transplanted to a paddy field at the Rural Development Administration, Suwon, Korea. A completely randomized design was employed with two replicates, each consisting of 4 plots of 5 m2. At 25 d after sowing, the seedlings were randomly transplanted within a 15 X 30 cm distance. Fertilizer was applied at 70N/40P/70K kg ha-1 after the last paddling and 45 d after transplantation. Yield parameters were scored for 10 plants per plot, and 20 plants per line.

To evaluate yield components of transgenic plants under drought field conditions, three independent T4 homozygous lines of the RCc3:OsNAC10 and GOS2:OsNAC10 plants, and NT controls were transplanted to a removable rain-off shelter with a 1 meter deep container filled with natural paddy soil and located at Myongji University, Yongin, Korea. A completely randomized design, transplanting distance and use of fertilizer was employed as described above for normal field conditions. Drought stress was applied at the panicle heading stage (from 10-d before heading to 20-d after heading) by flowing water through a drain at the bottom of the container. To prevent plants from dying, we irrigated twice when the plant leaves rolled during drought stress. After exposure to drought stress conditions, the polyvinyl roofs were removed and plants were irrigated until harvesting.

When the plants grown under normal and drought conditions had reached maturity and the grains had ripened, they were harvested and threshed by hand (separation of seeds from the vegetative parts). The unfilled and filled grains were taken apart, independently counted using a Countmate MC1000H (Prince Ltd, Korea), and weighed. The following agronomic traits were scored: flowering date, panicle length, number of tillers, number of panicles, spikelets per panicle, filling rate (%), total grain weight (g), and 1,000 grain weight (g). The results from three independent lines were separately analyzed by one way ANOVA and compared with those of the NT controls. The ANOVA was used to reject the null hypothesis of equal means of transgenic lines and NT controls (P<0.05). The SPSS version 16.0 was used to perform statistical analysis.

In another set, the following experiments were performed:

9.2.1. Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

Four T1 events are further evaluated in the T2 generation following the same evaluation procedure as for the T1 generation but with more individuals per event.

From the stage of sowing until the stage of maturity the plants were passed several times through a digital imaging cabinet. At each time point digital images (2048x1536 pixels, 16 million colours) were taken of each plant from at least 6 different angles.

Drought screen

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC goes below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters were recorded as detailed for growth under normal conditions.

Nitrogen use efficiency screen

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Salt stress screen

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.2.2. Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

When two experiments with overlapping events are carried out, a combined analysis is performed. This is useful to check consistency of the effects over the two experiments, and if this is the case, to accumulate evidence from both experiments in order to increase confidence in the conclusion. The method used is a mixed-model approach that takes into account the multilevel structure of the data (i.e. experiment - event - segregants). P values are obtained by comparing likelihood ratio test to chi square distributions.

9.2.3. Parameters measured

Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Examples 10: Results of the phenotypic evaluation of the transgenic plants

10.1. IAA2-like polypeptides

The results of the evaluation of transgenic rice plants in the T2 generation and expressing a nucleic acid comprising the longest Open Reading Frame in SEQ ID NO: 1 under non-stress conditions are presented below. See previous Examples for details on the generations of the transgenic plants.

The results of the evaluation of transgenic rice plants under non-stress conditions are presented below (Table E1). An increase of at least 5 % was observed for seed fill rate (fillrate) and harvest index (harvestindex), and of 2.5% for thousand kernel weight (TKW).

Table E1

| Yield-related trait | Percentage increase in transgenic plants compared to control plants |
|---|---|
| fillrate | 16.3 |
| TKW | 3 |
| harvestindex | 11.1 |

10.2. NAC10-like polypeptides

Results of the first set of experiments:

Stress Tolerance of RCc3::OsNAC10 and GOS2::OsNAC10 Plants at the Vegetative Stage

To investigate whether the overexpression of OsNAC10 correlated with stress tolerance in rice, four-week-old transgenic plants and NT controls were exposed to drought stress (Fig. 5). The NT plants started to show visual symptoms of drought-induced damage, such as leaf rolling and wilting with a concomitant loss of chlorophylls, at an earlier stage than the RCc3::OsNAC10 and GOS2::OsNAC10 plants. The transgenic plants also recovered faster than the NT plants upon re-watering. Consequently, the NT plants remained severely affected by the time at which some of the transgenic lines had partially recovered (Figure 7). To further verify the stress-tolerance phenotype, we measured the Fv/Fm values of the transgenic and NT control plants, all at the vegetative stage (Figure 8). The Fv/Fm values represent the maximum photochemical efficiency of PS II in a dark-adapted state (Fv, variable fluorescence; Fm, maximum fluorescence). The Fv/Fm levels were 15 to 30% higher in the RCc3::OsNAC10 and GOS2::OsNAC10 plants than in the NT plants under drought and low temperature conditions. Under moderate salinity conditions, the Fv/Fm levels were 10% higher in both transgenic plants over NT controls whereas, under severe salinity conditions, the levels remained similar to those of NT plants. Hence, our results indicate that the overexpression of OsNAC10 in transgenic rice increases the tolerance of these plants primarily to drought and low temperature stress conditions during the vegetative stage.

Overexpression of OsNAC10 in Roots Increases Rice Grain Yield Under Drought Conditions

A phenotypic evaluation of both RCc3::OsNAC10 and NT control plants revealed no major differences in the vegetative growth of the entire plants. To investigate whether the overexpression of OsNAC10 increase grain yield of transgenic rice plants under field conditions, we transplanted three independent T4 homozygous lines of the RCc3::OsNAC10 and nontransgenic (NT) controls, to a paddy field and grew them to maturity. A completely randomized design with two replicates was employed. In the RCc3::OsNAC10 plants under normal field conditions, however, total grain weight was increased by 5-14% compared with the NT controls, which appears to be due to increased number of filled grains (Figure 9, Table F). These observations prompted us to examine the yield components of the transgenic rice plants grown in the field drought conditions. Three independent lines of the RCc3::OsNAC10 and NT controls were transplanted to a paddy field with a removable rain-off shelter, and exposed to drought stress at the panicle heading stage (from 10-d before heading to 20-d after heading). Statistical analysis of the yield parameters showed that the decrease in grain yield under drought conditions was significantly smaller in the RCc3::OsNAC10 plants than that observed in the NT controls. Specifically, in the drought-treated RCc3:OsNAC10 plants, the number of filled grains was higher than the drought-treated NT plants by 22-47%, which resulted in an increase in the total grain weight by 19-29%, depending on the transgenic line (Figure 9 and Table G). Overall, our results suggest that the overexpression of OsNAC10 in roots gave a higher drought tolerance of transgenic rice at the reproductive stage, increasing grain yield significantly.

Table F: Agronomic Traits of the RCc3:OsNAC10 and GOS2:OsNAC10 Transgenic Rice Plants under Normal Field Conditions. *, The mean difference is significant at the 0.05 level (LSD).

| Construct | Culm length (cm) | Panicle length (cm) | No. of Panicles (/hill) | No. of Spikelets (/panicle) | No. of Spikelets (/hill) | Fill rate (%) | No. of filled Spikelets (/hill) | Total grain weight (g) | 1000 grain weight (g) |
|---|---|---|---|---|---|---|---|---|---|
| NT | 71.73 | 19.38 | 11.50 | 94.67 | 1084.15 | 89.91 | 975.90 | 24.33 | 22.42 |
| RCc3:OsNAC10-11 | 72.03 | 18.70 | 12.65* | 99.21* | 1253.60* | 91.07 | 1136.35* | 26.32* | 21.14* |
| %Δ | 0.42 | -3.48 | 10.0 | 04.80 | 15.63 | 1.28 | 16.44 | 8.16 | -5.69 |
| RCc3:OsNAC10-16 | 79.00* | 19.60 | 12.20 | 98.68 | 1200.20* | 91.84 | 1100.10* | 27.84* | 23.24 |
| %Δ | 10.14 | 1.16 | 6.09 | 4.24 | 10.70 | 2.14 | 12.7 | 314.43 | 3.68 |
| RCc3:OsNAC10-37 | 74.15* | 18.45* | 11.50 | 95.16 | 1092.80 | 92.24 | 1008.55 | 25.62 | 23.45* |
| %Δ | 3.38 | -4.77 | 0.00 | 0.52 | 0.80 | 2.59 | 3.35 | 5.28 | 4.61 |

Table G: Agronomic Traits of the RCc3:OsNAC10 and GOS2:OsNAC10 Transgenic Rice Plants under Field Drought Conditions. *, The mean difference is significant at the 0.05 level (LSD).

| Construct | Culm length (cm) | Panicle length (cm) | No. of Panicles (/ hill) | No.of Spikelets (/ panicle) | No. of total spikelets (/ hill) | Filling rate (%) | No. of filled spikelets (/ hill) | Total grainweight (g) | 1000 grainweight (g) |
|---|---|---|---|---|---|---|---|---|---|
| NT | 54.10 | 19.30 | 10.00 | 82.10 | 820.00 | 71.76 | 588.80 | 12.84 | 21.84 |
| RCc3:OsNAC10-11 | 53.60 | 18.20* | 12.40 | 84.59 | 1041.60* | 82.72* | 869.00* | 16.68* | 19.20* |
| %Δ | -0.92 | -5.70 | 24.0 | 03.04 | 27.02 | 15.27 | 47.59 | 29.91 | -12.11 |
| RCc3:OsNAC10-16 | 61.20* | 18.20* | 11.00 | 88.00 | 947.80 | 76.14 | 718.20 | 15.38 | 21.43 |
| %Δ | 13.12 | -5.70 | 10.00 | 7.19 | 15.59 | 6.10 | 21.98 | 19.78 | -1.89 |
| RCc3:OsNAC10-37 | 52.00 | 19.20 | 12.20 | 80.29 | 979.60 | 79.49 | 773.40* | 15.421 | 9.95* |
| %Δ | -3.88 | -0.52 | 22.00 | -2.20 | 19.46 | 10.78 | 31.35 | 20.09 | -8.66 |
| NT | 54.10 | 19.30 | 10.00 | 82.10 | 820.00 | 71.76 | 588.80 | 12.84 | 21.84 |
| GOS2:OsNAC10-22 | 58.10 | 18.50 | 10.60 | 73.01 | 774.80 | 53.99 | 431.20 | 8.62 | 20.17* |
| %Δ | 7.39 | -4.15 | 6.00 | -11.07 | -5.51 | -24.77 | -26.77 | -32.87 | -7.68 |
| GOS2:OsNAC10-37 | 65.70* | 18.90 | 14.60* | 82.66 | 1186.80* | 53.78 | 624.00 | 12.84 | 20.50* |
| %Δ | 21.44 | -2.07 | 46.00 | 0.68 | 44.73 | -25.05 | 5.98 | 0.00 | -6.17 |
| GOS2:OsNAC10-38 | 64.70* | 18.30 | 16.40* | 76.10 | 1219.00* | 48.14* | 594.40 | 12.54 | 20.97 |
| %Δ | 19.59 | -5.18 | 64.00 | -7.30 | 48.66 | -32.92 | 0.95 | -2.34 | -4.02 |

In the second set of experiments, an increase in seed yield was observed for plants transformed with SEQ ID NO: 28 under control of the RCc3 promoters. Three out of six tested lines had an increased fill rate, with an overall increase of 14.6% (p-value 0.0304).

Example 11: Transcriptional Activation Test in Yeast

11.1. NAC10-like polypeptides

To examine the transcriptional activity of OsNAC10 and C-terminal deletion mutants thereof a yeast transcriptional activation assay was performed. Full-length and C-terminal deletion mutants of OsNAC10 were amplified by PCR and inserted into the pGBKT7 vector (Clontech, Palo Alto, CA) generating a fusion with the GAL4 DNA-binding domain (BD), as described previously (Jeong et al., 2009). All the constructs were then verified by DNA sequence analysis. The constructs were introduced into Saccharomyces cerevisiae strain AH109 (Clontech) and transformed cells were grown on SD/-Trp medium for 3 days. Transformed colonies were plated onto SD/-Trp and SD/-Trp/-His medium to test for transcriptional activation.

GAL4 DNA binding domain-OsNAC10 fusion proteins were expressed in yeast cells, which were assayed for their ability to activate transcription from the GAL4 binding sequence. As shown in Figure 10, OsNAC10 and OsNAC10 ($\Delta$323-460) promoted yeast growth in the absence of histidine that was abolished upon a further deletion of C-terminal region to the position 244, demonstrating that OsNAC10 functions as a transcriptional activator.

Example 12: OsNAC10 is induced by stress

12.1. NAC10-like polypeptides

Rice 3'-Tiling Microarray Analysis

Expression profiling was conducted using the Rice 3"-Tiling Microarray, manufactured by NimbleGen Inc. (http://www.nimblegen.com), which contains 27,448 genes deposited at the IRGSP, RAP1 database (http://rapdb.lab.nig.ac.jp). Further information on this microarray including statistical analysis can be found at http://www.ggbio.com (GreenGene Biotech). Among the genes on the microarray, 20,507 are from representative RAP1 sequences with cDNA/EST supports and 6,941 genes have been predicted without cDNA/EST supports. Ten 60-nt long probes were designed from each gene starting at 60 bp ahead of the stop codon and with 10 bp shifts in position so that 10 probes covered 150 bp within the 3' region of the gene. In total, 270,000 probes were designed in this way (average size, 60-nt) to have Tm values of between 75°C and 85°C. Random GC probes (38,000) were used to monitor the hybridization efficiency and fiducial markers at the four corners (225) were included to assist with overlaying of the grid on the image. To identify stress-inducible NAC genes in rice, total RNA (100 $\mu$g) was prepared from 14-d-old rice (Oryza sativa cv Nipponbare) leaves of plants subjected to drought, high-salinity, ABA, and low-temperature stress conditions. For the high salinity and ABA treatments, the 14-d-old seedlings were transferred to a nutrient solution containing 400 mM NaCl or 100 $\mu$M ABA for 2 h in the greenhouse under continuous light of approximately 1000 $\mu$mol m-2 s -1. For drought treatment, 14-d-old seedlings were air-dried for 2 h under continuous light of approximately 1000 $\mu$mol m-2 s -1. For low temperature treatments, 14-d-old seedlings were exposed at 4°C in a cold chamber for 6 h under continuous light of 150 $\mu$mol m-2 s -1. For identification of genes up-regulated in RCc3:OsNAC10, GOS2:OsNAC10 plants, total RNA (100 $\mu$g) was prepared from root tissues of 14-d-old transgenic and nontransgenic rice (Oryza sativa cv Nipponbare) seedlings grown under normal growth conditions. The mRNA was purified using the Qiagen oligotex column (Qiagen, Valencia, CA), according to the manufacturer"s instructions. For normalization, data were processed with cubic alpine normalization using quartiles to adjust signal variation between chips, and with Robust Multi-Chip Analysis using a median polish algorithm implemented in NimbleScan (Workman et al., 2002; Irizarry et al., 2003). To assess the reproducibility of the microarray analysis, we repeated the experiments three times with independently prepared total RNAs and analyzed statistically of each data set using one-way ANOVA.

RT-PCR and qPCR Analysis

Total RNA was prepared as previously reported (Oh et al., 2008). For the analysis of genes by RT-PCR, a cDNA synthesis system (Invitrogen, Carlsbad, CA) was used according to the manufacturer"s instructions. PCR products were amplified using primers designed with Primer Designer 4 software (Sci-ed Software, Durham, NC). RT-PCR was carried out using the primer pairs at a final concentration of 10 pM each and 2 $\mu$L (equivalent to 5 ng total RNA) of cDNA as the template. PCR was performed at 95°C for 10 min, followed by 25 to 29 cycles of at 94°C for 30 s, 57°C for 30 s and 68°C for 1 min. Amplified products were resolved on a 2% agarose gel. To validate our RT-PCR results, we repeated each experiment twice with independently prepared total RNA.

For quantitative real-time PCR experiments, SuperScriptTM III Platinum® One-Step Quantitative RT-PCR system (Invitrogen, Carlsbad, CA) was used. For PCR reactions, a master mix of reaction components was prepared, according to the manufacturer"s protocol for Platinum® SYBR® Green qPCR SuperMix-UDG (Invitrogen, Carlsbad, CA). Thermocycling and fluorescence detection were performed using a Stratagene Mx3000p Real-Time PCR machine (Stratagene, La Jolla, CA). PCR was performed at 95°C for 10 min, followed by 40 cycles of at 94°C for 30 s, 57°C for 30 s and 68°C for 1 min. To validate our qPCR results, we repeated each experiment three times.

Northern blot analysis

Rice (Oryza sativa cv Nipponbare) seeds were germinated in soil and grown in the glasshouse (16 h light/8 h dark cycle) at 22 °C. For high-salinity and ABA treatments, 14-day-old seedlings were transferred to nutrient solution containing 400 mM NaCl or 100 $\mu$M ABA for the indicated time course in the glasshouse under continuous light of approximately 1000 $\mu$mol/m2/s. For drought treatment, 14-day-old seedlings were excised and air dried for the indicated time course under continuous light of approximately 1000 $\mu$mol/m2/s. For low-temperature treatments, 14-day-old seedlings were exposed at 4 °C in a cold chamber for the indicated time course under continuous light of 150 $\mu$mol/m2/s. The preparation of total RNA and RNA gel-blot analysis were performed as reported previously (Jang et al., 2002).

To identify stress-inducible OsNAC genes, we performed expression profiling with the Rice 3'-Tiling microarray (GreenGene Biotech, Yongin, Korea) using RNAs from 14-d-old rice leaves of seedlings subjected to drought, high-salinity, ABA, and low-temperature. When three replicates were averaged and compared with untreated leaves, a total of 18 OsNAC genes were found to be up-regulated by 1.9-fold or greater (P<0.05) by one or more stress conditions (Table H). Phylogenic analysis of the amino acid sequences of 18 OsNACs revealed the presence of 3 subgroups (I to III) (Figure 11). Comparison of the amino acid sequence spanning the NAC domain identified signature motifs by which these subgroups can be distinguished (Fig. 9). For example, signature motifs la-e, IIa-c, and IIIa are specific to subgroups I, II and III, respectively. In addition to sequence similarity, members of each subgroup are closely related in terms of their response to stress. For example, the expression of the genes in subgroup II is not induced by low temperature. One such stress-responsive gene, OsNAC10 (AK069257), representing subgroup I, was functionally characterized in this study.

Table H: Rice NAC Transcription Factor Genes Up-regulated Under Stress Conditions. Numbers in boldface indicate up-regulation by more than 1.9-fold (P<0.05) in plants grown under stress conditions. [a]Numbers for full-length cDNA sequences of the corresponding genes. [b]Numbers represent the mean of three independent biological replicates. These microarray data sets can be found at http://www.ncbi.nlm.nih.gov/geo/ (Gene Expression Omnibus, GEO). [c]P values were analyzed by one-way ANOVA. Accession numbers: SNAC1, AK067690; OsNAC6, AK068392; OsNAC5, AK102475.

| Sub groups | SEQ ID[a] | High Salinity | | Drought | | ABA | | Low temperature | |
|---|---|---|---|---|---|---|---|---|---|
| | | Mean[b] | P- value[c] | Mean[b] | P- value[c] | Mean[b] | P- value[c] | Mean[b] | P- value[c] |
| I | OsNAC10 | 16.22 | 0.000 | 14.91 | 0.000 | 13.81 | 0.000 | 0.88 | 0.788 |
| | SNAC1 | 17.31 | 0.000 | 17.18 | 0.000 | 1.90 | 0.013 | 6.96 | 0.000 |
| | OsNAC6 | 5.94 | 0.000 | 5.16 | 0.000 | 4.62 | 0.000 | 2.80 | 0.011 |
| | OsNAC5 | 6.74 | 0.000 | 7.10 | 0.000 | 1.39 | 0.190 | 0.72 | 0.268 |
| | Os07g0683200 | 11.26 | 0.000 | 1.14 | 0.709 | 6.00 | 0.001 | 0.69 | 0.415 |
| | Os01g0816100 | 5.36 | 0.001 | 3.24 | 0.004 | 2.23 | 0.041 | 5.35 | 0.007 |
| | Os12g0123700 | 15.72 | 0.000 | 11.70 | 0.000 | 15.18 | 0.000 | 0.75 | 0.359 |
| II | Os08g0115800 | 1.62 | 0.198 | 2.50 | 0.014 | 1.08 | 0.891 | 1.34 | 0.552 |
| | Os09g0497900 | 2.34 | 0.003 | 2.12 | 0.003 | 1.03 | 0.927 | 0.65 | 0.146 |
| | Os12g0610600 | 1.54 | 0.104 | 0.45 | 0.006 | 4.84 | 0.000 | 0.61 | 0.153 |
| | Os06g0675600 | 2.92 | 0.002 | 1.29 | 0.313 | 3.45 | 0.001 | 0.56 | 0.112 |
| | Os07g0684800 | 17.29 | 0.000 | 11.01 | 0.000 | 8.33 | 0.000 | 0.67 | 0.401 |
| | Os03g0327100 | 6.20 | 0.000 | 9.44 | 0.000 | 2.25 | 0.012 | 1.53 | 0.224 |

(continued)

| Sub groups | SEQ ID[a] | High Salinity | | Drought | | ABA | | Low temperature | |
|---|---|---|---|---|---|---|---|---|---|
| | | Mean[b] | P- value[c] | Mean[b] | P- value[c] | Mean[b] | P- value[c] | Mean[b] | P- value[c] |
| | Os04g0460600 | 0.65 | 0.199 | 0.53 | 0.044 | 2.10 | 0.045 | 1.12 | 0.822 |
| | Os02g0579000 | 2.71 | 0.002 | 1.56 | 0.051 | 2.14 | 0.009 | 1.07 | 0.48 |
| III | Os02g0555300 | 24.78 | 0.000 | 2.69 | 0.008 | 36.66 | 0.000 | 1.06 | 0.923 |
| | Os01g0862800 | 2.77 | 0.010 | 4.51 | 0.001 | 1.20 | 0.700 | 2.77 | 0.046 |
| | Os04g0437000 | 3.40 | 0.001 | 1.39 | 0.149 | 4.16 | 0.000 | 1.04 | 0.926 |

RT-PCR analysis of the OsNAC10 expression in various tissues at different stages of development revealed that it is predominantly expressed in roots and flowers (panicles) (Figure 12 A).

Also a RNA gel-blot analysis was performed using total RNAs from leaf tissues of 14-d-old seedlings exposed to high salinity, drought, ABA and low temperature (Figure 12 B). Consistent with results from microarray experiments, the expression of OsNAC10 was induced by drought, high salinity and ABA, but not by low temperature.

Example 13: Identification of Genes Up-Regulated by Overexpressed OsNAC10

13.1. NAC10-like polypeptides

To identify genes that are up-regulated by the overexpression of OsNAC10, we performed expression profiling of GOS2:OsNAC10 and RCc3:OsNAC10 plants in comparison with NT controls under normal growth conditions. Expression profiling with the Rice 3'-Tiling microarray was conducted using RNA samples extracted from 14-d-old roots of these transgenic plants and NT controls, all grown under normal growth conditions. Each data set was obtained from three biological replicates. As listed in Tables I, J and K, statistical analysis of each data set using one-way ANOVA identified 141 genes that are up-regulated by OsNAC10 with 3-fold or greater induction in the transgenic plants than in NT plants (P <0.05). Specifically, a total of 38 genes were commonly activated in both transgenic plants whereas 44 and 59 genes were specific to RCc3:OsNAC10 and GOS2:OsNAC10 plants, respectively. Based on our microarray data shown in Tables G and H, we selected 6 (3 stress-inducible and 3 stress-uninducible) out of 38 common target genes and verified their OsNAC10-dependent expression patterns in roots of transgenic plants under normal growth conditions by real time-PCR (Figure 13, control). To test whether their expression levels increase further under stress conditions, we measured the transcript levels of the 6 genes in roots and leaves of RCc3:OsNAC10, GOS2:OsNAC10 and NT plants after exposure to drought, high salinity and low temperature conditions (Figure 13). The transcript levels of OsNAC10 are higher in roots and leaves of GOS2:OsNAC10 plants and in roots of RCc3:OsNAC10 plants over respective NT controls, as expected. In case of three stress-inducible target genes, Cytochrome P450, 20G-Fe(II) oxygenase and NCED4, their transcript levels were increased at various levels within 2 hours of the stress treatments in NT roots and leaves. The transcript levels were also higher in the transgenic roots under stress conditions in comparison with stress-treated NT plants. Their transcript levels in the transgenic leaves, in contrast, remained similar to that of NT controls, suggesting that expression of the stress-inducible target genes is not OsNAC10-dependent in leaves. Likewise, one stress-uninducible target gene, Potassium transporter KUP3, was OsNAC10-dependent in roots, but not in leaves. Interestingly, expression of two stress-uninducible target genes, Ser/Thr kinase and OPR2, were strictly OsNAC10-dependent in roots and leaves of both transgenic plants. The OsNAC10-dependent expression of the two genes in the transgenic leaves could not be justified because the OsNAC10 transcript level was not increased in RCc3:OsNAC10 leaves. It is noteworthy that the two stress-uninducible target genes were outstanding in fold-increases (over 100-fold) in roots and leaves of both RCc3:OsNAC10 and GOS2:OsNAC10 plants. Overall, these results suggest that both the constitutive and the root-specific overexpression of OsNAC10 gene enhance stress tolerance of transgenic plants at the vegetative stage by activating distinct groups of target genes.

Table I: Up-regulated Genes in RCc3:OsNAC10 and GOS2:OsNAC10 plants in Comparison to Nontransgenic Controls: [a]Numbers for full-length cDNA sequences of the corresponding genes. [b]Numbers represent the mean of three independent biological replicates. These microarray data sets can be found at http://www.ncbi.nlm.nih.gov/geo/ (Gene Expression Omnibus, GEO). [c]P values were analyzed by one-way ANOVA.

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| RCc3::OsNAC10 and GOS2::OsNAC10 | | | | | |
| Stress Inducible | | | | | |
| Cytochrome P450 | Os02g0503900 | 9.28 | 0.00 | 4.99 | 0.00 |
| 9-cis-epoxycarotenoid dioxygenase (NCED4) | Os07g0154100 | 7.83 | 0.00 | 4.17 | 0.00 |
| Zn-finger protein | Os11g0702400 | 7.71 | 0.00 | 14.94 | 0.00 |
| WRKY family transcription factor | Os09g0417800 | 7.56 | 0.00 | 5.94 | 0.00 |
| Potassium transporter | Os03g0575200 | 6.17 | 0.00 | 3.04 | 0.00 |
| AP2 | Os08g0474000 | 5.79 | 0.00 | 4.11 | 0.00 |
| Mitogen-activated protein kinase kinase | Os04g0339800 | 5.76 | 0.00 | 9.10 | 0.00 |
| HLH, helix loop helix domain | Os04g0301500 | 3.76 | 0.00 | 3.67 | 0.00 |
| Cytochrome P450 family protein | Os02g0570700 | 3.46 | 0.00 | 7.89 | 0.00 |
| 20G-Fe(II) oxygenase | Os04g0581100 | 7.67 | 0.00 | 3.19 | 0.00 |
| Pathogenesis-related protein | Os07g0129300 | 5.22 | 0.00 | 3.01 | 0.00 |
| DUF581 | Os03g0183500 | 3.75 | 0.00 | 3.01 | 0.00 |
| Auxin-responsive SAUR gene family member | Os06g0671600 | 3.33 | 0.00 | 5.83 | 0.00 |
| 1-deoxy-D-xylulose 5-phosphate synthase | Os07g0190000 | 3.16 | 0.00 | 5.32 | 0.00 |
| Late embryogenesis abundant protein | Os01g0705200 | 3.00 | 0.00 | 6.03 | 0.00 |
| Transcription, Translation, Replication | | | | | |
| Leucine-rich repeat family protein | Os08g0203400 | 8.01 | 0.00 | 3.56 | 0.00 |
| WRKY family transcription factor | Os09g0417600 | 7.84 | 0.00 | 9.28 | 0.00 |
| NAC family protein | Os11g0154500 | 3.89 | 0.00 | 6.64 | 0.00 |
| Zinc finger family protein | Os11g0687100 | 5.14 | 0.00 | 8.97 | 0.00 |
| Cellular processes | | | | | |
| Ste20-like Ser/Thr kinase (Ser/Thr kinase) | Os06g0486400 | 37.37 | 0.00 | 47.67 | 0.00 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| RCc3::OsNAC10 and GOS2::OsNAC10 | | | | | |
| Stress Inducible | | | | | |
| Lectin protein kinase | Os07g0130400 | 6.52 | 0.00 | 7.01 | 0.00 |
| Transmembrane protein kinase | Os07g0251900 | 3.97 | 0.00 | 4.68 | 0.00 |
| Wall-associated kinase | Os02g0111600 | 3.50 | 0.00 | 5.96 | 0.00 |
| Tyrosine kinase | Os10g0174800 | 3.49 | 0.00 | 6.67 | 0.00 |
| Metabolism | | | | | |
| 12-oxophytodienoate reductase (OPR2) | Os06g0215900 | 91.77 | 0.00 | 106.21 | 0.00 |
| Acyl-CoA synthetase | Os03g0130100 | 3.87 | 0.00 | 3.42 | 0.00 |
| Multicopper oxidases | Os09g0507300 | 3.16 | 0.00 | 4.22 | 0.00 |
| GCN5-related N-acetyltransferase | Os05g0376600 | 3.12 | 0.00 | 3.05 | 0.00 |
| Potassium transporter KUP3 | Os09g0448200 | 7.76 | 0.00 | 5.73 | 0.00 |
| Heavy metal transporter | Os04g0469000 | 3.86 | 0.00 | 3.53 | 0.00 |
| Detoxification protein | Os04g0464100 | 3.33 | 0.00 | 3.19 | 0.00 |
| Chitinase | Os01g0687400 | 3.08 | 0.00 | 3.49 | 0.00 |
| Glycosyl hydrolase | Os05g0298700 | 3.08 | 0.00 | 3.52 | 0.00 |
| Poorly characterized | | | | | |
| Expressed protein | Os11g0282700 | 50.21 | 0.00 | 78.51 | 0.00 |
| Protein kinase | Os10g0134500 | 4.49 | 0.00 | 6.02 | 0.00 |
| Protein kinase domain containing protein. | Os10g0151100 | 3.19 | 0.00 | 4.48 | 0.00 |
| Protein kinase domain containing protein. | Os11g0694100 | 3.13 | 0.00 | 3.64 | 0.00 |
| SAM-dependent methyltransferases | Os04g0104900 | 3.09 | 0.00 | 5.43 | 0.00 |

Table J: Up-regulated Genes in RCc3::OsNAC10 Plants in Comparison to Nontransgenic Controls: [a]Numbers for full-length cDNA sequences of the corresponding genes. [b]Numbers represent the mean of three independent biological replicates. These microarray data sets can be found at http://www.ncbi.nlm.nih.gov/geo/ (Gene Expression Omnibus, GEO). [c]P values were analyzed by one-way ANOVA.

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| RCc3:OsNAC10 | | | | | |
| Glycoside hydrolase | Os05g0247800 | 8.68 | 0.00 | 1.96 | 0.01 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| RCc3:OsNAC10 | | | | | |
| AP2 domain containing protein RAP2.6 | Os09g0457900 | 10.35 | 0.00 | 1.95 | 0.01 |
| Glycoprotein | Os11g0514500 | 3.73 | 0.00 | 1.92 | 0.00 |
| Calcium-transporting ATPase | Os10g0418100 | 14.13 | 0.00 | 1.91 | 0.00 |
| Arginine decarboxylase | Os04g0107600 | 3.28 | 0.00 | 1.88 | 0.00 |
| Glycine-rich cell wall structural protein | Os10g0451700 | 17.53 | 0.00 | 1.78 | 0.00 |
| NADH dependent Glutamate Synthase | Os05g0555600 | 4.26 | 0.00 | 1.56 | 0.01 |
| Rypsin-alpha amylase inhibitor protein. | Os10g0505000 | 5.37 | 0.00 | 1.47 | 0.03 |
| L-lactate dehydrogenase | Os06g0104900 | 4.18 | 0.00 | 1.31 | 0.06 |
| FAD linked oxidase | Os06g0548200 | 3.95 | 0.00 | 1.30 | 0.06 |
| Heat shock protein | Os01g0136100 | 3.84 | 0.00 | 1.27 | 0.16 |
| Glycoside hydrolase | Os11g0701200 | 3.39 | 0.00 | 1.26 | 0.43 |
| Leucine rich repeat | Os11g0701800 | 4.28 | 0.00 | 1.21 | 0.40 |
| NAD-dependent mannitol dehydrogenase | Os04g0612700 | 9.04 | 0.00 | 1.21 | 0.55 |
| Glycoside hydrolase | Os08g0519300 | 3.41 | 0.00 | 1.15 | 0.39 |
| Cinnamoyl CoA reductase | Os02g0808800 | 13.09 | 0.00 | 1.12 | 0.77 |
| Decarboxylase isozyme | Os05g0469600 | 9.38 | 0.00 | 1.11 | 0.49 |
| H+/oligopeptide symporter | Os03g0138700 | 4.13 | 0.00 | 1.09 | 0.77 |
| Phosphoribosylamine glycine ligase | Os12g0197100 | 3.99 | 0.00 | 1.07 | 0.72 |
| Serine/threonine protein kinase | Os03g0269300 | 5.12 | 0.00 | 1.07 | 0.72 |
| CW-type Zn-finger | Os05g0404700 | 3.65 | 0.00 | 1.06 | 0.78 |
| Chitinase | Os11g0701500 | 4.11 | 0.00 | 1.05 | 0.87 |
| Decarboxylase | Os05g0469600 | 10.74 | 0.00 | 1.04 | 0.81 |
| Chitinase | Os11g070180 | 4.70 | 0.00 | 1.03 | 0.92 |
| Glycine-rich protein | Os06g0317200 | 3.85 | 0.00 | -1.07 | 0.64 |
| Tyrosine kinase | Os09g0551500 | 6.09 | 0.00 | -1.12 | 0.70 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| RCc3:OsNAC10 | | | | | |
| Serine palmitoyltransferase | Os09g0551500 | 3.36 | 0.00 | -1.15 | 0.69 |
| Chitinase | Os11g0701400 | 5.08 | 0.00 | -1.15 | 0.65 |
| Agmatine coumaroyltransferase | Os04g0664500 | 3.12 | 0.00 | -1.22 | 0.34 |
| Heat shock protein DnaJ family protein | Os04g0675400 | 7.76 | 0.00 | -1.24 | 0.38 |
| AP2 domain containing protein RAP2.2 | Os07g0674800 | 3.48 | 0.00 | -1.25 | 0.13 |
| Heat shock protein | Os03g0267000 | 4.14 | 0.00 | -1.28 | 0.10 |
| Glycoside hydrolase | Os11g0701900 | 3.91 | 0.00 | -1.46 | 0.14 |
| Ent-kaurene oxidase | Os06g0568600 | 3.50 | 0.00 | -1.54 | 0.01 |
| Glycoside hydrolase, family 18 protein | Os08g0518900 | 5.83 | 0.00 | -1.57 | 0.02 |
| Class III chitinase | Os11g0701100 | 3.42 | 0.00 | -1.63 | 0.03 |
| Chitinase | Os08g0518800 | 3.51 | 0.00 | -1.77 | 0.02 |
| Aspartyl protease | Os01g0937500 | 4.12 | 0.00 | -1.85 | 0.00 |
| Serine/Threonine protein kinases | Os07g0262800 | 3.08 | 0.04 | -2.18 | 0.09 |
| AAA ATPase | Os12g0431100 | 3.56 | 0.00 | -3.32 | 0.00 |
| Class III chitinase | Os11g0701000 | 3.98 | 0.00 | -3.35 | 0.00 |
| phosphate dikinase | Os03g0432100 | 3.43 | 0.00 | -3.69 | 0.00 |
| Phosphoenolpyruvate carboxykinase | Os10g0204400 | 9.92 | 0.00 | -3.82 | 0.00 |
| Glycoside hydrolase | Os11g0702200 | 3.82 | 0.00 | -4.67 | 0.00 |

Table K: Up-regulated Genes in GOS2:OsNAC10 Plants in Comparison to Nontransgenic Controls: [a]Numbers for full-length cDNA sequences of the corresponding genes. [b]numbers represent the mean of three independent biological replicates. These microarray data sets can be found at http://www.ncbi.nlm.nih.gov/geo/ (Gene Expression Omnibus, GEO). [c]P values were analyzed by one-way ANOVA.

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| GOS2:OsNAC10 | | | | | |
| MATH domain containing protein | Os10g0429400 | 1.95 | 0.00 | 3.19 | 0.00 |
| Chitinase | Os03g0132900 | 1.94 | 0.00 | 3.30 | 0.00 |
| AP2 | Os07g0410700 | 1.91 | 0.00 | 4.11 | 0.00 |
| Dehydrogenase/reductase | Os07g0663700 | 1.90 | 0.00 | 3.43 | 0.00 |
| ATPase | Os09g0517200 | 1.89 | 0.00 | 3.01 | 0.00 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| GOS2:OsNAC10 | | | | | |
| En/Spm-like transposon proteins family protein | Os02g0530800 | 1.86 | 0.25 | 4.16 | 0.01 |
| Zn-finger, RING domain containing protein | Os02g0759400 | 1.84 | 0.00 | 4.83 | 0.00 |
| AAA-type ATPase-like protein. | Os03g0584400 | 1.84 | 0.01 | 3.74 | 0.00 |
| Serine/threonine-protein kinase receptor | Os04g0632600 | 1.84 | 0.11 | 4.06 | 0.00 |
| Mlo-related protein family protein | Os02g0562600 | 1.80 | 0.07 | 3.21 | 0.00 |
| Lipid phosphate phosphatase 2 | Os01g0666000 | 1.72 | 0.00 | 4.17 | 0.00 |
| Protein kinase | Os10g0136500 | 1.69 | 0.03 | 7.18 | 0.00 |
| Phytoene synthase | Os09g0555500 | 1.61 | 0.02 | 4.52 | 0.00 |
| Protein kinase family protein | Os07g0129800 | 1.59 | 0.01 | 3.14 | 0.00 |
| Group 3 LEA (Type I) protein | Os05g0542500 | 1.51 | 0.18 | 3.62 | 0.00 |
| Nicotianamine synthase 9 | Os07g0689600 | 1.49 | 0.02 | 3.06 | 0.00 |
| Glucosyltransferase | Os04g0320700 | 1.46 | 0.06 | 3.12 | 0.00 |
| Zn-finger | Os02g0252400 | 1.44 | 0.04 | 5.61 | 0.00 |
| Cytochrome P450 family protein | Os02g0569400 | 1.42 | 0.05 | 3.34 | 0.00 |
| Small heat stress protein class CIII. | Os02g0782500 | 1.39 | 0.03 | 4.11 | 0.00 |
| UDP-glucosyltransferase family protein | Os04g0206600 | 1.39 | 0.20 | 7.85 | 0.00 |
| Dehydrin RAB 16D | Os11g0453900 | 1.39 | 0.06 | 3.23 | 0.00 |
| Myb | Os04g0583900 | 1.39 | 0.20 | 8.57 | 0.00 |
| RNA-directed RNA polymerase | Os02g0736200 | 1.37 | 0.08 | 3.20 | 0.00 |
| Cyclin-like F-box domain containing protein | Os11g0539600 | 1.35 | 0.15 | 7.36 | 0.00 |
| Myb | Os04g0508500 | 1.35 | 0.08 | 5.49 | 0.00 |
| RIR1b protein | Os10g0569600 | 1.28 | 0.11 | 5.56 | 0.00 |
| Cyclin-like F-box domain containing protein | Os02g0159800 | 1.28 | 0.10 | 3.42 | 0.00 |
| HGWP repeat containing protein | Os08g0133700 | 1.28 | 0.64 | 3.14 | 0.02 |
| ATPase | Os12g0485900 | 1.24 | 0.24 | 5.10 | 0.00 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| GOS2:OsNAC10 | | | | | |
| Zn-finger | Os01g0264000 | 1.24 | 0.17 | 4.66 | 0.00 |
| En/Spm-like transposon proteins family protein | Os08g0367000 | 1.23 | 0.67 | 3.15 | 0.01 |
| Beta-glucosidase aggregating factor. | Os12g0227500 | 1.19 | 0.28 | 3.08 | 0.00 |
| UDP-glucuronosyl and UDP-glucosyl transferase | Os04g0565200 | 1.17 | 0.61 | 3.55 | 0.00 |
| Peptidase S41A | Os06g0318600 | 1.16 | 0.41 | 5.01 | 0.00 |
| Myb | Os07g0558100 | 1.12 | 0.48 | 3.06 | 0.00 |
| UDP-glucosyltransferase family protein. | Os04g0206500 | 1.12 | 0.73 | 4.43 | 0.00 |
| Serine/threonine protein kinase | Os05g0318600 | 1.05 | 0.86 | 4.05 | 0.00 |
| HAT dimerisation domain containing protein | Os12g0597800 | 1.01 | 0.97 | 4.92 | 0.00 |
| Calcium-binding EF-hand domain protein | Os01g0845900 | 1.01 | 0.97 | 3.86 | 0.00 |
| Abhydrolase | Os11g0118000 | 1.00 | 0.99 | 3.07 | 0.00 |
| MutS IV domain containing protein | Os07g0486000 | -1.81 | 0.07 | 53.93 | 0.00 |
| Epoxide hydrolase | Os10g0498300 | -1.04 | 0.88 | 42.54 | 0.00 |
| HAT dimerisation domain containing protein | Os01g0293600 | -1.08 | 0.79 | 12.01 | 0.00 |
| TPR-like domain containing protein | Os07g0171100 | -1.32 | 0.17 | 9.47 | 0.00 |
| AAA ATPase | Os09g0445700 | -1.03 | 0.88 | 6.82 | 0.00 |
| Cytochrome P450 family protein | Os02g0601500 | -1.19 | 0.30 | 6.34 | 0.00 |
| Casein kinase II alpha subunit | Os03g0762000 | -1.12 | 0.40 | 6.29 | 0.00 |
| Beta-glucosidase. | Os03g0323300 | -1.04 | 0.78 | 5.16 | 0.00 |
| Zn-finger, B-box domain containing protein | Os02g0606200 | -1.57 | 0.04 | 4.91 | 0.00 |
| Myb, DNA-binding domain containing protein | Os06g0728700 | -5.12 | 0.00 | 3.84 | 0.00 |
| OSH15 protein (Homeobox gene). | Os07g0129700 | -1.14 | 0.24 | 3.75 | 0.00 |
| Efflux Carrier family protein. | Os05g0481900 | -1.13 | 0.46 | 3.69 | 0.00 |
| UDP-glucosyltransferase family protein. | Os07g0490100 | -1.20 | 0.40 | 3.68 | 0.00 |
| basic region leucin zipper | Os07g0182000 | -1.37 | 0.11 | 3.65 | 0.00 |

(continued)

| Gene name | Loc No[a] | RCc3::OsNAC10 | | GOS2::OsNAC10 | |
|---|---|---|---|---|---|
| | (IRGSP) | Mean[b] | P val[c] | Mean[b] | P val[c] |
| GOS2:OsNAC10 | | | | | |
| zinc knuckle (CCHC-type) family protein | Os01g0775100 | -1.04 | 0.78 | 3.29 | 0.00 |
| Multitransmembrane protein | Os01g0700100 | -1.14 | 0.38 | 3.19 | 0.00 |
| Serine/Threonine protein kinases | Os08g0501000 | -1.26 | 0.25 | 3.15 | 0.00 |
| FYVE/PHD zinc finger domain containing protein | Os03g0421000 | -1.28 | 0.30 | 3.05 | 0.00 |

**Claims**

1. A method for enhancing yield-related traits in plants relative to control plants, comprising modulating expression in a plant of a nucleic acid encoding a NAC10-like polypeptide, wherein said NAC10-like polypeptide comprises a NAM domain (Pfam PF02365).

2. Method according to claim 1, wherein said NAC10-like polypeptide comprises one or more of the following motifs:

    (i) Motif I: A/P/S/N/V/T T/V/L/I/A/S T/P/S/D/V/Q D/V/I/F I A/T/G/P,
    (ii) Motif II: R/N/K E/G/S I/R/S/Q/A/V/L R/Q A/P N/S/R,
    (iii) Motif III: C/Y R/K L/I/V S/Y/H/F/R N/K/Q K D/K/N/C/S/T/G/M/A/R.

3. Method according to claim 1 or 2, wherein said modulated expression is effected by introducing and expressing in a plant a nucleic acid encoding a NAC10-like polypeptide.

4. Method according to any one of claims 1 to 3, wherein said nucleic acid encoding a NAC10-like polypeptide encodes any one of the proteins listed in Table A2 or is a portion of such a nucleic acid, or a nucleic acid capable of hybridising with such a nucleic acid; and/or wherein said nucleic acid sequence encodes an orthologue or paralogue of any of the proteins given in Table A2.

5. Method according to any one of claims 1 to 4, wherein said enhanced yield-related traits comprise increased yield, preferably increased biomass and/or increased seed yield relative to control plants.

6. Method according to any one of claims 1 to 5, wherein said enhanced yield-related traits are obtained under non-stress conditions and/or wherein said enhanced yield-related traits are obtained under conditions of drought stress, salt stress or nitrogen deficiency.

7. Method according to any one of claims 3 to 6, wherein said nucleic acid is operably linked to a root specific or to a constitutive promoter, preferably to an RCc3 or a GOS2 promoter, most preferably to an RCc3 or a GOS2 promoter from rice.

8. Method according to any one of claims 1 to 7, wherein said nucleic acid encoding a NAC10-like polypeptide is of plant origin, preferably from a dicotyledonous plant, further preferably from the family Poaceae, more preferably from the genus *Oryza,* most preferably from *Oryza sativa.*

9. Construct comprising:

    (i) a nucleic acid encoding a NAC10-like polypeptide as defined in any one of claims 1, 2 or 4;
    (ii) one or more control sequences capable of driving expression of the nucleic acid sequence of (i); and optionally
    (iii) a transcription termination sequence.

**10.** Construct according to claim 9, wherein one of said control sequences is a root specific or a constitutive promoter, preferably an RCc3 or a GOS2 promoter, most preferably an RCc3 or a GOS2 promoter from rice.

**11.** Use of a construct according to claim 9 or 10 in a method for making plants having increased yield, particularly increased biomass and/or increased seed yield relative to control plants; or use of a nucleic acid encoding a NAC10-like polypeptide in increasing yield, particularly in increasing seed yield and/or shoot biomass in plants, relative to control plants.

**12.** Method for the production of a transgenic plant having increased yield, particularly increased biomass and/or increased seed yield relative to control plants, comprising:

(i) introducing and expressing in a plant a nucleic acid encoding a NAC10-like polypeptide as defined in any one of claims 1, 2 or 4; and
(ii) cultivating the plant cell under conditions promoting plant growth and development.

**13.** Transgenic plant having increased yield, particularly increased biomass and/or increased seed yield, relative to control plants, resulting from modulated expression of a nucleic acid encoding a NAC10-like polypeptide as defined in any one of claims 1, 2 or 4, or a transgenic plant cell derived from said transgenic plant; or plant, plant part or plant cell transformed with a construct according to claim 9 or 10; or plant or part thereof, including seeds, obtainable by a method according to any one of claims 1 to 8, wherein said plant or part thereof comprises a recombinant nucleic acid encoding a NAC10-like polypeptide.

**14.** Transgenic plant according to claim 13, or a transgenic plant cell derived therefrom, wherein said plant is a crop plant or a monocot or a cereal, such as rice, maize, wheat, barley, millet, rye, triticale, sorghum emmer, spelt, secale, einkorn, teff, milo and oats.

**15.** Harvestable parts of a plant according to claim 13, wherein said harvestable parts are preferably shoot biomass and/or seeds; or products derived from said plant and/or from said harvestable parts of a plant.

```
                                            1                                                    50
                            corn     (1)  -------------------------------------------------
         O.sativa_Os05g0523300#1     (1)  -MEEEFKDKGLPPTLLHLIPDGREWKVKEADGEGSRNTN---LDADEDKE
            T.aestivum_TC350242#1     (1)  -------------------------------------------------
   corn_EU965307__IAA6__ACG37425     (1)  MGDSGGRRGPPLPRLLDLIPDGKERKGR-EAQDAGRSKNSGGFGGEEDAK  .
              Z.mays_TC410949#1      (1)  MGDSGGRRGPPLPRLLDLIPDGKERKGR-EAQDAGRSKNSGGFGGEEDAK
            H.vulgare_TC162609#1     (1)  -------------------------------------------------
         O.sativa_Os01g0741900#1     (1)  MEEGSNKREGLPPQLLDLIPDEKEWKLR-EALGLGRSRN-AGFDGEEDKK
        OsIBCD003255_319_AUX-IAA     (1)  MEEGSNKREGLPPQLLDLIPDEKEWKLR-EALGLGRSRN-AGFDGEEDKK
            T.aestivum_TC289502#1     (1)  MEESSMKREAMP-RLLDLIPDEKEWSLRGGAPGQARSKN-TGFGSEEDEK
            T.aestivum_TC297212#1     (1)  MEESSMKREAMP-RLLDLIPDEKEWSLRGGAPGQGRSKN-TGFGSDEDEK
                        Consensus    (1)  M E   KR GLPP LLDLIPD KEWKLR A   GRSKN  GF GEED K
```

```
                                            51                                                   100
                            corn     (1)  -------------------------------------------------
         O.sativa_Os05g0523300#1    (47)  LELKLGLPGVQQEERAADSREKIQ----QQQRESSSEPSIGCFPTHSKP-
            T.aestivum_TC350242#1     (1)  -------------------------------------------------
   corn_EU965307__IAA6__ACG37425    (50)  LELKLGPPGLTLVEEGTTATVPRDGGVLQRETTTTPTLSLGCFPRNPSKP
              Z.mays_TC410949#1     (50)  LELKLGPPGLTLVEEGTTATVPRDGGVLQRETTTTPTLSLGCFPRNPSKP
            H.vulgare_TC162609#1     (1)  -------------------------------------------------
         O.sativa_Os01g0741900#1    (49)  LDLKLGLPGFIEDDEAETLRDY-------RLQQESPSLSLSFFPKHSKTT
        OsIBCD003255_319_AUX-IAA    (49)  LDLKLGLPGFIEDDEAETLRDY-------RLQQECPSLSLGFFPKHSKTT
            T.aestivum_TC289502#1    (49)  LELKLGLPGLVEEEPAASSRENN------RVHQESPALSLGYPPRHST--
            T.aestivum_TC297212#1    (49)  LELKLGLPGLVQEEPAASSREK-------RVHQESPALYLGYPPRHSTA-
                        Consensus   (51)  LELKLGLPGL EE A TSRE      R     SP LSLG FPRHS
```

```
                                            101                                                  150
                            corn     (1)  -------------------------------------------------
         O.sativa_Os05g0523300#1    (92)  TTSIGTTGAKRGFFAIVGATLEGYNQSHR----DTEECGKELTLGDEN--
            T.aestivum_TC350242#1     (1)  -------------------------------------------------
   corn_EU965307__IAA6__ACG37425   (100)  AVDAATAGTKRGFFDT-AVEAKTEGRDERMEQQAGAGCGNELALDEKTAA
              Z.mays_TC410949#1    (100)  AVDAASSWDEEGVLTPPPSRPRQKVRDERMEQQAGAGCGNELALDEKTAA
            H.vulgare_TC162609#1     (1)  --------MRRSRSSR-REQQRVGRNWQR-----RKTQRPRPWVRGR--K
         O.sativa_Os01g0741900#1    (92)  SSTTTTTGAKRGFIDT-VEDKTEGYNDQK--QQARAGCGKELAVEEM--I
        OsIBCD003255_319_AUX-IAA    (92)  SSTTTTTGAKRGFIDT-VEDKTEGYNDQK--QQARAGCGKELAVEEM--I
            T.aestivum_TC289502#1    (91)  TITTTTTGAKRGFLDT-VEAKAQGYEKEQKQQARAAACGKELAVEEN--T
            T.aestivum_TC297212#1    (91)  TTTTTTTGAKRGFLDT-VEAKAQGYEKEQ---ARAATCGKELAMEEN--T
                        Consensus  (101)  T T  TTGAKRGFLDT  E   G   R Q   AGCGKELAVEE
```

```
                                            151                                                  200
                            corn     (1)  -MAGETKKGCCCPPSSSHDSDAGPAV---------HRGDVLPVVGWPPVR
         O.sativa_Os05g0523300#1   (136)  -MAGERKKGCCPSPPCSAAAHSSNPQ---------GRGAIPPVVGWPPIR
            T.aestivum_TC350242#1     (1)  -------------------------------------------------
   corn_EU965307__IAA6__ACG37425   (149)  AAASERQKGSCCPPTPQHAPPAATVHSGAHVLQLGRR-PSAPVVGWPPVR
              Z.mays_TC410949#1    (150)  AAASERQKGSCCPPTPQHAPPAATVHSGAHVLQLGRR-PSAPVVGWPPVR
            H.vulgare_TC162609#1    (35)  AAVPHRRR----MPLLLLHLRATSATDR----RRGEEGLAVPVVGWPPIR
         O.sativa_Os01g0741900#1   (137)  AAVSERKKGCCPPPFPPHGAPATPAR---NRPQTQGRGAAAPVVGWPPIR
        OsIBCD003255_319_AUX-IAA   (137)  AAVSERKKGCCPPPPPPHGAPATPAR---NRPQTQGR------------
            T.aestivum_TC289502#1   (138)  ATVGERKKGCCPPPPPPHAPPATPARNIGNRPQARGRGAAAPVVGWPPIR
            T.aestivum_TC297212#1   (135)  EAVGERKKGCCPPPPPAHAPPATPARNIGNRPQARGRGAAAPVVGWPPIR
                        Consensus  (151)  AAV ERKKGCCPPPPP HA PATPA    Q  GRG AAPVVGWPPIR
```

```
                                            201                                                  250
                            corn    (41)  SFRRNLANASSSKQSLEQQQQNDDEASCDKAKQTCKRSPLIKINMDGIPI
         O.sativa_Os05g0523300#1   (176)  SFRRNLTNGSSFKQ----SPERQNDEADDKAKPICKKRPLVKINMDGIPI
            T.aestivum_TC350242#1     (1)  -MELHLSNN---------PLSDKNVEATDRAKPVCKKKPLIKINMDGIPI
   corn_EU965307__IAA6__ACG37425   (198)  SFRRNLAHHHHGSS-SKQPTEPQNSEASRKEKPACCKNPLVKINMDGIPI
              Z.mays_TC410949#1    (199)  SFRRNLAHHHHGSS-SKQPTEPQNSEASRKEKPACCKNPLVKINMDGIPI
            H.vulgare_TC162609#1    (77)  SFRRNLASTSA----SKQVHEPQIAEADAKAALNCKKSPLVKINMDGIPI
         O.sativa_Os01g0741900#1   (184)  SFRRNLASSSS----SKHSPEPQNDNANAKVTLTCKKNPLVKINMDGIPI
        OsIBCD003255_319_AUX-IAA   (171)  --R-NLASSSS----SKHSPEPQNDNANAKVTLTCKKNPLVKINMDGIPI
            T.aestivum_TC289502#1   (188)  SFRRNLASTSA----SKQPPEPQIGEADAKAVLDCKKSPLVKINMDGIPI
            T.aestivum_TC297212#1   (185)  SFRRNLASTSA----SKQPPEPQIGEANAKAVLDCKKSPLVKINMDGIPI
                        Consensus  (201)  SFRRNLASTSS   SKQPPEPQN EA AKAKL CKK PLVKINMDGIPI
```

# FIGURE 1

```
                                     251                                        300
                    corn      (91)  GRKINLSAYDSYQKLSSAVQDLFCGFLDAQKDESRG----RGAEEKMFSG
    O.sativa_Os05g0523300#1  (222)  GRKVDLQIYDSYQKLSSAVEELFRGFLEAQKDLSCAESGEQGAEDKIFSG
       T.aestivum_TC350242#1   (41)  GRKVDLASCDNYHKLSSAVEELFRGFLEAQQDLSCDESGVRGAEEKLFSG
corn_EU965307__IAA6__ACG37425 (247)  GRKVDLAAYDSYERLSLGVKELFHGFLQAQKDMS------P-TAGKIFSQ
        Z.mays_TC410949#1     (248)  GRKVDLAAYDSYERLSLGVKELFHGFLQAQKDMS------P-TAGKIFSQ
       H.vulgare_TC162609#1   (123)  GRKVDLAACDSYERLSLAVKDLFHGFLEVQRDTPRAERAQQGADEKIFSQ
    O.sativa_Os01g0741900#1  (230)  GRKIDLAAYNSYDGLSSAVKQLFHGFLQAQKDQTNAQIAQQGADDKIFYQ
   OsIBCD003255_319_AUX-IAA  (214)  GRKIDLAAYNSYDGLSSAVKQLFHGFLQAQKDQTNAQIAQQGADDKIFYQ
       T.aestivum_TC289502#1  (234)  GRKVDLAACDSYGRLSLAVKDLFHGFLQVQRDPSKVDRTQQGADEKIFSQ
       T.aestivum_TC297212#1  (231)  GRKVDLAACDSYERLSLAVKDLFHGFLQVQRDPSKVERTQQGADENIFSR
               Consensus     (251)  GRKVDLAAYDSYERLSSAVKDLFHGFLQAQKD S AE AQQGADEKIFSQ


                                     301                                        350
                    corn     (137)  LLDGTGEYTLVCEDSEGGRTLVGHLPWNVFVSTAKRLRVMESSELPHGLI
    O.sativa_Os05g0523300#1  (272)  LLDGTGVYTLVYEDNDGDRMLAGDIPWKVFVSTVKRLRVMRRSELPHDMI
       T.aestivum_TC350242#1   (91)  LLDGTGEYALVYEDDEGDRMLVGDIPWSVFVSTAKRLRVMRRSELPQRMT
corn_EU965307__IAA6__ACG37425 (290)  LLDGSGEYTLVYEDNEGDRMLVGDVPWNVFVSTAKRLRVLRSSELAKGLV
        Z.mays_TC410949#1     (291)  LLDGSGEYTLVYEDNEGDRMLVGDVPWNVFVSTAKRLRVLRSSELAKGLV
       H.vulgare_TC162609#1   (173)  LLDGSGEYTLVYEDNEGDRMLVGDVPWNVFVSTAKRLRVLRSSELSHGLA
    O.sativa_Os01g0741900#1  (280)  LLDGSGEYTLVYEDSEGDRMLVGDVPWKVFVSTAKRLRVLRSSELSHTLI
   OsIBCD003255_319_AUX-IAA  (264)  LLDGSGEYTLVYEDSEGDRMLVGDVPWKVFVSTAKRLRVLRSSELSHTLI
       T.aestivum_TC289502#1  (284)  LLDGSGEYTLVYEDSEGDRMLVGDVPWNVFVSTAKRLRVLRSSELSHDLI
       T.aestivum_TC297212#1  (281)  LLDGSGEYTLVYEDSEGDRMLVGDVPWNVFVSTAKRLRVLRSSELSHGLI
               Consensus     (301)  LLDGSGEYTLVYEDSEGDRMLVGDVPWNVFVSTAKRLRVLRSSELSHGLI


                                     351          369
                    corn     (187)  KTASERADD----------
    O.sativa_Os05g0523300#1  (322)  GADPVK-------------
       T.aestivum_TC350242#1  (141)  GANSTKVADC---------
corn_EU965307__IAA6__ACG37425 (340)  GTRPPQIAVAPRRGPPDC-
        Z.mays_TC410949#1     (341)  GTRPPQIAVAPRRGPPDC-
       H.vulgare_TC162609#1   (223)  GVTLERAANC---------
    O.sativa_Os01g0741900#1  (330)  GATARV-------------
   OsIBCD003255_319_AUX-IAA  (314)  GATARV-------------
       T.aestivum_TC289502#1  (334)  GATPERAANC---------
       T.aestivum_TC297212#1  (331)  GATPERTAHG---------
               Consensus     (351)  GATP R A
```

# FIGURE 1 (continued)

**FIGURE 2**

MPSSGGAMPA**LPPGFRFHPTDEELIVHYLMNQAASV**

**KCP**<u>**VPIIAE**</u>**VNIYKCNPWDLPGKALFGENEWYFFSP**

**RDRKY**<u>**PNGARPNR**</u>**AAGSGYWKATGTDKSILSTPTSD**

**NIGVKKALVFYKGKPPKGVKTDWIMHEYRLT**GTSAN

STTTTKQRRASSMTMRLDDWVL<u>CRIHKKSN</u>DFNSSD

QHDQEPEESTVEQLEDIHDNNSSEQPPAPADMNNQQ

SDFQPMTAMSMSKSCSLTDLLNTIDCAALSQFLLDG

SSDAIAEPPAPPSPLIYTTPHPNYQTLNYNINSNSS

MPHAFESRLDHHDGYVNNYNVNGLRRKRMMACSATS

FDDGSSSNDFVHAVVKKPQLLPSDSRGSGFGGGYCN

QQLSETATGFQFQNGNLLSHPFPLNNHLQMQ

**FIGURE 3**

CLUSTAL 2.0.11 multiple sequence alignment

```
OsNAC10_like1     --MPSSGG-----------AMPALPPGFRFHPTDEELIVHYLMNQAASVKCPVPIIAEV
Os11g03300        --MPSSGG-----------AMPALPPGFRFHPTDEELIVHYLMNQAASVKCPVPIIAEV
OsNAC10_SEQID2    --MPSSGG-----------AMPALPPGFRFHPTDEELIVHYLMNQAASVKCPVPIIAEV
Os12g0123700      --MPSSGG-----------AMPALPPGFRFHPTDEELIVHYLMNQAASIKCPVPIIAEV
Zm_ZM07MC20223    --MPRSGGGGNGGGISSSSRVMPNLPAGFRFHPTDEELMVHYLMRQAASMPCPVPIIAEV
Gm_GM06MC02339    --MEGSTT------------SSELPPGFRFHPTDEELIVYYLCNQATSKPCPASIIPEV
Gm_GM06MC00030    --MEGGTT------------SSELPPGFRFHPTDEELIVYYLCNQATSKPCPASIIPEV
Mt_AC140030_19.5  --MES-SA------------SSELPPGFRFHPTDEELIVHYLCNQATSKPCPASIIPEV
Mt_AC202309_11.3  --MES-SA------------SSELPPGFRFHPTDEELIVHYLCNQATSKPCPASIIPEV
Gm_GM06MC38096    --MEN-RT------------SSVLPPGFRFHPTDEELIVYYLCNQASSRPCPASIIPEV
Mt_CT573505_5.4   --ME-----------------LPPGFRFHPTDEELIVYYLCNQATSQPCPASIIPEV
Pt_scaff_VIII.823 --MQGKTI------------YNQLPPGFRFHPTDEELIVYYLRNQATSRPCPASIIPEV
Sl_TC204003       --MVGKIS------------S-DLPPGFRFHPTDEELIMYYLRYQATSRPCPVSIIPEI
Sl_CDS_43590      --MVGKIS------------S-DLPPGFRFHPTDEELIMYYLRYQATSRPCPVSIIPEI
Sl_TC200098       --MVGKNN------------SNHLPPGFRFHPTDEELIMYYLRNQATSKPCPSSIIPEV
AT1G69490.1       --MEVTSQ------------S-TLPPGFRFHPTDEELIVYYLRNQTMSKPCPVSIIPEV
Os_CDS_20741      --MVLSNPA------------MLPPGFRFHPTDEELIVHYLRNRAASSPCPVSIIADV
OS_CDS_21636      --MVLSNPA------------MLPPGFRFHPTDEELIVHYLRNRAASSPCPVSIIADV
Zm_ZM07MStraceDB  --MTMAATTMMSAAARGE--TSHLPPGFRFPPTDEELILHYLRNRASSAPCPVPIIADV
Os07g0683200      --MSSAATS-------------LPPGFRFHPTDEELILHYLRSRATAGQCPVPIIADV
Ac_CF438306       --MTNQAPL-------------LPPGFRFHPTDEELILFYLKNRAEATPCPVSIIAEV
AT3G04070.1       MISKDPRSS-------------LPPGFRFHPTDEELILHYLRKKVSSSPVPLSIIADV
AT3G04070.2       MISKDPRSS-------------LPPGFRFHPTDEELILHYLRKKVSSSPVPLSIIADV
Gm_GM06MC20707    --MGNPESN-------------LPPGFRFHPTDEELILHYLSKKVASIPLTVSIIAEV
Mt_AC145753_12.5  --MGTPQTN-------------LPPGFRFHPTDAELILHYLRKKIASIPLPVSIIAEV
Pt_scaff_XIII.531 --MKNPQSS-------------LPPGFRFHPTDEELILHYLKKKLASTPFPVSIIADV
Pt_scaff_XIX.359  --MKNQQSS-------------LPPGFRFHPTDEELILHYLRKKVASTPFPVSIIADV
Mt_AC128638_20.4  --MD---KNPHS---------EIQLPPGFRFHPSDEELIVHYLRNKVTSSPLPASFIAEI
Mt_AC150891_8.5   --MEGQQNGSKS---------NYTFPPGFRFHPSDEELIVHYLQNKIKSRPLPASIIAEI
                                      :*.**** *:* **::.**   :   :    . .:*.::
```

```
OsNAC10_like1     NIYKCNPWDLPGKALFGENEWYFFSPRDRKYPNG-RPNRAAGSGYWKATGTDKSILS---
Os11g03300        NIYKCNPWDLPGKALFGENEWYFFSPRDRKYPNG-RPNRAAGSGYWKATGTDKSILS---
OsNAC10_SEQID2    NIYKCNPWDLPGKALFGENEWYFFSPRDRKYPNGARPNRAAGSGYWKATGTDKSILS---
Os12g0123700      NIYKCNPWDLPGKALFGENEWYFFSPRDRKYPNGARPNRAAGSGYWKATGTDKSILS---
Zm_ZM07MC20223    NIYQCNPWDLPAKALFGDKEWFFFSPRDRKYPNGARPNRAAGSGYWKATGTDKAILSSS-
Gm_GM06MC02339    DLYKFDPWELPDKTEFGENEWYFFSPRDRKYPNGVRPNRATVSGYWKATGTDKAIYS---
Gm_GM06MC00030    DLYKFDPWELPDKTEFGENEWYFFTPRDRKYPNGVRPNRATVSGYWKATGTDKAIYS---
Mt_AC140030_19.5  DIYKFDPWELPDKSEFEENEWYFFSPRERKYPNGVRPNRATLSGYWKATGTDKAIKS---
Mt_AC202309_11.3  DIYKFDPWELPDKSEFEENEWYFFSPRERKYPNGVRPNRATLSGYWKATGTDKAIKS---
Gm_GM06MC38096    DIYKFDPWELPDKTDFGEKEWYFFSPRERKYPNGVRPNRATVSGYWKATGTDKAIYS---
Mt_CT573505_5.4   DIYKFDPWELPDKSGFGENEWYFFTPRERKYPNGVRPNRATVSGYWKATGTDKSIFS---
Pt_scaff_VIII.823 DIYKFDPWQLPEKAEFGENEWYFFTPRDRKYPNGVRPNRATVSGYWKATGTDKAIHS---
Sl_TC204003       DVYKFDPWVLPEKAEFGDNEWYFFTPRDRKYPNGVRPNRAAVSGYWKATGTDKAIYS---
Sl_CDS_43590      DVYKFDPWVLPEKAEFGDNEWYFFTPRDRKYPNGVRPNRAAVSGYWKATGTDKAIYS---
Sl_TC200098       DVYKFDPWELPEKTEFGEKEWYFFTPRDRKYPNGVRPNRAAVSGYWKATGTDKGIYS---
AT1G69490.1       DIYKFDPWQLPEKTEFGENEWYFFSPRERKYPNGVRPNRAAVSGYWKATGTDKAIHS---
Os_CDS_20741      DIYKFDPWDLPSKENYGDREWYFFSPRDRKYPNGIRPNRAAGSGYWKATGTDKPIHSSG-
OS_CDS_21636      DIYKFDPWDLPSKENYGDREWYFFSPRDRKYPNGIRPNRAAGSGYWKATGTDKPIHSSG-
Zm_ZM07MStraceDB  DIYKFDPWDLPARALYGDEYYFFSPRDRKYPNGIRPNRAAGSG-WKATGTDKPIHDA--
Os07g0683200      DIYKFDPWDLPSKAVYGESEWYFFSPRDRKYPNGIRPNRAAGSGYWKATGTDKPIHDS--
Ac_CF438306       DIYKFDPWNLPDKAMFGDKEWYFFTPRDRKYPNGVRPNRAAGSGYWKATGTDKPIVTT--
AT3G04070.1       DIYKSDPWDLPAKAPFGEKEWYFFSPRDRKYPNGARPNRAAASGYWKATGTDKLIAVP--
AT3G04070.2       DIYKSDPWDLPAKAPFGEKEWYFFSPRDRKYPNGARPNRAAASGYWKATGTDKLIAVP--
Gm_GM06MC20707    DIYKLDPWDLPAKATFGEKEWYFFSPRDRKYPNGARPNRAAASGYWKATGTDKTIVTS--
Mt_AC145753_12.5  DIYKLDPWDLPAKASFGEKEWYFFSPRDRKYPNGARPNRAAASGYWKATGTDKTIVAS--
Pt_scaff_XIII.531 DIYKFDPWDLPAKASLGEKEWYFFSPRDRKYPNGARPNRAAASGYWKATGTDKIIMASTM
Pt_scaff_XIX.359  DIYKFDPWDLPAKAALGEKEWYFFSPRDRKYPNGARPNRAAASGYWKATGTDKVIMASTI
Mt_AC128638_20.4  NLYKYNPWELPSKALFGEEEWYFFTPRDRKYPKGLRPNRAAGVGYWKATGTDKPILTS--
Mt_AC150891_8.5   DLYKYNPWELPKKSLFGEEEWYFFSPRDRKYPNGLRPNRTAASGYWKATGTDKPIISS--
                  ::*: :** ** :    : *::**:**:****:* ****::  * ******** *
```

## FIGURE 4

```
OsNAC10_like1        -TPTSDN-----IGVKKALVFYKGKPPKGVKTDWIMHEYRLTGTSANSTTTTKQRRASS-
Os11g03300           -TPTSDN-----IGVKKALVFYKGKPPKGVKTDWIMHEYRLTGTSANSTTTTKQRRASS-
OsNAC10_SEQID2       -TPTSDN-----IGVKKALVFYKGKPPKGVKTDWIMHEYRLTGTSANSTTTTKQRRASS-
Os12g0123700         -TPTSDN-----IGVKKALVFYKGKPPKGVKTDWIMHEYRLTGTSANNTTTTKQRRASS-
Zm_ZM07MC20223       -TPTSHGGANIVVGVKKALVFYGGRPPKGTKTDWIMHEYRLSGAADDDCKGSTRRRVSSS
Gm_GM06MC02339       ---GSKN-----VGVKKSLVFYKGRPPKGAKTDWIMHEYRLA-----ESKIPASRKIGS-
Gm_GM06MC00030       ---GSKH-----VGVKKSLVFYKGRPPKGAKTDWIMHEYRLA-----ESKIPSSRKIGS-
Mt_AC140030_19.5     ---GSKQ-----IGVKKSLVFYKGRPPKGVKTDWIMHEYRLI-----GSQKQTSKHIGS-
Mt_AC202309_11.3     ---GSKQ-----IGVKKSLVFYKGRPPKGVKTDWIMHEYRLI-----GSQKQTSKHIGS-
Gm_GM06MC38096       ---GSKH-----VGVKKALVFYKGKPPKGLKTDWIMHEYRLI-----GSRRQANRQVGS-
Mt_CT573505_5.4      ---GSKH-----IGVKKALVFYKGRPPKGIKTDWIMHEYRLI-----GSRRQANRQIGS-
Pt_scaff_VIII.823    ---GSKY-----VGVKKALVFYKGRPPKGAKTDWIMHEYRLN-----DPRKQANKQNGS-
Sl_TC204003          ---ANKY-----VGIKKALVFYKGKPPKGVKTDWIMHEYRLS-----DSKSQTSKQSGS-
Sl_CDS_43590         ---ANKY-----VGIKKALVFYKGKPPKGVKTDWIMHEYRLS-----DSKSQTSKQSGS-
Sl_TC200098          ---GTKY-----VGIKKALVFYKGKPPKGIKTDWIMHEYRLS-----ESRTQPTRPNGS-
AT1G69490.1          ---GSSN-----VGVKKALVFYKGRPPKGIKTDWIMHEYRLH-----DSRKASTKRNGS-
Os_CDS_20741         -----GAATNESVGVKKALVFYKGRPPKGTKTNWIMHEYRLAAAD--AHAANTYRPMK--
OS_CDS_21636         -----GAATNESVGVKKALVFYKGRPPKGTKTNWIMHEYRLAAAD--AHAANTYRPMK--
Zm_ZM07MStraceDB     -------ATGEGVGVKKALVFYRGRPPRGSKTNWIMHEYRLAAVAGCTSPLAAYRPPS--
Os07g0683200         -------ATGESVGVKKALVFYRGRPPKGTKTSWIMHEYRLAADP-LAAAANTYKPSSSS
Ac_CF438306          -------IGNENIGVKKALVFYIGKPPKGVKTNWIMHEYRLSESN-IKSSNNIARSTR--
AT3G04070.1          ----NGEGFHENIGIKKALVFYRGKPPKGVKTNWIMHEYRLADS----LSPKRINSSRSG
AT3G04070.2          ----NGEGFHENIGIKKALVFYRGKPPKGVKTNWIMHEYRLADS----LSPKRINSSRSG
Gm_GM06MC20707       LQGGAQES----VGVKKALVFYKGRPPKGVKTNWIMHEYRLVD------NNKPIKLKDS-
Mt_AC145753_12.5     LPCGGGRSQENIIGVKKALVFYKGKPPKGIKTNWIMHEYRLVD------NNKPIKLKDS-
Pt_scaff_XIII.531    APGGVVG-GQENIGVKKALVFYKGKPPKGVKTNWIMHEYRLADNPACNNNKKSMKPKDS-
Pt_scaff_XIX.359     TPGGVIIQGQENVGVKKALVFYKGKPPKGVKTDWIMHEYRLADTSSYSYN-KPMKPRDS-
Mt_AC128638_20.4     --CGLKS-----IGVKKALVFYKGRPPKGSKTEWIMHEYRLH-----DSMISNSNHSGS-
Mt_AC150891_8.5      --CESKH-----IGVKKALVFYSGRPPKGVKTDWIMNEYRLV-----DTTAKSFKLKGS-
                        :*:**:****  *:**:*  **.***:****
```

```
OsNAC10_like1        ---------------MTMRLD-WVLCRIHKKSNDFN--SSDQHDQEPEE------STV-E
Os11g03300           ---------------MTMRLD-WVLCRIHKKSNDFN--SSDQHDQEPEE------STV-E
OsNAC10_SEQID2       ---------------MTMRLDDWVLCRIHKKSNDFN--SSDQHDQEPEE------STV-E
Os12g0123700         ---------------MTMRLDDWVLCRIHKKSNDFN--SSDQHDQEPEG------STVDE
Zm_ZM07MC20223       -------------SSSSMRLDDWVLCRIHKKSNDFQLSSSSEHEHEQEEPAAGGSATTVE
Gm_GM06MC02339       ----------------MRLDDWVLCRIYKKKSMVK--------------------ALE
Gm_GM06MC00030       ----------------MRLDDWVLCRIYKKKSMVK--------------------ALE
Mt_AC140030_19.5     ----------------MRLDDWVLCRIYKKKHMGK--------------------TLQ
Mt_AC202309_11.3     ----------------MRLDDWVLCRIYKKKHMGK--------------------TLQ
Gm_GM06MC38096       ----------------MRLDDWVLCRIYKKKNIGK--------------------SME
Mt_CT573505_5.4      ----------------MRLDDWVLCRIYKKKNITK--------------------SLE
Pt_scaff_VIII.823    ----------------MRLDDWVLCRIYKKRHTIG--------------------HLE
Sl_TC204003          ----------------MRLDDWVLCRIYKKKNLGR--------------------TIE
Sl_CDS_43590         ----------------MRLDDWVLCRIYKKKNLGR--------------------TIE
Sl_TC200098          ----------------MRLDDWVLCRIYKKKNLER--------------------AIE
AT1G69490.1          ----------------MRLDEWVLCRIYKKRGASK--------------------LLN
Os_CDS_20741         ------------FRNTSMRLDDWVLCRIYKKS--SHASPL---------------AVPP
OS_CDS_21636         ------------FRNTSMRLDDWVLCRIYKKS--SHASPL---------------AVPP
Zm_ZM07MStraceDB     ----------KFRNVSMRLDDWVLCRIYKKS--GHASPR---------------VPPL
Os07g0683200         ----------RFRNVSMRLDDWVLCRIYKKS--GQASPM---------------MPPL
Ac_CF438306          ------------IRNSSMRLDDWVLCRIYQKG--GHLKED---------------SVEN
AT3G04070.1          GSEVNNNFGDRNSKEYSMRLDDWVLCRIYKKSHASLSSPD--------------VALV
AT3G04070.2          GSE-----------LDDWVLCRIYKKSHASLSSPD--------------VALV
Gm_GM06MC20707       ----------------SMRLDDWVLCRIYKKSKHALTST---------------EAS-
Mt_AC145753_12.5     ----------------SMRLDDWVLCRIYKKSKCALTST---------------ESSE
Pt_scaff_XIII.531    ----------------SMRLDDWVLCRIYKKSHALTSSP---------------RALI
Pt_scaff_XIX.359     ----------------SMRLDDWVLCRIYKKAHALTSCA---------------RAEM
Mt_AC128638_20.4     ----------------MRLDEWVLCRVRQKMGSPR--------------------SSW
Mt_AC150891_8.5      ----------------MRLDDWVLCRVRNKGYSLK--------------------NLS
                        ** *****: :*
```

# FIGURE 4 (continued)

```
OsNAC10_like1         QLED--IHDNNSS-EQPPAPADMNNQQS------DFQPMTAMS----------------
Os11g03300            QLED--IHDNNSS-EQPPAPADMNNQQS------DFQPMTAMS----------------
OsNAC10_SEQID2        QLED--IHDNNSS-EQPPAPADMNNQQS------DFQPMTAMS----------------
Os12g0123700          QLED--IHDNNSSSQQPPAPPDMNNQQS------DFQPMTAMS----------------
Zm_ZM07MC20223        ELDALVVVDNSSSSDTTTTTITTNDNNSTETAATHHDPQTMMM----------------
Gm_GM06MC02339        HK------EAHPEVQIAN-LAPANH--DVE--------QKMMN----------------
Gm_GM06MC00030        HK------EAQPKVQIAN-LAAANQ--DVE--------QKMMN----------------
Mt_AC140030_19.5      QK------EDYSTHQFNDSIITNND--DGE--------LEMMN----------------
Mt_AC202309_11.3      QK------EDYSTHQFNDSIITNND--DGE--------LEMMN----------------
Gm_GM06MC38096        AK------EDYPIAQINL--TPANN--NSE--------QELVK----------------
Mt_CT573505_5.4       TN------EDYPTNQINM--TQTND--DNE--------QELVK----------------
Pt_scaff_VIII.823     EK------TEN-TVDAHLDVTPAND--VSE--------QQMMK----------------
Sl_TC204003           MMK---VEEEELEAQNVSTTNNEIE--VVG-------GPQTMK----------------
Sl_CDS_43590          MMK---VEEEELEAQNVSTTNNEIE--VVG-------GPQTMK----------------
Sl_TC200098           MMK---VEEDTQEPQIMSVTNPIHE--SVAS-----NGQQTLK----------------
AT1G69490.1           EQEGF-MDEVLMEDETKVVVNEAERRTEEEI-----MMMTSMK----------------
Os_CDS_20741          LSDHEQDEPCALEENAPLYAPSSSSAASMIL-------QGAAAGAFPSLHAAAAATQRTA
OS_CDS_21636          LSDHEQDEPCALEENAPLYAPSSSSAASMIL-------QGAAAGAFPSLHAAAAATQRTA
Zm_ZM07MStraceDB      A-DYEHLDRDGPSP---------------------------------------------
Os07g0683200          AADYDHDEPSGVLDDAYSFYAPPMISTTLIP-------K--------------------
Ac_CF438306           ESSFEDNK-------------------------------------------------LE
AT3G04070.1           TSNQEHEENDNEPFVDRGTFLPNLQN-----------DQPLK---------------R
AT3G04070.2           TSNQEHEENDNEPFVDRGTFLPNLQN-----------DQPLK---------------R
Gm_GM06MC20707        IGVGEVDQAEEHLFQE--TLLPISKSPAPPP-------QNTLI---------------S
Mt_AC145753_12.5      TMVGEVEHAEETQFQE--TLFPITKNTTSPL-------QNTLM---------------S
Pt_scaff_XIII.531     SSEHDQEEEEEEQQQQ--QFVQETLLPISN--------KNNLM---------------S
Pt_scaff_XIX.359      SSEHEQEEEEEHFVPE--NPLPSLKGPISN--------KSALM---------------S
Mt_AC128638_20.4      EDSNELSYEST------SQFQQMTVNSNPEP-------VKNYV----------------
Mt_AC150891_8.5       ENQENPSEPTIPLNLPRGEERPTNVNLRSDI-------ITDYQ----------------


OsNAC10_like1         MSKSCSLTDLLNTIDCA----LS-QFLLDGSSDAIAEPPAPPSPLIYTTPHPNYQTLNYN
Os11g03300            MSKSCSLTDLLNTIDCA----LS-QFLLDGSSDAIAEPPAPPSPLIYTTPHPNYQTLNYN
OsNAC10_SEQID2        MSKSCSLTDLLNTIDCAA---LS-QFLLDGSSDAIAEPPAPPSPLIYTTPHPNYQTLNYN
Os12g0123700          MSKSCSLTDLLNNLDCAA---LS-QFLLDGSSDAIAELPAPPSPLIYPN-----QTLNYN
Zm_ZM07MC20223        LSKSCSLTDLLDSIDYAA---LSSQMLLLDAPHPHADDPPPHMVYYPPAASATHQQAIIA
Gm_GM06MC02339        LPRTWSLTYLLD-MNYLG---P---ILSDGSYCSTLDFQINNG-D--------------
Gm_GM06MC00030        LPRTWSLAYLLD-MNYLG---P---ILSDGSYCPTLDFQINNA-NIE-FDPFVKSQPVE-
Mt_AC140030_19.5      LTRSCSLTYLLD-MNYFG---P---ILSDGS---TLDFQINNS-NIG-IDPYVKPQPVE-
Mt_AC202309_11.3      LTRSCSLTYLLD-MNYFG---P---ILSDGS---TLDFQINNS-NIG-IDPYVKPQPVE-
Gm_GM06MC38096        FPRTSSLTHLLE-MDYLG---PISHILPDASYNSTFDFQINTA-NGG-IDPFVKPQPVE-
Mt_CT573505_5.4       FPRTCSLTNLLD-MDYMG---PISQILSDGSYNSTFEFQINTA-HGGTIDP--------
Pt_scaff_VIII.823     FPRTCSLSHLLE-LEYLG---SISQLLSGDTYNSNLDLQNLMS-NAG-TDHVEKIQLGQ-
Sl_TC204003           LPRICSLSHLLE-LDYFG---SIPQLLSDNLLYDDQGYTMNNVNNTSNVDQVSSQQ----
Sl_CDS_43590          LPRICSLSHLLE-LDYFG---SIPQLLSDNLLYDDQGYTMNNVNNTSNVDQVSSQQ----
Sl_TC200098           LPRTCSLSHLLE-MDYFG---SISQLFDDNNSYN--TISQNNT-LMTNVNGYVMPH----
AT1G69490.1           LPRTCSLAHLLE-MDYMG---PVSHIDNFSQFDHLHQPDSESS----------------
Os_CDS_20741          MQKIPSISDLLNEYSLSQ---LFDDGGAAAAAPLQEMARQPDHHHHQQQQHALFGHPVMN
OS_CDS_21636          MQKIPSISDLLNEYSLSQ---LFDDGGAAAAAPLQEMARQPDHHHHQQQQHALFGHPVMN
Zm_ZM07MStraceDB      ---------------------------------------------------------
Os07g0683200          LPKIPSISELFDEHALAQ---IFD-----------AAADPPADHH---QHALAVHPSLN
Ac_CF438306           IQRSFSLSELLNEADFFA---VVE-----------IYRNSGGYRECLVQT---------
AT3G04070.1           QKSSCSFSNLLDATDLTF---LANFLNET-----PENRSESDFSFMIGNFSNPDIYGNHY
AT3G04070.2           QKSSCSFSNLLDATDLTF---LANFLNET-----PENRSESDFSFMIGNFSNPDIYGNHY
Gm_GM06MC20707        QKS-VSFSNLLDAMDYSI---LSSFLSENHTNHPPGVGSSTGFNTGNLD-QQCSPIDNSN
Mt_AC145753_12.5      QKS-VSFSNLLDAMDYSM---LSSFLSEN-SNNPSGIGTSSGFNTENFNQQQSSHINTCN
Pt_scaff_XIII.531     QKS-CSFSNLFDAMDYSM---WSSFLADT-PFNPTGFESNPTLNSTATQLDQP-FFSNSN
Pt_scaff_XIX.359      QKS-CSFSNLLDAMDYSL---LSSLLADT-QFNPTGFESNPALNSTASQLDQPPFFSNSN
Mt_AC128638_20.4      QNEYPMLPYILASKSALP----NSIGMTSDNVNIQASSYDDDLNVIGAQFLSATTEGLFN
Mt_AC150891_8.5       FKDYQIIASILVGGVIPPTENMSSLSVNGSKFNNLNSVSEEGFNKANSQTTNYSLECYFN
```

# FIGURE 4 (continued)

```
OsNAC10_like1      INSNSS--------MPHAFESRLDHHDGYVNNYN----VNGLR-RKRMMAC-------S
Os11g03300         INSNSS--------MPHAFESRLDHHDGYVNNYN----VNGLR-RKRMMAC-------S
OsNAC10_SEQID2     INSNSS--------MPHAFESRLDHHDGYVNNYN----VNGLR-RKRMMAC-------S
Os12g0123700       INNN----------MPHAFESRLDHHDGYVNNYN----VNGLR-RKRMMAC-------S
Zm_ZM07MC20223     NNTNSNDDYYDDDNLLPTAAATAVDAAAAAVLLSSSPADTNGVNKRKRVTAVDYYGAAEP
Gm_GM06MC02339     ------------------------------------------------------------
Gm_GM06MC00030     ---------------MNYNYEADSGKYQVKQSSTIDQPIYVKQMYDLLG-----------
Mt_AC140030_19.5   ---------------MTNHYEADS------HSSITNQPIFVKQMHNYLA-----------
Mt_AC202309_11.3   ---------------MTNHYEADS------HSSITNQPIFVKQMHNYLA-----------
Gm_GM06MC38096     --------------I--PYATDSGKYQVKQNSTINPTIFVNQVYDQRG-----------
Mt_CT573505_5.4    -----------------TYAAGLEKYNVKQNSSFG-----NQVFDQRE-----------
Pt_scaff_VIII.823  ---------------MSLQHTDRGKFQGNLQGSTLNQPLFMNPTMYGFQ-----------
Sl_TC204003        ---------------QNTNNITSNNCNIFFNYQQPLFVNPTFQSQ-------------
Sl_CDS_43590       ---------------QNTNNITSNNCNIFFNYQQPLFVNPTFQSQ-------------
Sl_TC200098        ---------------QAMEKFQLGEVSQIS-------MNPSYQFQ-------------
AT1G69490.1        -----------------WFGDLQFNQDEILNHHR----QAMFKF--------------
Os_CDS_20741       --HFIAN------NSMVQLAHLDPSSSAAASTSAGAVVEPPAVTGKRKRSSDGGEPTIQA
OS_CDS_21636       --HFIAN------NSMVQLAHLDPSSSAAASTSAGAVVEPPAVTGKRKRSSDGGEPTIQA
Zm_ZM07MStraceDB   ------------------------------------------------------------
Os07g0683200       --QLLG-------VGDNFLAECYP-STASTATVAG---------GKRKASPAG-----DY
Ac_CF438306        ------------------------------------------------------------
AT3G04070.1        ------------------LDQKLPQLSSPTSETSGI--------GSKRERVDFAEETINA
AT3G04070.2        ------------------LDQKLPQLSSPTSETSGI--------GSKRERVDFAEETINA
Gm_GM06MC20707     ----------------GYMFQKNNPQLNPSVSNMENMM-------RPKRQVSNMDEETLYP
Mt_AC145753_12.5   ---------------NYMSQKN-PQSN----------------TLKHQLSNVDEDMLYP
Pt_scaff_XIII.531  SASNSNS------TSSGSFLQKLPQLNTSMPNMQEN--------KLKRQLPHIDEDLLHP
Pt_scaff_XIX.359   IAGGG--------GGGCFLQKLPQLSASAPNNVDN--------KLKRQLSHIDEDMLHP
Mt_AC128638_20.4   K-------------PLKRKAVEDNVAMDFYDVLNKSLSREVDGRKQSLEKDLSKGYNFY
Mt_AC150891_8.5    ---------------PLKRKSNEDDDQFENLISFNS---------KFNSENKLCE-----


OsNAC10_like1      AT-FDDGSSSN--DFVHAVVKKPQLLPSDSRGSG-FGGGYCN-----QQLSETATG----
Os11g03300         AT-FDDGSSSN--DFVHAVVKKPQLLPSDSRGSG-FGGGYCN-----QQLSETATG----
OsNAC10_SEQID2     ATSFDDGSSSN--DFVHAVVKKPQLLPSDSRGSG-FGGGYCN-----QQLSETATG----
Os12g0123700       ATSFDDGSSSSSSDFLH-VAKKPLLLPSDSRGSG-FGGGYCN-----QQLSETATG----
Zm_ZM07MC20223     PTFFHHHLDDGGNSFFSTTKKLKPPPPSDSRHGGHFGTATASSYRDSQQLVDSASSSGVF
Gm_GM06MC02339     ------------------------------------------------------------
Gm_GM06MC00030     ------------------------------------------------------------
Mt_AC140030_19.5   ------------------------------------------------------------
Mt_AC202309_11.3   ------------------------------------------------------------
Gm_GM06MC38096     ------------------------------------------------------------
Mt_CT573505_5.4    ------------------------------------------------------------
Pt_scaff_VIII.823  ------------------------------------------------------------
Sl_TC204003        ------------------------------------------------------------
Sl_CDS_43590       ------------------------------------------------------------
Sl_TC200098        ------------------------------------------------------------
AT1G69490.1        LPPAAAAAKKPNGSCVG-ATFQIGS--ALQGSS----LGLSHQMLLHSNMGMNLDLDLEL
Os_CDS_20741       LPPAAAAAKKPNGSCVG-ATFQIGS--ALQGSS----LGLSHQMLLHSNMGMN------
OS_CDS_21636       ------------------------------------------------------------
Zm_ZM07MStraceDB   AGGGHTPAKRLNGSCFDVAPQSVVG--GLQATPSSVLAGLNHQMLPPQLF---------
Os07g0683200       ------------------------------------------------------------
Ac_CF438306        SKKMMN-TYSYN---NSIDQMDHS---MMQQPS-----FLNQELMMSSHLQYQG------
AT3G04070.1        SKKMMN-TYSYN---NSIDQMDHS---MMQQPS-----FLNQELMMSSHLQYQG------
AT3G04070.2        SKKYLSSSCNFPT--NINNQYENPQWNYLMKQS-----LLNQRLMLGPHLQFQG------
Gm_GM06MC20707     SKKYLSSSCNFP---NINSQYEN----YLMKQS-----LMNQQLLLGPHHQYQG------
Mt_AC145753_12.5   SKKFMN-SCSFTNTNNN-TQTDMGQ-YNFVSQP-----FLDQQLLLSPHLQFLG------
Pt_scaff_XIII.531  SKKFMN-SCSFTNTNNISTQTDNGQ-YNFLSQP-----FLNQQFLLSPHLQFQG------
Pt_scaff_XIX.359   SFDQWTSIIQPQELNSLTFTG-YT-----------------------------------
Mt_AC128638_20.4   -----SPSIDFQKLNKFTFTERYQQ---------------------------------
Mt_AC150891_8.5
```

# FIGURE 4 (continued)

```
OsNAC10_like1        -FQFQNGNLLSHPFP-LN--------NHLQMQ
Os11g03300           -FQFQNGNLLSHPFP-LN--------NHLQMQ
OsNAC10_SEQID2       -FQFQNGNLLSHPFP-LN--------NHLQMQ
Os12g0123700         -FQFQNGNMLSHPFP-LNQQ--LLLNNHLQMQ
Zm_ZM07MC20223       FHQYGYGYSSSNPFLNLNQQQQLLLNSHIGMQ
Gm_GM06MC02339       -------------------------------
Gm_GM06MC00030       -------------------------------
Mt_AC140030_19.5     -------------------------------
Mt_AC202309_11.3     -------------------------------
Gm_GM06MC38096       -------------------------------
Mt_CT573505_5.4      -------------------------------
Pt_scaff_VIII.823    -------------------------------
Sl_TC204003          -------------------------------
Sl_CDS_43590         -------------------------------
Sl_TC200098          -------------------------------
AT1G69490.1          -------------------------------
Os_CDS_20741         RLGFA--------------------------
OS_CDS_21636         -------------------------------
Zm_ZM07MStraceDB     -------------------------------
Os07g0683200         -------------------------------
Ac_CF438306          -------------------------------
AT3G04070.1          -------------------------------
AT3G04070.2          -------------------------------
Gm_GM06MC20707       -------------------------------
Mt_AC145753_12.5     -------------------------------
Pt_scaff_XIII.531    -------------------------------
Pt_scaff_XIX.359     -------------------------------
Mt_AC128638_20.4     -------------------------------
Mt_AC150891_8.5      -------------------------------
```

## FIGURE 4 (continued)

FIGURE 5

FIGURE 6

FIGURE 7

FIGURE 8

FIGURE 9

EP 2 957 637 A2

FIGURE 10

FIGURE 11

FIGURE 12

FIGURE 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5811238 A
- US 6395547 B
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A

- WO 9853083 A, Grierson
- WO 9953050 A, Waterhouse
- US 4987071 A
- US 5116742 A
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- US 5164310 A
- US 5159135 A

**Non-patent literature cited in the description**

- **REBETZKE et al.** *Crop Science,* 2002, vol. 42, 739
- **GARDENER et al.** Physiology of Crop Plants. Iowa State University Press, 1985, 68-73
- **TITTONELL et al.** *Agric Ecosys & Environ,* 2005, vol. 105, 213
- **WANG et al.** *Planta,* 2003, vol. 218, 1-14
- **REED.** *Trends in Plant Science,* 2001, vol. 6, 420-425
- **JAIN et al.** *Funct Integr Genomics,* 2006, vol. 6 (1), 47-59
- **CREIGHTON.** Proteins. W.H. Freeman and Company, 1984
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137

- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics.,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689

- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640

- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767

- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- A generalized profile syntax for biomolecular sequences motifs and its function in automatic sequence interpretation. **BUCHER ; BAIROCH.** ISMB-94; . Proceedings 2nd International Conference on Intelligent Systems for Molecular Biology. AAAI Press, 1994, 53-61
- **GASTEIGER et al.** *Nucleic Acids Res.,* 2003, vol. 31, 3784-3788
- **FUKAKI et al.** *Plant J.,* 2005, vol. 44, 382-395
- Current Protocols in Molecular Biology. Current Protocols, 1994, vol. 1, 2
- Current Protocols in Molecular Biology. Wiley
- **FRINK et al.** *Proc Natl Acad Sci USA,* 1999, vol. 96 (4), 1175-1180
- **SAMBROOK J ; FRITSCH EF ; MANIATIS T.** Molecular Cloning, A Laboratory Manual. 1989
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd (UK) and Blackwell Scientific Publications (UK), 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **PARK.** *Bioinformatics,* 2003, vol. 19, 1656-1663
- **PARK ; KANEHISA.** *Bioinformatics,* 2003, vol. 19, 1656-1663
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158